# EUROPEAN PATENT SPECIFICATION

(11) **EP 2 265 125 B1**
(45) Date of publication and mention of the grant of the patent: **14.08.2019**
(21) Application number: 09733509.5
(22) Date of filing: 15.04.2009
(51) Int. Cl.: A01N 55/02, A61K 31/555, A61K 31/472, A61K 31/4725, A61K 38/13, A61P 11/00, A61P 17/00, A61P 17/06, A61P 17/14, A61P 29/00, A61P 19/02, A61K 9/00

(54) **TOPICAL LFA-1 ANTAGONISTS FOR USE IN LOCALIZED TREATMENT OF IMMUNE RELATED DISORDERS**
TOPISCHE LFA-1-ANTAGONISTEN ZUR VERWENDUNG BEI DER LOKALEN BEHANDLUNG VON IMMUNERKRANKUNGEN
ANTAGONISTES DE LFA-1 TOPIQUES UTILISÉS DANS LE TRAITEMENT LOCALISÉ DE TROUBLES DE NATURE IMMUNITAIRE

(30) Priority: 15.04.2008 US 45240
(43) Date of publication of application: 29.12.2010
(73) Proprietor: SARcode Bioscience Inc., Brisbane, CA 94005 (US)
(72) Inventor: BURNIER, John, Pacifica CA 94404 (US); GADEK, Thomas, Oakland CA 94611 (US)
(74) Representative: Carpmaels & Ransford LLP
(86) International application number: PCT/US2009/002389
(87) International publication number: WO 2009/128934

(56) References cited:
- US-A1- 2002 045 582
- US-A1- 2005 148 588
- US-A1- 2005 267 098
- US-A1- 2006 281 739
- US-A1- 2007 142 317
- US-B2- 7 314 938

## Description

### BACKGROUND OF THE INVENTION

The (CD11/CD18) family of adhesion receptor molecules comprises four highly related cell surface glycoproteins; LFA-1 (CD11a/CD18), Mac-1 (CD11b/CD18), p150.95 (CD11c/CD18) and (CD11d/CD18). The CD11/CD18 family is related structurally and genetically to the larger integrin family of receptors that modulate cell adhesive interactions, which include; embryogenesis, adhesion to extracellular substrates, and cell differentiation (Hynes, R. O., Cell 48:549-554 (1987); Kishimoto et al., Adv. Immunol. 46:149-182 (1989); Kishimoto et al., Cell 48:681-690 (1987); Ruoslahti et al., Science 238:491-497 (1987)). LFA-1 is a heterodimeric adhesion molecule present on the surface of all mature leukocytes except a subset of macrophages and is considered the major lymphoid integrin. The expression of Mac-1, p150.95 and CD11d/CD18 is predominantly confined to cells of the myeloid lineage (which include neutrophils, monocytes, macrophage and mast cells). LFA-1 and Mac-1 (CD11b/CD18) are known to be of primary importance to function of leukocytes (Li et al. (2006) Am J Pathology 169:1590-1600). LFA-1 in particular is involved in migration of leukocytes to sites of inflammation (Green et al. (2006) Blood 107:2101-11).

Functional studies have suggested that *LFA*-1 interacts with several ligands, including ICAM-1 (Rothlein et al., J. Immunol. 137:1270-1274 (1986), ICAM-2, (Staunton et al., Nature 339:361-364 (1989)), ICAM-3 (Fawcett et al., Nature 360:481-484 (1992); Vezeux et al., Nature 360:485-488, (1992); de Fougerolles and Springer, J. Exp. Med. 175:185-190 (1990)) and Telencephalin (Tian et al., J. Immunol. 158:928-936 (1997)). Normal interaction of LFA-1 with ICAMs acts as costimulatory molecules in the peptide-MHC complex (Grakoui et al. (1999) Science 285:221-7; Malissen (1999) Science 285:207-8). ICAMs 1-3 are known to regulate lymphocytes and T-cell activation (Perez et al. (2007) BMC Immunol. 8:2). ICAM-4 is a red blood cell specific ligand and ICAM-5 is known to recruit leukocytes to neurons in the central nervous system (Ihanus et al. (2007) Blood 109:802-10; Tian et al. (2000) Eur J. Immunol. 30:810-8). Upon binding, LFA-1 undergoes a conformational change that results in higher affinity binding and receptor clustering (Hogg et al. (2003) J Cell Sci. 116:4695-705; Takagi et al. (2002) Cell 110:599-611).

During an inflammatory response peripheral blood leukocytes are recruited to the site of inflammation or injury by a series of specific cellular interactions. The lymphocyte function associated antigen-1 (LFA-1) has been identified as the major integrin that mediates lymphocyte adhesion and activation leading to a normal immune response, as well as several pathological states (Springer, T. A., Nature 346: 425-434 (1990)). The binding of LFA-1 to ICAMs mediate a range of lymphocyte functions including lymphokine production of helper T-cells in response to antigen presenting cells, T-lymphocyte mediated target cells lysis, natural killing of tumor cells, and immunoglobulin production through T-cell-B-cell interactions. Thus, many facets of lymphocyte function involve the interaction of the LFA-1 integrin and its ICAM ligands. These LFA-1 :ICAM mediated interactions have been directly implicated in numerous inflammatory disease states including; graft rejection, dermatitis, psoriasis, asthma and rheumatoid arthritis.

US 2006/281739 A1 discloses compounds and methods for the treatment of LFA-1 mediated diseases.

US 2005/267098 A1 discloses compounds, pharmaceutical compositions thereof and methods for the use thereof for the treatment of disorders mediated by the CD11/CD18 family of cellular adhesion molecules.

### SUMMARY OF THE INVENTION

Embodiments of the present invention are set out in the enclosed claims.

In one aspect, the present invention provides a pharmaceutical formulation comprising an LFA-1 antagonist or a pharmaceutically acceptable salt or ester thereof, and an excipient formulated for topical administration to skin, wherein the LFA-1 antagonist has a systemic clearance rate greater than about 2 mL/min/kg when administered to a subject, and wherein the formulation is:
(a) a gel comprising about 1% W/V of a LFA-1 antagonist; up to about 15% W/V Dimethyl Isosorbide; up to about 25% W/V Transcutol; up to about 1% W/V Hydroxyethyl Cellulose; up to about 12%W/V Hexylene glycol, up to about 0.15% W/V Methylparaben; up to about 0.05% W/V Propylparaben; and water; or
(b) a ointment comprising about 1% W/V of a LFA-1 antagonist, up to about 10% W/V Dimethyl Isosorbide; up to about 0.02% W/V Butylated Hydroxytoluene; up to about 2% W/V Span 80; up to about 10% W/V White Wax; and White Petrolatum; or
(c) a water based lotion comprising about 1% W/V of a LFA-1 antagonist, up to about 15% W/V Dimethyl Isosorbide; up to about 25% W/V Transcutol; up to about 12% W/V Hexylene glycol; up to about 5% W/V Propylene Glycol; and pH 6.0 25% Trolamine, wherein the lotion is buffered to a pH of about 4.0 to about 7.5; or
(d) an aqueous solution buffered to a pH of about 6.0 to about 8.0 with Sodium Phosphate, Monobasic, comprising about 1% W/V of a LFA-1 antagonist, up to about 0.1% W/V EDTA, and, optionally, up to about 0.4% w/w Methylparaben and up to about 0.02% w/w Propylparaben;
and wherein the LFA-1 antagonist comprises a compound of one of the following formulae and/or a pharmaceutically acceptable salt or ester thereof:

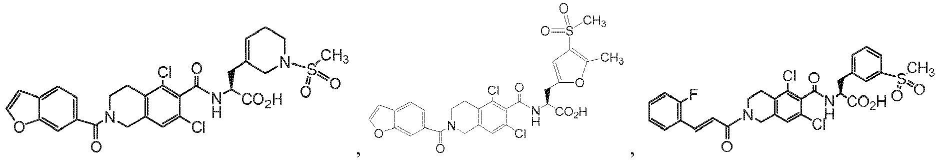

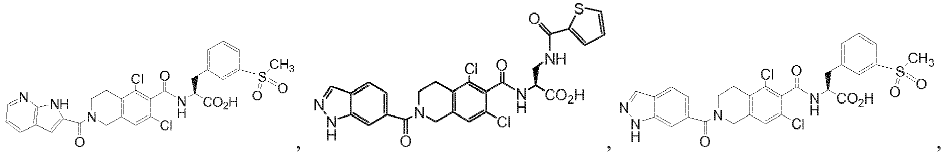

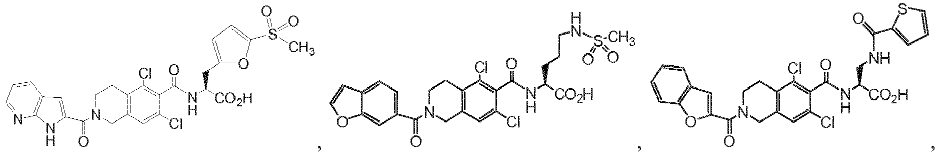

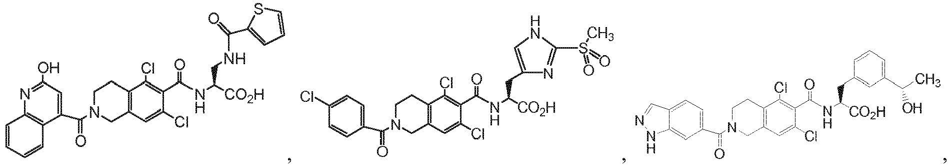

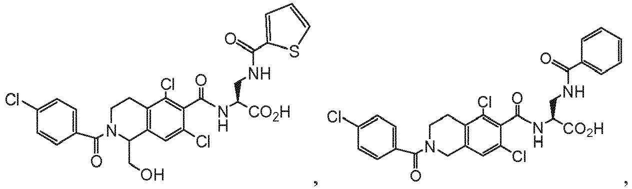

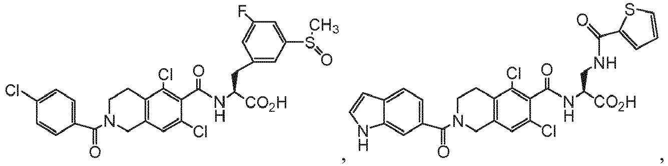

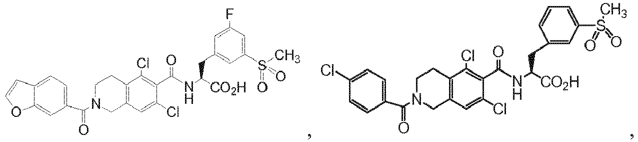

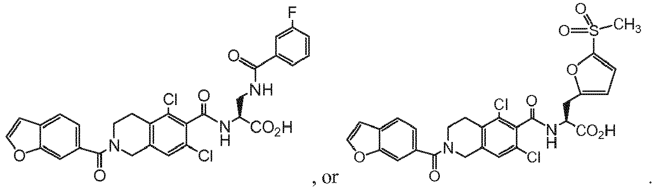

In another aspect, the present invention provides a pharmaceutical formulation of the invention as defined above, for use in the treatment of an inflammatory or immune related disorder in a subject, wherein the inflammatory or immune related disorder is psoriasis, irritant contact dermatitis, eczematous dermatitis, seborrhoeic dermatitis, cutaneous manifestations of immunologically-mediated disorders, alopecia, alopecia areata, scleredoma, scar formation, atopic dermatitis, hidradentis supporativa, rheumatoid arthritis, psoriatic arthritis, or arthralgia.

In one aspect of the disclosure, a pharmaceutical formulation is provided comprising an LFA-1 antagonist or a pharmaceutically acceptable salt or ester thereof, and an excipient formulated for topical administration, wherein the LFA-1 antagonist has a systemic clearance rate greater than about 2 mL/min/kg when administered to a subject.

In another aspect of the disclosure, a method for treatment of an inflammatory or immune related disorder in a subject is provided including topically administering to the subject in need thereof a formulation including an LFA-1 antagonist or a pharmaceutically acceptable salt or ester thereof, and a pharmaceutically acceptable excipient, wherein the LFA-1 antagonist has a systemic clearance rate greater than about 2 mL/min/kg when administered to a subject. In one embodiment, following administration, the LFA-1 antagonist is present in a therapeutically effective concentration within about 1 mm of an epithelial surface to which the formulation is applied and is present in blood plasma below a therapeutically effective level, within about 4 hours after administration. In yet another embodiment, following administration, the LFA-1 antagonist is present in a therapeutically effective concentration within about 1 mm of an epithelial surface to which the formulation is applied and is present in blood plasma below a therapeutically effective level, within about 4 hours following administration. In other embodiments, the LFA-1 antagonist has a local tissue concentration of greater than about 10 nM within about 4 hours following administration. In various embodiments, the LFA-1 antagonist has a local tissue concentration of greater than about 1 µM and a systemic concentration as measured in plasma of less than about 100 nM, within about 4 hours following administration. In some embodiments, the local tissue concentration of the LFA-1 antagonist is maintained at greater than about 10 nM for at least about 8 hours following administration.

In some embodiments, the LFA-1 antagonist is a directly competitive antagonist.

In one embodiment, the LFA-1 antagonist achieves a local tissue concentration of greater than about 1 µM within about 4 hours following administration to a subject. In some embodiments, the local tissue concentration of the LFA-1 antagonist is maintained at a concentration of greater than about 10 nM for at least about 8 hours when administered to a subject.

In one embodiment of the disclosure, the LFA-1 antagonist comprises a compound of Formula I or II and/or its pharmaceutically acceptable salts or esters, having the following structures: 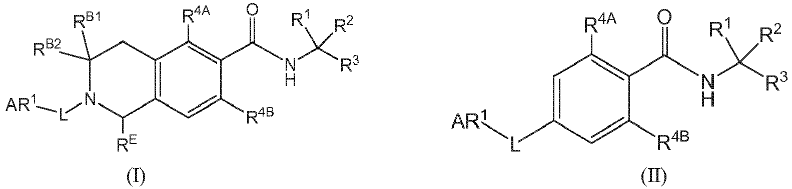
wherein R¹ and R² are each independently hydrogen, an amino acid side chain, -(CH₂)ₘOH, -(CH₂)ₘaryl, - (CH₂)ₘheteroaryl, wherein m is 0-6, -CH(R^{1A})(OR^{1B}), -CH(R^{1A})(NHR^{1B}), U-T-Q, or an aliphatic, alicyclic, heteroaliphatic or heteroalicyclic moiety optionally substituted with U-T-Q,
wherein U is absent, -O-, -S(O)₀₋₂-, -SO₂N(R^{1A}), -N(R^{1A})-, -N(R^{1A})C(=O)-, -N(R^{1A})C(=O)-O-, - N(R^{1A})C(=O)-N(R^{1B})-, -N(R^{1A})-SO₂-, -C(=O)-, -C(=O)-O-, -O-C(=O)-, aryl, heteroaryl, alkylaryl, alkylheteroaryl,-C(=O)-N(R^{1A})-, -OC(=O)N(R^{1A})-, -C(=N-R^{1E})-, -C(=N-R^{1E})-O-, -C(=N-R^{1E})-N(R^{1A})-, -O-C(=N-R^{1E})-N(R^{1A})-, - N(R^{1A})C(=N-R^{1E})-, -N(R^{1A})C(=N-R^{1E})-O-, -N(R^{1A})C(=N-R^{1E})-N(R^{1B})-, -P(=O)(OR^{1A})-O-, or -P(=O)(R^{1A})-O-;
T is absent, an aliphatic, heteroaliphatic, aryl, heteroaryl, alkylaryl or alkylheteroaryl moiety; and
Q is hydrogen, halogen, cyano, isocyanate, -OR^{1B}; -SR^{1B}; -N(R^{1B})₂, -NHC(=O)OR^{1B}, -NHC(=O)N(R^{1B})₂, - NHC (=O)R^{1B}, -NHSO₂R^{1B}, NHSO₂N(R^{1b})₂, -NHSO₂NHC(=O)OR^{1B}, -NHC(=O)NHSO₂R^{1B}, - C(=O)NHC(=O)OR^{1B}, C(=O)NHC(=O)R^{1B}, -C(=O)NHC(=O) N(R^{1B})₂, -C(=O)NHSO₂R^{1B}, -C(=O)NHSO₂N(R^{1B}) ₂, C(=S)N(R^{1B})₂, -SO₂R^{1B}, -SO₂OR^{1B}, -SO₂N(R^{1B})₂, -SO₂-NHC (=O)OR^{1B}, -OC(=O)-N(R^{1B})₂, -OC(=O)R^{1B}, - OC(=O)NHC(=O)R^{1B}, -OC(=O)NHSO₂R^{1b}, -OSO₂R^{1B}, or an aliphatic heteroaliphatic, aryl or heteroaryl moiety, or wherein R¹ and R² taken together are an alicyclic or heterocyclic moiety, or together are 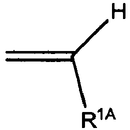
wherein each occurrence of R^{1A} and R^{1B} is independently hydrogen, an aliphatic, alicyclic, heteroaliphatic, heterocyclic, aryl, heteroaryl, alkylaryl or alkylheteroaryl moiety, -C(=O)R^{1C}, or -C(=O)NR^{1C}R^{1D}; wherein each occurrence of R^{1C} and R^{1D} is independently hydrogen, hydroxyl, or an aliphatic, heteroaliphatic, aryl, heteroaryl, alkylaryl or alkylheteroaryl moiety; and R^{1E} is hydrogen, an aliphatic, alicyclic, heteroaliphatic, heterocyclic, aryl, heteroaryl, alkylaryl or alkylheteroaryl moiety, -CN, -OR^{1C},-NR^{1C} R^{1D} or -SO₂R^{1C};
R³ is -C(=O)OR^{3A}, -C(=O)H, -CH₂OR^{3A}, -CH₂OC(=O)-alkyl, -C(=O)NH(R^{3A}), -CH₂X⁰; wherein each occurrence of R^{3A} is independently hydrogen, a protecting group, an aliphatic, alicyclic, heteroaliphatic, heteroalicyclic, aryl, heteroaryl, alkylaryl, alkylheteroaryl, heteroalkylaryl heteroalkylheteroaryl moiety, or pharmaceutically acceptable salt or ester, or R^{3A}, taken together with R¹ and R², forms a heterocyclic moiety; wherein X⁰ is a halogen selected from F, Br or I;
wherein R^{4A} and R^{4B} are independently a halogen selected from F, Cl, Br or I; and R^{B1}, R^{B2} and R^{E} is independently hydrogen or substituted or unsubstituted lower alkyl.

AR¹ is a monocyclic or polycyclic aryl, heteroaryl, alkylaryl, alkylheteroaryl, alicyclic or heterocyclic moiety; and,
L is absent or is V-W-X-Y-Z, wherein each occurrence of V, W, X, Y and Z is independently absent, C=O, NR^{L1}, -O-, -C(R^{L1})=, =C(R^{L1})-, -C(R^{L1})(R^{L2}), C(=N-O R^{L1}), C(=NR^{L1}), -N=, S(O)₀₋₂ ; a substituted or unsubstituted C₁₋₆ alkenylidene or C₂₋₆ alkenylidine chain wherein up to two non-adjacent methylene units are independently optionally replaced by-C(=O)-, -CO₂-, -C(=O)C(=O)-, -C(C=O)NR^{L3}-, -OC(=O)-, -OC(=O)NR^{L3}-, - NR^{L3}NR^{L4}-, -NR^{L3}NR^{L4}C(=O)-, -NR^{L3}C(=O)-, NR^{L3}CO₂-, NR^{L3}C(=O)NR^{L4}-, -S(=O)-, -SO₂-, -NR^{L3}SO₂-, - SO₂NR^{L3}, -NR^{L3}SO₂NR^{L4}, -O-, -S-, or -NR^{L3}-; wherein each occurrence of R^{L3}and R^{L4} is independently hydrogen, alkyl, heteroalkyl, aryl, heteroaryl or acyl; or an aliphatic, alicyclic, heteroaliphatic, heteroalicyclic, aryl, heteroaryl, alkylaryl or alkylheteroaryl moiety; and each occurrence of R^{L1} and R^{L2} is independently hydrogen, hydroxyl, protected hydroxyl, amino, protected amino, thio, protected thio, halogen, cyano, isocyanate, carboxy, carboxyalkyl, formyl, formyloxy, azido, nitro, ureido, thioureido, thiocyanato, alkoxy, aryloxy, mercapto, sulfonamido, benzamido, tosyl, or an aliphatic, alicyclic, heteroaliphatic, heteroalicyclic, aryl, heteroaryl, alkylaryl or alkylheteroaryl moiety, or wherein one or more occurrences of R^{L1} and R^{L2}, taken together, or taken together with one of V, W, X, Y or Z can form an alicyclic or heterocyclic moiety or can form an aryl or heteroaryl moiety.

In various embodiments of the disclosure, the LFA-1 antagonist has one of the following formulae:

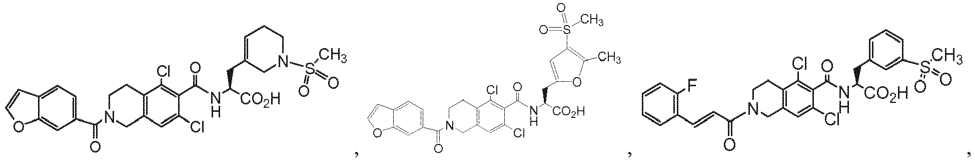

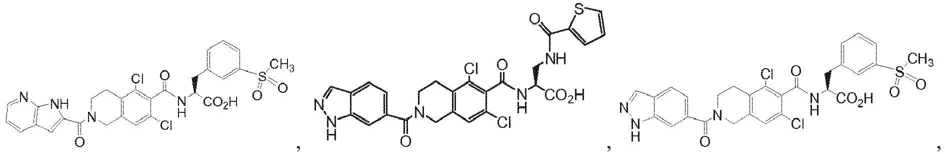

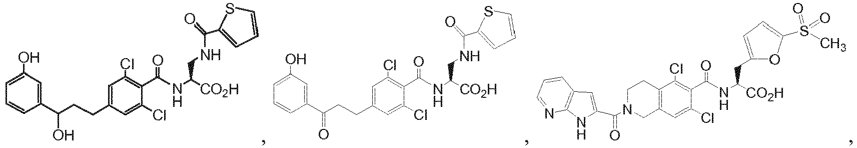

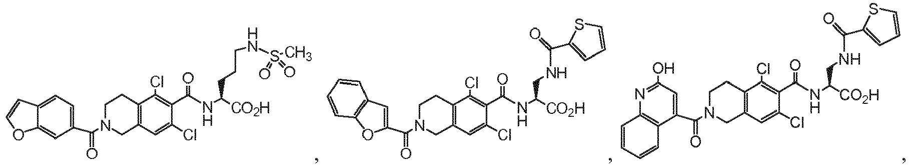

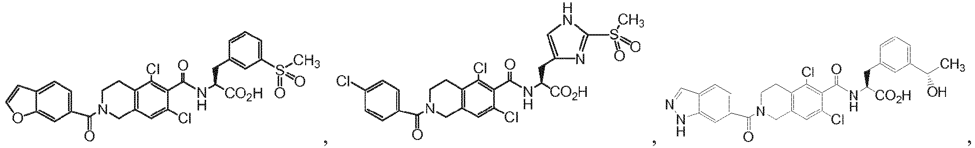

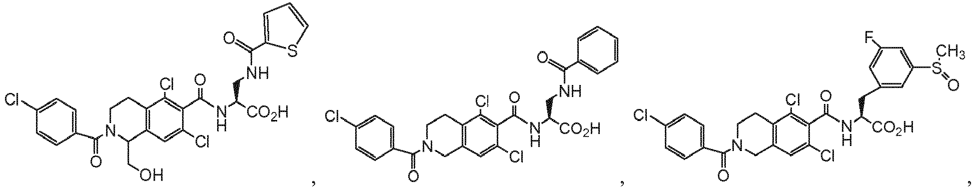

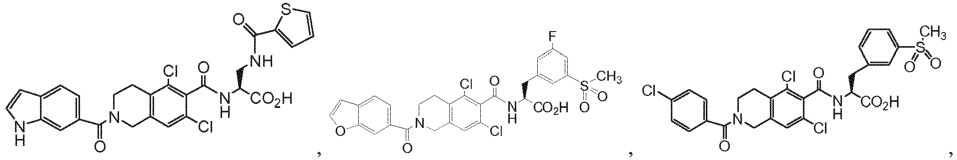

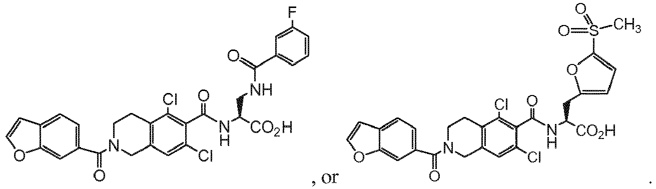

In some embodiments of the disclosure, the LFA-1 antagonist is a compound having the following formula:

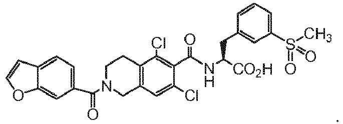

In other embodiments of the disclosure, the LFA-1 antagonist is any of Form A, Form B, Form C, Form D, Form E, an amorphous form, or a combination thereof of the compound having the following formula:

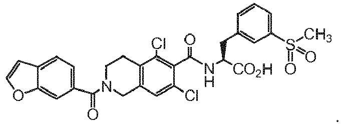

In yet other embodiments of the disclosure, the LFA-1 antagonist is form A of the compound having the following formula:

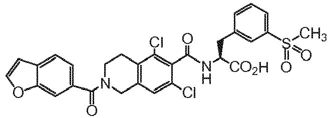

In one embodiment, the LFA-1 antagonist is a sodium, potassium, lithium, magnesium, zinc, or calcium salt. In one embodiment, the LFA-1 antagonist inhibits T-cell attachment to ICAM-1 by about 50% or more at a concentration of about 100 nM.

In some embodiments of the disclosure, the formulation is in the form of a gel, cream, lotion, solution, suspension, emulsion, ointment, powder, crystalline forms, spray, foam, salve, paste, plaster, paint, slow release nanoparticle, slow release microparticle, or bioadhesive.

In one embodiment, the excipient is water, buffered aqueous solution, surfactant, volatile liquid, starch, polyol, granulating agent, microcrystalline cellulose, diluent, lubricant, acid, base, salt, emulsion, oil, wetting agent, chelating agent, antioxidant, sterile solution, complexing agent or disintegrating agent. In one embodiment, the surfactant is oleic acid, cetylpyridinium chloride, soya lecithin, polyoxyethylene sorbitan monolaurate, polyoxyethylene sorbitan monostearate, polyoxyethylene sorbitan monooleate, polyoxyethylene stearyl ether, polyoxyethylene oleyl ether, polyoxyethylene-polyoxypropylene-ethylenediamine block copolymer, polyoxypropylene-polyoxyethylene block copolymer or castor oil ethoxylate.

In other embodiments, the formulation comprises a topical penetration enhancer. In some embodiments, the topical penetration enhancer is a sulfoxide, ether, surfactant, alcohol, fatty acid, fatty acid ester, polyol, amide, terpene, alkanone or organic acid. In yet other embodiments, the formulation comprises at least one additional therapeutic agent. In other embodiments, the additional therapeutic agent is an antioxidant, antiinflammatory agent, antimicrobial agent, antiangiogenic agent, anti-apoptotic agent, vascular endothelial growth factor inhibitor, antiviral agent, calcineurin inhibitor, corticosteroid, immunomodulator, or lubricating eye drop. In yet other
embodiments, the additional therapeutic agent is cyclosporine, Rebamipide, diquafasol, or lubricating eye drops. In some embodiments, the formulation is a gel comprising about 1% W/V of a LFA-1 antagonist; up to about 15% W/V Dimethyl Isosorbide; up to about 25% W/V Transcutol; up to about 1% W/V Hydroxyethyl Cellulose; up to about 12%W/V Hexylene glycol, up to about 0.15% W/V Methylparaben; up to about 0.05% W/V Propylparaben; and water.

In some embodiments, the formulation is an ointment comprising about 1% W/V of a LFA-1 antagonist, up to about 10% W/V Dimethyl Isosorbide; up to about 0.02% W/V Butylated Hydroxytoluene; up to about 2% W/V Span 80; up to about 10% W/V White Wax; and White Petrolatum.

In various embodiments, the formulation is a water based lotion comprising about 1% W/V of a LFA-1 antagonist, up to about 15% W/V Dimethyl Isosorbide; up to about 25% W/V Transcutol; up to about 12% W/V Hexylene glycol; up to about 5% W/V Propylene Glycol; and pH 6.0 25% Trolamine, wherein the lotion is buffered to a pH of about 4.0 to about 7.5.

In some embodiments, the formulation is an aqueous solution buffered to a pH of about 6.0 to about 8.0 with Sodium Phosphate, Monobasic,comprising about 1% W/V of a LFA-1 antagonist, up to about 0.1% W/V EDTA, and, optionally, up to about 0.4% w/w Methylparaben and up to about 0.02% w/w Propylparaben.

In one embodiment, the LFA-1 antagonist is form A of the compound.

In yet other embodiments, the LFA-1 antagonist inhibits T-cell attachment to ICAM-1 by about 50% or more at a concentration of about 100 nM.

In some embodiments, the formulation is topically applied to skin, eyes, mouth, nose, vaginal mucosa, or anal mucosa. In one embodiment of the disclosure, the formulation is in the form of a gel, cream, lotion, solution, suspension, emulsion, ointment, powder, crystalline forms, spray, foam, salve, paste, plaster, paint, slow release nanoparticle, slow release microparticle, or bioadhesive. In yet another embodiment, the formulation comprises a surfactant which is oleic acid, cetylpyridinium chloride, soya lecithin, polyoxyethylene sorbitan monolaurate, polyoxyethylene sorbitan monostearate, polyoxyethylene sorbitan monooleate, polyoxyethylene stearyl ether, polyoxyethylene oleyl ether, polyoxyethylene-polyoxypropylene-ethylenediamine block copolymer, polyoxypropylene-polyoxyethylene block copolymer or castor oil ethoxylate.

In other embodiments, the method comprises a topical penetration enhancer. In one embodiment, the topical penetration enhancer is a sulfoxide, ether, surfactant, alcohol, fatty acid, fatty acid ester, polyol, amide, terpene, alkanone, or organic acid.

In some embodiments, the formulation comprises at least one additional therapeutic agent. In one embodiment, the additional therapeutic agent is an antioxidant, antiinflammatory agent, antimicrobial agent, antiangiogenic agent, anti-apoptotic agent, vascular endothelial growth factor inhibitor or antiviral agent.

In yet other embodiments, the formulation is administered in a dose from about 0.01 to about 5 mg.

In various embodiments of the disclosure, the inflammatory or immune disorder is intraocular inflammation, periocular inflammation, ocular surface inflammation, Keratoconjunctivitis, keratoconjunctivitis sicca (KCS, aka Dry Eye), KCS in patients with Sjogren's syndrome, age related macular degeneration (AMD), allergic conjunctivitis, uveitis, inflammation of the eye from contact lens wear, inflammation of the the cornea from contact lens wear, inflammation of the periocular tissue from contact lens wear, inflammation of the eye following surgery, intraocular inflammation, retinitis, edema, retinopathy, corneal inflammation, Graves' disease (Basedow disease) or Graves ophthalmopathy.

In other embodiments of the disclosure, the inflammatory or immune disorder is psoriasis, irritant contact dermatitis, eczematous dermatitis, seborrhoeic dermatitis, cutaneous manifestations of immunologically-mediated disorders, alopecia, alopecia areata, adult respiratory distress syndrome, pulmonary fibrosis, scleredoma, scar formation, chronic obstructive pulmonary disease (COPD), atopic dermatitis, inflammation from kidney transplant, asthma, hidradentis supporativa, rheumatoid arthritis, psoriatic arthritis, Sjogren's Syndrome, uveitis, Graft vs. Host disease (GVHD), Oral Lichen Planus, arthralgia or Islet Cell Transplant inflammation.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

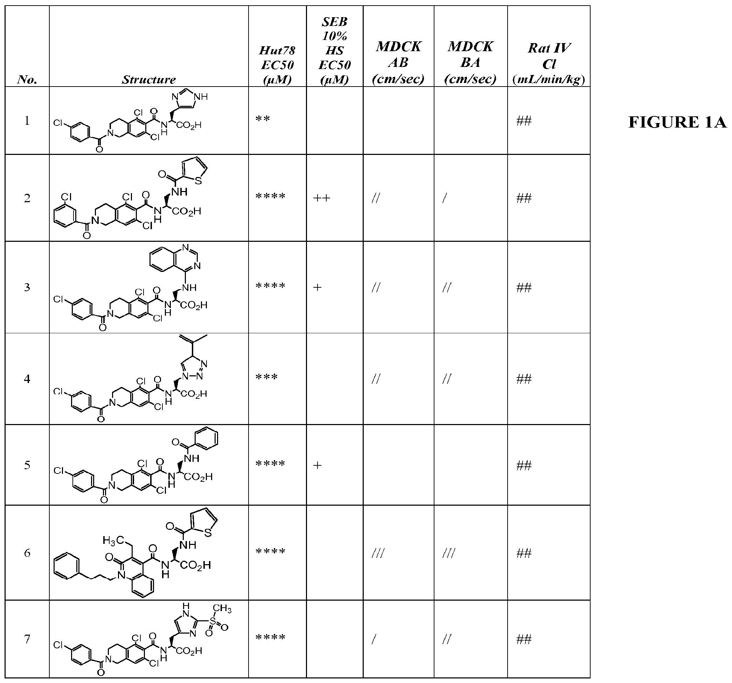

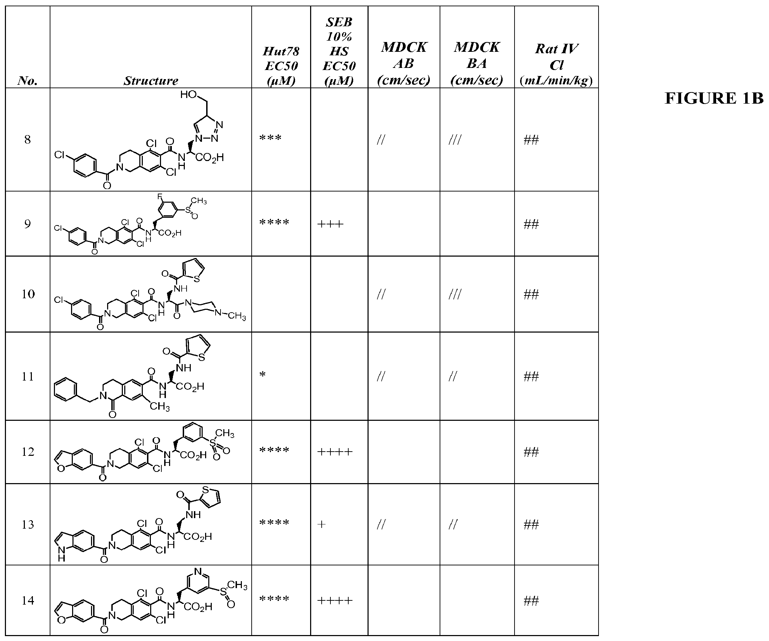

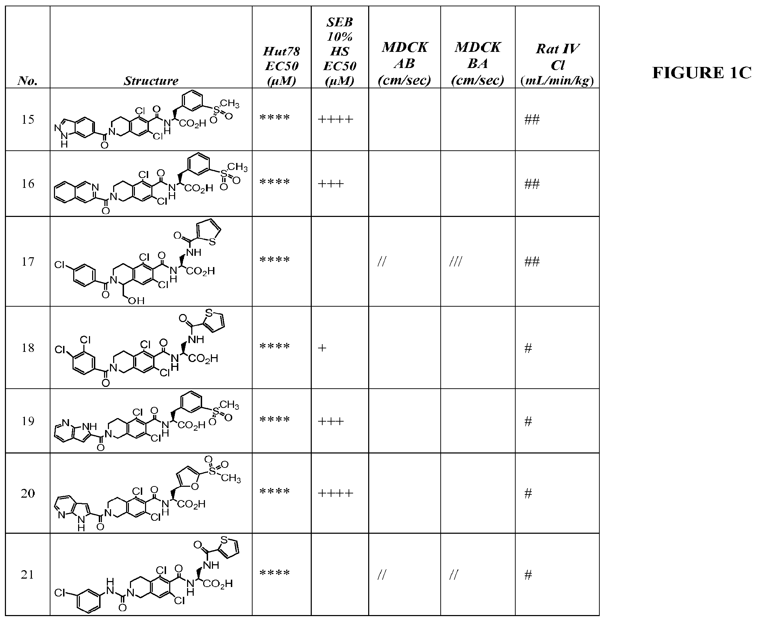

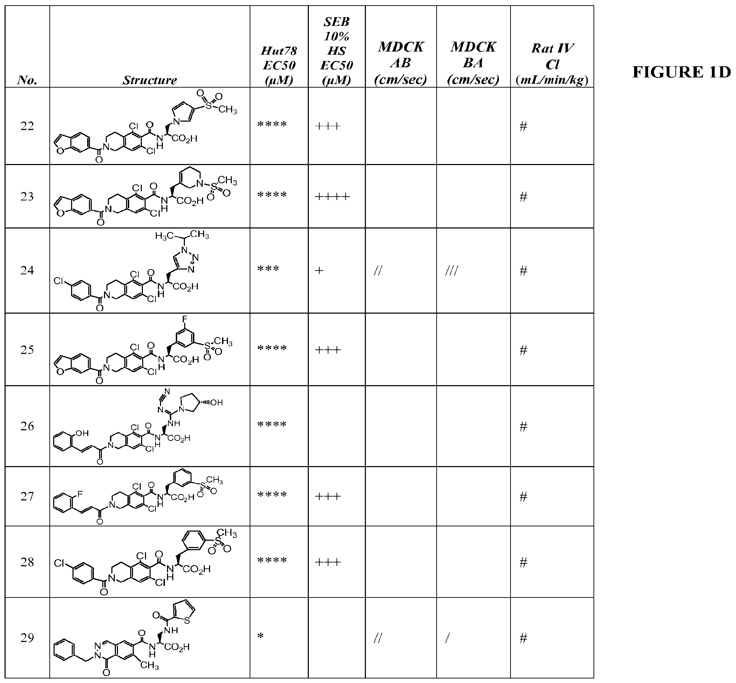

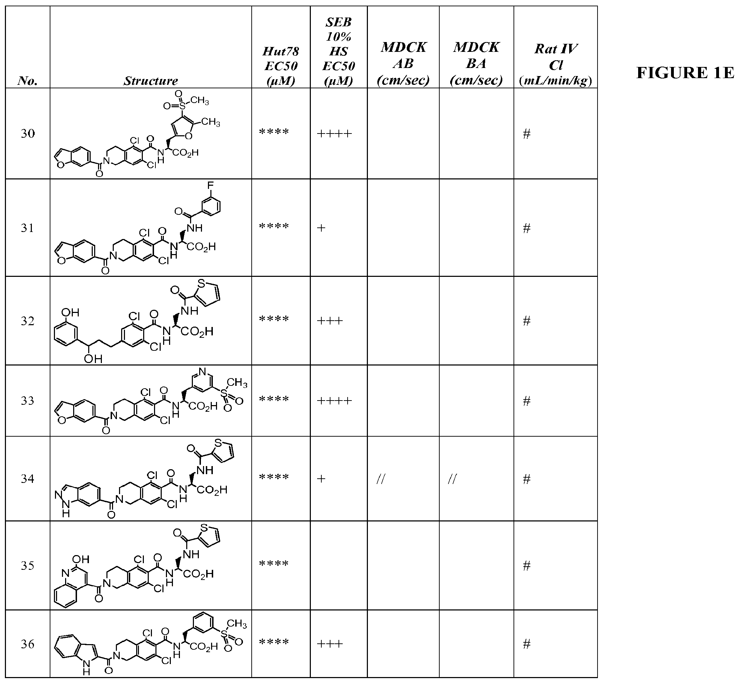

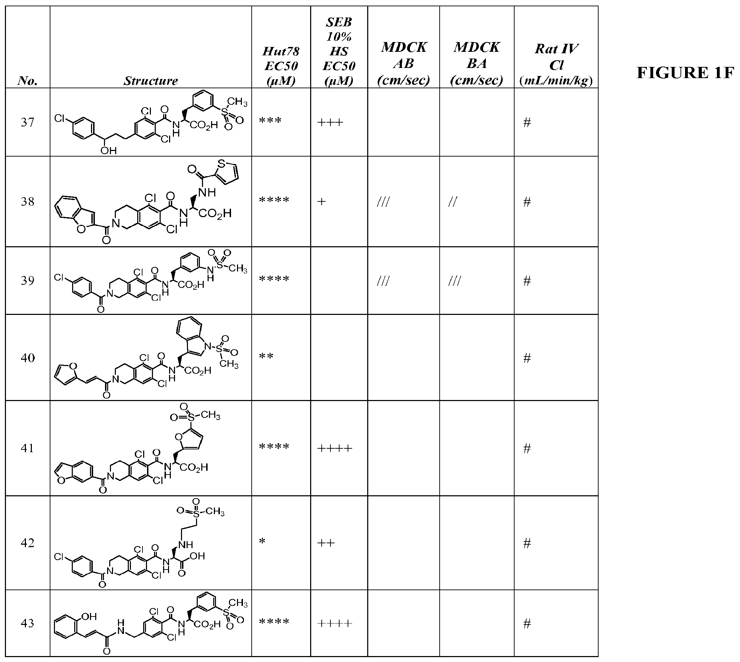

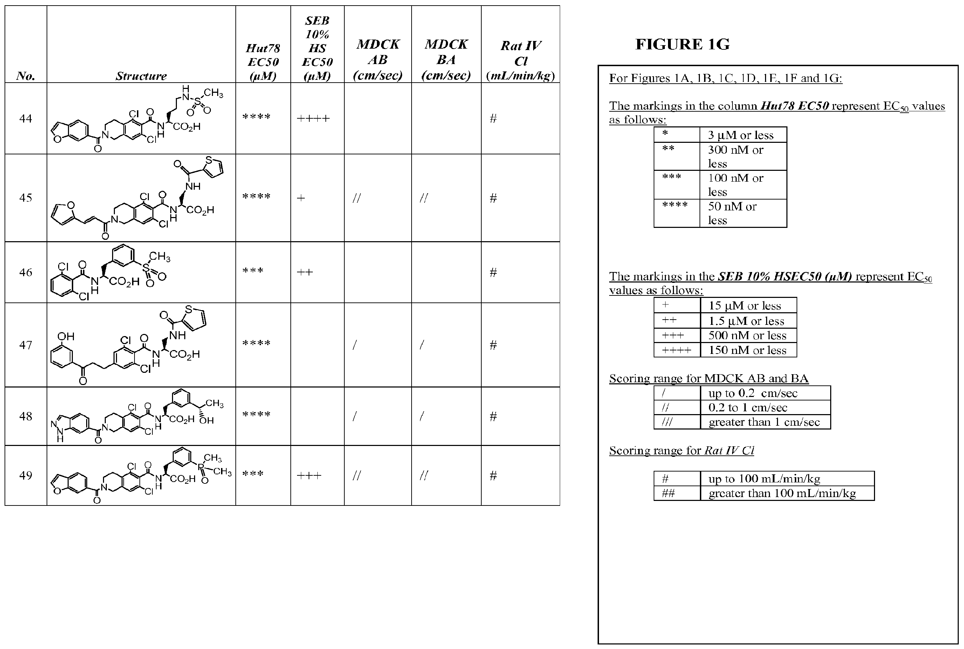

**Figure 1** shows the results of a lymphocyte adhesion inhibition assay and IL-2 release assay. For the inhibition assay, EC50 values were calculated for inhibition of binding between Jurkat T-cells and immobilized ICAM-1. For the IL-2 release assay, EC50 values were calculated for inhibition of IL-2 production from peripheral blood mononuclear cells following the addition of staph enterotoxin B antigen. This was done in the presence of 10% human serum. Compounds of structures No. 5, 7 ,9, 13, 15, 17, 19, 20, 23, 25, 27, 28, 30, 31, 34, 35, 38, 41, 44, and 48 relate to the invention. The remaining compounds are provided as reference examples.
**Figure 2** is a graphical representation of histopathological evaluation of biopsies taken before and after treatment of a dog eye with Compound 12.
**Figure 3** illustrates the mean change in Schirmer test score at weeks, 2, 4, 8, and 12 for eyes in dogs treated with Compound 12.
**Figure 4** illustrates percentage of dog eyes with a Schirmer test score of greater than 10 mm at 2, 4, 8, and 12 weeks with a formulation of 1% Compound 12 (TID; three times daily).
**Figure 5** illustrates percentage of eyes with a greater than 4 mm improvement in Schirmer test score at 2, 4, 12, 16, and 26 weeks for subjects treated with a formulation of 1% Compound 12 (TID) compared to literature results for 2% CsA (BID; two times daily).
**Figure 6** illustrates a timecourse of mean plasma levels of Compound 12 treatment (human) with 5% Compound 12.
**Figure 7** illustrates tear Cₘᵢₙ levels for human subjects treated with 1% Compound 12 QD (once a day).
**Figure 8** illustrates the dose/ drug Cₘₐₓ tear level relationship for administration of Compound 12 in humans (QD and TID).
**Figure 9** illustrates the dose/ AUC and dose/ mean Cₘₐₓ tear level relationship for human subjects treated QD with Compound 12.
**Figure 10** is a graphical representation of a whole body autoradiograph for a male Sprague Dawley Animal 0.5 hour after a single topical ocular administration of [¹⁴C]-Compound 12 (1 mg/eye).
**Figure 11** is a graphical representation of a whole-body autoradiograph for a male Sprague Dawley Animal 2 hours after a single topical ocular administration of [¹⁴C]-Compound 12(1 mg/eye).
**Figure 12** is a graphical representation of a whole-body autoradiograph for a male Sprague Dawley Animal 8 hours after a single topical ocular administration of [¹⁴C]-Compound 12(1 mg/eye).
**Figure 13** is a graphical representation of a whole-body autoradiograph for a male Sprague Dawley Animal 12 hours after a single topical ocular administration of [¹⁴C]-Compound 12 (1 mg/eye).
**Figure 14** is a graphical representation of a whole-body autoradiograph for a male Sprague Dawley Animal 24 hours after a single topical ocular administration of [¹⁴C]-Compound 12 (1 mg/eye).
**Figure 15** illustrates rat ocular pharmacokinetics of [¹⁴C]-Compound 12.
**Figure 16** illustrates dog ocular pharmacokinetics of [¹⁴C]-Compound 12.
**Figure 17** is a graphical representation of the timecourse of drug plasma levels for Compound 12 following single IV doses in rats.
**Figure 18** is a graphical representation of the timecourse of drug plasma levels for Compound 12 following single IV doses in dogs.
**Figure 19** illustrates the dose/drug AUC (in tears) relationship for Compound 12 administered to dogs.
**Figure 20** illustrates the drug tear concentration profiles of Compound 12 measured after 13 weeks of TID ocular dosing in rabbits.
**Figure 21** illustrates the drug tear concentration profiles of Compound 12 measured after 13 weeks of TID ocular dosing in dogs.
**Figure 22** illustrates mean drug tear concentrations in right and left eyes of rabbits following topical instillation of a single dose of Compound 12.
**Figure 23** illustrates the drug plasma level in rats for various topical applications of Compound 12.

### DETAILED DESCRIPTION OF THE INVENTION

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the appended claims define the scope of the invention.

Unless defined otherwise, all technical and scientific terms used herein have the same meaning as is commonly understood by one of skill in the art to which this invention belongs.

As used in the specification and claims, the singular form "a", "an" and "the" includes plural references unless the context clearly dictates otherwise.

As used herein, "agent" or "biologically active agent" refers to a biological, pharmaceutical, or chemical compound or other moiety. Non-limiting examples include simple or complex organic or inorganic molecule, a peptide, a protein, an oligonucleotide, an antibody, an antibody derivative, antibody fragment, a vitamin derivative, a carbohydrate, a toxin, or a chemotherapeutic compound. Various compounds is synthesized, for example, small molecules and oligomers (e.g., oligopeptides and oligonucleotides), and synthetic organic compounds based on various core structures. In addition, various natural sources can provide compounds for screening, such as plant or animal extracts. A skilled artisan can readily recognize that there is no limit as to the structural nature of the agents of the present invention.

The term "agonist" as used herein refers to a compound having the ability to initiate or enhance a biological function of a target protein, whether by inhibiting the activity or expression of the target protein. Accordingly, the term "agonist" is defined in the context of the biological role of the target polypeptide. While preferred agonists herein specifically interact with (e.g. bind to) the target, compounds that initiate or enhance a biological activity of the target polypeptide by interacting with other members of the signal transduction pathway of which the target polypeptide is a member are also specifically included within this definition.

The terms "antagonist" and "inhibitor" are used interchangeably, and they refer to a compound having the ability to inhibit a biological function of a target protein, whether by inhibiting the activity or expression of the target protein. Accordingly, the terms "antagonist" and "inhibitors" are defined in the context of the biological role of the target protein. While preferred antagonists herein specifically interact with (e.g. bind to) the target, compounds that inhibit a biological activity of the target protein by interacting with other members of the signal transduction pathway of which the target protein is a member are also specifically included within this definition. A preferred biological activity inhibited by an antagonist of LFA-1, for example, is associated with an undesired inflammatory or immune response as manifested in inflammatory or autoimmune disease, respectively.

A "directly competitive inhibitor" or "directly competitive antagonist" refers to a ligand, which includes biomolecules, peptides, and synthetic small organic molecules, which binds directly to the active site of the biological target molecule, and directly prevents a substrate from binding to it. For example, a directly competitive inhibitor of the interaction of LFA-1 and ICAM-1, binds to LFA-1 at the site where ICAM-1 binds, and thus directly prevents ICAM-1 from binding.

"Allosteric inhibitor" as used herein refers to a ligand which includes biomolecules, peptides, and synthetic small organic molecules, that binds to a biological target molecule at a site other than the binding site of the interaction which is being inhibited. The interaction changes the shape of the biological target molecule so as to disrupt the usual complex between the biological target molecule and its substrate. This results in inhibition of the normal activity of such complex formation. For example, an allosteric inhibitor of the interaction of LFA-1 and ICAM-1, binds to LFA-1 at a site other than that where ICAM-1 binds, but it disrupts the binding site of ICAM-1 such that the interaction of LFA-1 and ICAM-1 is reduced.

The term "selective inhibition" or "selectively inhibit" as applied to a biologically active agent refers to the agent's ability to selectively reduce the target signaling activity as compared to off-target signaling activity, via direct or interact interaction with the target.

"Th1" and "Th2" as used herein refere to helper T cells which are found in two distinct cell types, Th1 and Th2, distinguished by the cytokines they produce and respond to and the immune responses they are involved in. Th1 cells produce pro-inflammatory cytokines like IFN-g, TNF-b and IL-2, while Th2 cells produce the cytokines IL-4, IL-5, IL-6 and IL-13.

An "anti-cancer agent", "anti-tumor agent" or "chemotherapeutic agent" refers to any agent useful in the treatment of a neoplastic condition. One class of anti-cancer agents comprises chemotherapeutic agents. "Chemotherapy" means the administration of one or more chemotherapeutic drugs and/or other agents to a cancer patient by various methods, including intravenous, oral, intramuscular, intraperitoneal, intravesical, subcutaneous, transdermal, buccal, or inhalation or in the form of a suppository.

The term "cell proliferation" refers to a phenomenon by which the cell number has changed as a result of division. This term also encompasses cell growth by which the cell morphology has changed (e.g., increased in size) consistent with a proliferative signal.

The term "co-administration," "administered in combination with," and their grammatical equivalents, as used herein, encompasses administration of two or more agents to an animal so that both agents and/or their metabolites are present in the animal at the same time. Co-administration includes simultaneous administration in separate compositions, administration at different times in separate compositions, or administration in a composition in which both agents are present.

The term "effective amount" or "therapeutically effective amount" refers to that amount of a compound described herein that is sufficient to effect the intended application including but not limited to disease treatment, as defined below. The therapeutically effective amount may vary depending upon the intended application (*in vitro or in vivo*), or the subject and disease condition being treated, e.g., the weight and age of the subject, the severity of the disease condition, and the manner of administration, which can readily be determined by one of ordinary skill in the art. The term also applies to a dose that will induce a particular response in target cells, e.g. reduction of platelet adhesion and/or cell migration. The specific dose will vary depending on the particular compounds chosen, the dosing regimen to be followed, whether it is administered in combination with other compounds, timing of administration, the tissue to which it is administered, and the physical delivery system in which it is carried.

As used herein, "treatment" or "treating," or "palliating" or "ameliorating" are used interchangeably herein. These terms refers to an approach for obtaining beneficial or desired results including but not limited to therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made. The compositions may be administered to a subject to prevent progression of physiological symptoms or to prevent progression of the underlying disorder

A "therapeutic effect," as that term is used herein, encompasses a therapeutic benefit and/or a prophylactic benefit as described above. A prophylactic effect includes delaying or eliminating the appearance of a disease or condition, delaying or eliminating the onset of symptoms of a disease or condition, slowing, halting, or reversing the progression of a disease or condition, or any combination thereof.

As used herein, the term "pharmaceutically acceptable salt" refers to those salts which are suitable for pharmaceutical use, preferably for use in the tissues of humans and lower animals without undue irritation, and allergic response. Pharmaceutically acceptable salts of amines, carboxylic acids, and other types of compounds, are well known in the art. For example, S. M. Berge, et al., describe pharmaceutically acceptable salts in detail in J Pharmaceutical Sciences, 66: 1-19 (1977). The salts can be prepared in situ during the final isolation and purification of the compounds of the disclosure, or separately by reacting a free base or free acid function with a suitable reagent, as described generally below. For example, a free base function can be reacted with a suitable acid. Furthermore, where the compounds of the disclosure carry an acidic moiety, suitable pharmaceutically acceptable salts thereof may, include metal salts such as alkali metal salts, e. g. sodium or potassium salts; and alkaline earth metal salts, e.g. calcium or magnesium salts. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzoate, bisulfate, borate, butyrate, camphorate, camphorsulfonate, citrate, cyclopentanepropionate, digluconate, dodecylsulfate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, nicotinate, nitrate, oleate, oxalate, palmitate, pectinate, persulfate, 3-phenylpropionate, phosphate, picrate, pivalate, propionate, stearate, succinate, sulfate, tartrate, thiocyanate, p- toluenesulfonate, undecanoate, and valerate salts. Representative alkali or alkaline earth metal salts include sodium, lithium, potassium, calcium, and magnesium. Further pharmaceutically acceptable salts include, when appropriate, nontoxic ammonium, quaternary ammonium, and amine cations formed by direct reaction with the drug carboxylic acid or by using counterions such as halide, hydroxide, carboxylate, sulfate, phosphate, nitrate, sulfonate and aryl sulfonate.

"Pharmaceutically acceptable carrier" or "pharmaceutically acceptable excipient" includes any and all solvents, dispersion media, coatings, antibacterial and antifungal agents, isotonic and absorption delaying agents. The use of such media and agents for pharmaceutically active substances is well known in the art. Except insofar as any conventional media or agent is incompatible with the active ingredient, its use in the therapeutic compositions of the invention is contemplated. Supplementary active ingredients can also be incorporated into the compositions.

"Prodrug" is meant to indicate a compound that may be converted under physiological conditions or by solvolysis to a biologically active compound described herein. Thus, the term "prodrug" refers to a precursor of a biologically active compound that is pharmaceutically acceptable. A prodrug may be inactive when administered to a subject, i.e. an ester, but is converted *in vivo* to an active compound, for example, by hydrolysis to the free carboxylic acid. The prodrug compound often offers advantages of solubility, tissue compatibility or delayed release in a mammalian organism (*see, e.g.,* Bundgard, H., Design of Prodrugs (1985), pp. 7-9, 21-24 (Elsevier, Amsterdam). A discussion of prodrugs is provided in Higuchi, T., et al., "Pro-drugs as Novel Delivery Systems," A.C.S. Symposium Series, Vol. 14, and in Bioreversible Carriers in Drug Design, ed. Edward B. Roche, American Pharmaceutical Association and Pergamon Press, 1987. The term "prodrug" is also meant to include any covalently bonded carriers, which release the active compound in vivo when such prodrug is administered to a mammalian subject. Prodrugs of an active compound, as described herein, may be prepared by modifying functional groups present in the active compound in such a way that the modifications are cleaved, either in routine manipulation or in vivo, to the parent active compound. Prodrugs include compounds wherein a hydroxy, amino or mercapto group is bonded to any group that, when the prodrug of the active compound is administered to a mammalian subject, cleaves to form a free hydroxy, free amino or free mercapto group, respectively. Examples of prodrugs include, but are not limited to, acetate, formate and benzoate derivatives of an alcohol or acetamide, formamide and benzamide derivatives of an amine functional group in the active compound.

"Localized treatment" as used herein refers to treatment of an immune or inflammatory disorder wherein the drug is delivered locally and is not delivered via systemic delivery. This may include many different local areas or a few different local areas within, for example, the gastrointestinal tract to which drug is delivered to the gastrointestinal mucosa from within the lumen of the GI tract. Another example is treatment of skin, wherein the drug may be applied to many different locations or a few different locations on the skin, and wherein drug is delivered to tissues within and adjacent to the skin by absorption through the skin. Alternatively, drug may be delivered via suppository to anal mucosa and absorbed through the epithelial surfaces to tissue within and adjacent to the mucosa of the lower GI tract.

"Local delivery" as used herein refers to drug compound being carried to the site of therapeutic use. It includes, for example, applying a formulation directly to area of skin that is being treated, spraying a formulation to an area of skin being treated, spraying or inhaling a formulation intranasally to administer drug to the nasal passages, or instilling eye drops to an eye to treat the eye. In the present invention, "local delivery" also encompasses orally or nasally administering a formulation which is carried to the gastrointestinal tract, wherein the drug is brought in contact with the gastrointestinal mucosa, where the drug is absorbed into the surrounding tissue and exerts a therapeutic effect, without being directly delivered to that site from the blood circulatory system.

"Local tissue concentration" as used herein, refers to the concentration of LFA-1 antagonist within the tissue area to which the LFA-1 antagonist has been delivered and absorbed.

"Subject" refers to an animal, such as a mammal, for example a human. The methods described herein can be useful in both human therapeutics and veterinary applications. In some embodiments, the patient is a mammal, and in some embodiments, the patient is human.

The term "*in vivo*" refers to an event that takes place in a subject's body.

The term "*in vitro*" refers to an event that takes places outside of a subject's body. For example, an *in vitro* assay encompasses any assay run outside of a subject assay. *In vitro* assays encompass cell-based assays in which cells alive or dead are employed. In vitro assays also encompass a cell-free assay in which no intact cells are employed.

Unless otherwise stated, structures depicted herein are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon are within the scope of this invention.

The compounds of the present disclosure may also contain unnatural proportions of atomic isotopes at one or more of atoms that constitute such compounds. For example, the compounds may be radiolabeled with radioactive isotopes, such as for example tritium (³H), iodine-125 (¹²⁵I) or carbon-14 (¹⁴C). All isotopic variations of the compounds of the present disclosure, whether radioactive or not, are encompassed within the scope of the present disclosure.

When ranges are used herein for physical properties, such as molecular weight, or chemical properties, such as chemical formulae, all combinations and subcombinations of ranges and specific embodiments therein are intended to be included. The term "about" when referring to a number or a numerical range means that the number or numerical range referred to is an approximation within experimental variability (or within statistical experimental error), and thus the number or numerical range may vary from, for example, between 1% and 15% of the stated number or numerical range. The term "comprising" (and related terms such as "comprise" or "comprises" or "having" or "including") includes those embodiments, for example, an embodiment of any composition of matter, composition, method, or process, that "consist of' or "consist essentially of" the described features.

Abbreviations used herein have their conventional meaning within the chemical and biological arts.

The term "aliphatic", as used herein, includes both saturated and unsaturated, straight chain (unbranched) or branched aliphatic hydrocarbons, which are optionally substituted with one or more functional groups. As will be appreciated by one of ordinary skill in the art, "aliphatic" is intended herein to include, but is not limited to, alkyl, alkenyl, alkynyl moieties. Thus, as used herein, the term" alkyl" includes straight and branched alkyl groups. An analogous convention applies to other generic terms such as "alkenyl", and "alkynyl".

Furthermore, as used herein, the terms "alkyl", "alkenyl", and "alkynyl", encompass both substituted and unsubstituted groups. In certain embodiments, as used herein, "lower alkyl" is used to indicate those alkyl groups (substituted, unsubstituted, branched or unbranched) having about 1-6 carbon atoms.

In certain embodiments, the alkyl, alkenyl and alkynyl groups employed in the disclosure contain about 1-20 aliphatic carbon atoms. In certain other embodiments, the alkyl, alkenyl, and alkynyl groups employed in the disclosure contain about 1-10 aliphatic carbon atoms. In yet other embodiments, the alkyl, alkenyl, and alkynyl groups employed in the disclosure contain about 1-8 aliphatic carbon atoms. In still other embodiments, the alkyl, alkenyl, and alkynyl groups employed in the disclosure contain about 1-6 aliphatic carbon atoms. In yet other embodiments, the alkyl, alkenyl, and alkynyl groups employed in the disclosure contain about 1-4 carbon atoms. Illustrative aliphatic groups thus include, but are not limited to, for example, methyl, ethyl, n-propyl, isopropyl, allyl, n-butyl, sec- butyl, isobutyl, tert-butyl, n-pentyl, sec-pentyl, isopentyl, tert-pentyl, n-hexyl, and sec- hexyl, moieties, which again, may bear one or more substituents.

Alkenyl groups include, but are not limited to, for example, ethenyl, propenyl, and butenyl. Representative alkynyl groups include, but are not limited to, ethynyl, and 2-propynyl.

The term "lower alkylene" as used herein refers to a hydrocarbon chain which links together two other groups, i.e. is bonded to another group at either end, for example methylene, ethylene, and butylene. Such a substituent is preferably from 1 to 10 carbons and more preferably from 1 to 5 carbons. Such groups may be substituted, preferably with an amino, acetylamino (a lower alkylcarbonyl group bonded via a nitrogen atom), or cyclo lower alkyl group. By the latter is meant a saturated hydrocarbon ring, preferably with a total of 3 to 10 methylenes (inclusive of the attachment carbons), more preferably 3 to 6.

The term "alicyclic", as used herein, refers to compounds which combine the properties of aliphatic and cyclic compounds and include but are not limited to monocyclic, or polycyclic aliphatic hydrocarbons and bridged cycloalkyl compounds, which are optionally substituted with one or more functional groups.

As will be appreciated by one of ordinary skill in the art, "alicyclic" is intended herein to include, but is not limited to, cycloalkyl, cycloalkenyl, and cycloalkynyl moieties, which are optionally substituted with one or more functional groups.

Illustrative alicyclic groups thus include, but are not limited to, for example, cyclopropyl,-CH₂-cyclopropyl, cyclobutyl, -CH₂-cyclobutyl, cyclopentyl,-CH₂- cyclopentyl, cyclohexyl,-CH₂-cyclohexyl, cyclohexenylethyl, cyclohexanylethyl, and norbornyl moieties, which again, may bear one or more substituents.

The term "alkoxy" or "alkyloxy", as used herein refers to a saturated or unsaturated parent molecular moiety through an oxygen atom. In certain embodiments, the alkyl group contains about 1-20 aliphatic carbon atoms. In certain other embodiments, the alkyl group contains about 1-10 aliphatic carbon atoms. In yet other embodiments, the alkyl group employed in the disclosure contains about 1-8 aliphatic carbon atoms. In still other embodiments, the alkyl group contains about 1-6 aliphatic carbon atoms. In yet other embodiments, the alkyl group contains about 1-4 aliphatic carbon atoms. Examples of alkoxy, include but are not limited to, methoxy, ethoxy, isopropoxy, n-butoxy, i-butoxy, sec-butoxy, tert-butoxy, neopentoxy, and n- hexloxy.

The term "lower alkoxy" as used herein refers to a lower alkyl as defined above which may be branched or unbranched as also defined above and which is bonded by an oxygen to another group (i.e. alkyl ethers).

The term "alkylamino" refers to a group having the structure- NHR' wherein R' is alkyl, as defined herein. The term "aminoalkyl" refers to a group having the structure NH₂R'-, wherein as defined herein. In certain embodiments, the alkyl group contains about 1-20 aliphatic carbon atoms. In certain other embodiments, the alkyl group contains about 1-10 aliphatic carbon atoms. In yet other embodiments, the alkyl group employed in the disclosure contains about aliphatic carbon atoms. In still other embodiments, the alkyl group contains about 1-6 aliphatic carbon atoms. In yet other embodiments, the alkyl group contains about 1-4 aliphatic carbon atoms. Examples of alkylamino include, but are not limited to, methylamino.

Some examples of substituents of the above-described aliphatic (and other) moieties of compounds of the disclosure include, but are not limited to aliphatic; alicyclic; heteroaliphatic; heterocyclic; aromatic; heteroaromatic; aryl; heteroaryl; alkylaryl; heteroalkylaryl ; alkylheteroaryl; heteroalkylheteroaryl; alkoxy; aryloxy; heteroalkoxy ; heteroaryloxy; alkylthio; arylthio; heteroalkylthio ; Rₓ independently includes, but is not limited to, aliphatic, alicyclic, heteroaliphatic, heterocyclic, aryl, heteroaryl, alkylaryl, alkylheteroaryl, heteroalkylaryl or heteroalkylheteroaryl, wherein any of the aliphatic, alicyclic, heteroaliphatic, heterocyclic, alkylaryl, or alkylheteroaryl substituents described above and herein may be substituted or unsubstituted, branched or unbranched, saturated or unsaturated, and wherein any of the aryl or heteroaryl substituents described above and herein may be substituted or unsubstituted. Additional examples of generally applicable substituents are illustrated by the specific embodiments shown in the Examples that are described herein.

In general, the term "aromatic moiety", as used herein, refers to a stable mono-or polycyclic, unsaturated moiety having preferably 3-14 carbon atoms, each of which maybe substituted or unsubstituted. In certain embodiments, the term "aromatic moiety" refers to a planar ring having p-orbitals perpendicular to the plane of the ring at each ring atom and satisfying the Huckel rule where the number of pi electrons in the ring is (4n+2) wherein n is an integer. A mono-or polycyclic, unsaturated moiety that does not satisfy one or all of these criteria for aromaticity is defined herein as "non-aromatic", and is encompassed by the term "alicyclic".

In general, the term "heteroaromatic moiety", as used herein, refers to a stable mono-or polycyclic, unsaturated moiety having preferably 3-14 carbon atoms, each of which may be substituted or unsubstituted; and comprising at least one heteroatom selected from O, S, and N within the ring in place of a ring carbon atom). In certain embodiments, the term "heteroaromatic moiety" refers to a planar ring comprising at least one heteroatom, having p-orbitals perpendicular to the plane of the ring at each ring atom, and satisfying the Huckel rule where the number of pi electrons in the ring is (4n+2) wherein n is an integer.

It will also be appreciated that aromatic and heteroaromatic moieties, as defined herein may be attached via an alkyl or heteroalkyl moiety and thus also include- (alkyl) aromatic,- (heteroalkyl) aromatic,- (heteroalkyl) heteroaromatic, and - (heteroalkyl) heteroaromatic moieties. Thus, as used herein, the phrases"aromatic or heteroaromatic moieties "and" aromatic, (heteroalkyl) aromatic,- (heteroalkyl) heteroaromatic, and (heteroalkyl) heteroaromatic" are interchangeable. Substituents include, but are not limited to, any of the previously mentioned substituents, e.g., the substituents recited for aliphatic moieties, or for other moieties as disclosed herein, resulting in the formation of a stable compound.

The term "aryl", as used herein, does not differ significantly from the common meaning of the term in the art, and refers to an unsaturated cyclic moiety comprising at least one aromatic ring. In certain embodiments, "aryl" refers to a mono-or bicyclic carbocyclic ring system having one or two aromatic rings including, but not limited to, phenyl, naphthyl, tetrahydronaphthyl, indanyl, and indenyl.

The term "heteroaryl" as used herein, does not differ significantly from the common meaning of the term in the art, and refers to a cyclic aromatic radical having from five to ten ring atoms of which one ring atom is selected from S, and N; zero, one or two ring atoms are additional heteroatoms independently selected from S, and N; and the remaining ring atoms are carbon, the radical being joined to the rest of the molecule via any of the ring atoms, such as, for example, pyridyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, and isoquinolinyl.

It will be appreciated that aryl and heteroaryl groups (including bicyclic aryl groups) can be unsubstituted or substituted, wherein substitution includes replacement of one or more of the hydrogen atoms thereon independently with any one or more of the following moieties including, but not limited to: aliphatic; alicyclic; heteroaliphatic; heterocyclic; aromatic; heteroaromatic; aryl; heteroaryl; alkylaryl; heteroalkylaryl; alkylheteroaryl; heteroalkylheteroaryl; alkoxy; aryloxy ; heteroalkoxy; heteroaryloxy; alkylthio; arylthio; heteroalkylthio; heteroarylthio; F; Cl; Br; I; -OH; -NO₂; -CN; -CF₃; - CH₂CF₃; -CHCl₂; -CH₂OH; -CH₂CH₂OH; -CH₂NH₂; -CH₂SO₂CH₃; -C(=O)Rₓ; -C(=O)N(Rₓ)₂; -OC(=O)Rₓ; -OCO₂Rₓ; - OC(=O)N(Rₓ)₂; -N(Rₓ)₂; -S(O)₂Rₓ; -NRₓ(CO)Rₓ wherein each occurrence of Rₓ independently includes, but is not limited to, aliphatic, alicyclic, heteroaliphatic, heterocyclic, aromatic, heteroaromatic, aryl, heteroaryl, alkylaryl, alkylheteroaryl, heteroalkylaryl or heteroalkylheteroaryl wherein any of the aliphatic, alicyclic, heteroaliphatic, heterocyclic, alkylaryl, or alkylheteroaryl substituents described above and herein may be substituted or unsubstituted, branched or unbranched, saturated or unsaturated, and wherein any of the aromatic, heteroaromatic, aryl, heteroaryl,- (alkyl) aryl or- (alkyl) heteroaryl substituents described above and herein may be substituted or unsubstituted. Additionally, it will be appreciated, that any two adjacent groups taken together may represent a 4, 5, 6, or 7-membered substituted or unsubstituted alicyclic or heterocyclic moiety. Additional examples of generally applicable substituents are illustrated by the specific embodiments shown in the Examples that are described herein.

The term "cycloalkyl", as used herein, refers specifically to groups having three to seven, preferably three to ten carbon atoms. Suitable cycloalkyls include, but are not limited to cyclopropyl, cyclobutyl, cyclopentyl, cyclohexyl, and cycloheptyl, which, as in the case of aliphatic, alicyclic, heteroaliphatic or heterocyclic moieties, may optionally be substituted with substituents including, but not limited to aliphatic; alicyclic; heteroaliphatic; heterocyclic; aromatic ; heteroaromatic; aryl; heteroaryl; alkylaryl; heteroalkylaryl; alkylheteroaryl ; heteroalkylheteroaryl; alkoxy; aryloxy ; heteroalkoxy; heteroaryloxy ; alkylthio; heteroarylthio; F; Cl; Br; I; -OH; -NO₂; -CN; -CF₃; -CH₂CF₃; -CHCl₂; -CH₂OH; - CH₂CH₂OH; -CH₂NH₂; -CH₂SO₂CH₃; -C(=O)Rₓ; -C(=O)N(Rₓ)₂; -OC(=O)Rₓ; -OCO₂Rₓ; -OC(=O)N(Rₓ)₂; -N(Rₓ)₂; -S(O) ₂Rₓ; -NRₓ(CO)Rₓ wherein each occurrence of Rₓ independently includes, but is not limited to, aliphatic, alicyclic, heteroaliphatic, heterocyclic, aromatic, heteroaromatic, aryl, heteroaryl, alkylaryl, alkylheteroaryl, heteroalkylaryl or heteroalkylheteroaryl, wherein any of the aliphatic, alicyclic, heteroaliphatic, heterocyclic, alkylaryl, or alkylheteroaryl substituents described above and herein may be substituted or unsubstituted, branched or unbranched, saturated or unsaturated, and wherein any of the aromatic, heteroaromatic, aryl or heteroaryl substituents described above and herein may be substituted or unsubstituted. Additional examples of generally applicable substituents are illustrated by the specific embodiments shown in the Examples that are described herein.

The term "heteroaliphatic", as used herein, refers to aliphatic moieties in which one or more carbon atoms in the main chain have been substituted with a heteroatom. Thus, a heteroaliphatic group refers to an aliphatic chain which contains one or more oxygen, sulfur, nitrogen, phosphorus or silicon atoms, e. place of carbon atoms. Heteroaliphatic moieties may be linear or branched, and saturated or unsaturated. In certain embodiments, heteroaliphatic moieties are substituted by independent replacement of one or more of the hydrogen atoms thereon with one or more moieties including, but not limited to aliphatic; alicyclic; heteroaliphatic; heterocyclic; aromatic; heteroaromatic; aryl; heteroaryl; alkylaryl ; alkylheteroaryl; alkoxy; aryloxy; heteroalkoxy; heteroaryloxy ; alkylthio ; arylthio ; heteroarylthio; F; Cl; Br; I; -OH; -NO₂; -CN; -CF₃; -CH₂CF₃; -CHCl₂; -CH₂OH; -CH₂CH₂OH; - CH₂NH₂; -CH₂SO₂CH₃; -C(=O)Rₓ; -C(=O)N(Rₓ)₂; -OC(=O)Rₓ; -OCO₂Rₓ; -OC(=O)N(Rₓ)₂; -N(Rₓ)₂; -S(O)₂Rₓ; - NRₓ(CO)Rₓ wherein each occurrence of Rₓ independently includes, but is not limited to, aliphatic, alicyclic, heteroaliphatic, heterocyclic, aromatic, heteroaromatic, aryl, heteroaryl, alkylaryl, alkylheteroaryl, heteroalkylaryl or heteroalkylheteroaryl, wherein any of the aliphatic, alicyclic, heteroaliphatic, heterocyclic, alkylaryl, or alkylheteroaryl substituents described above and herein may be substituted or unsubstituted, branched or unbranched, saturated or unsaturated, and wherein any of the aromatic, heteroaromatic, aryl or heteroaryl substituents described above and herein may be substituted or unsubstituted. Additional examples of generally applicable substituents are illustrated by the specific embodiments shown in the Examples that are described herein.

The term "heterocycloalkyl", "heterocycle" or "heterocyclic", as used herein, refers to compounds which combine the properties of heteroaliphatic and cyclic compounds and include, but are not limited to, saturated and unsaturated mono-or polycyclic cyclic ring systems having 5-16 atoms wherein at least one ring atom is a heteroatom selected from S and N (wherein the nitrogen and sulfur heteroatoms may be optionally be oxidized), wherein the ring systems are optionally substituted with one or more functional groups, as defined herein. In certain embodiments, the term "heterocycloalkyl", "heterocycle" or "heterocyclic" refers to a non-aromatic 5-, 6-or 7-membered ring or a polycyclic group wherein at least one ring atom heteroatom selected from S and N (wherein the nitrogen and sulfur heteroatoms may be optionally be oxidized), including, but not limited to, a bi-or tri-cyclic group, comprising fused six-membered rings having between one and three heteroatoms independently selected from oxygen, sulfur and nitrogen, wherein (i) each 5-membered ring has 0 to 2 double bonds, each 6-membered ring has 0 to 2 double bonds and each 7-membered ring has 0 to 3 double bonds, (ii) the nitrogen and sulfur heteroatoms may be optionally be oxidized, (iii) the nitrogen heteroatom may optionally be quaternized, and (iv) any of the above heterocyclic rings may be fused to an aryl or heteroaryl ring. Representative heterocycles include, but are not limited to, heterocycles such as furanyl, pyranyl, pyrrolyl, thienyl, pyrrolidinyl, pyrazolinyl, pyrazolidinyl, imidazolinyl, imidazolidinyl, piperidinyl, piperazinyl, oxazolyl, oxazolidinyl, isooxazolyl, isoxazolidinyl, dioxazolyl, thiadiazolyl, oxadiazolyl, tetrazolyl, triazolyl, thiatriazolyl, thiadiazolyl, oxadiazolyl, morpholinyl, thiazolyl, thiazolidinyl, isothiazolyl, isothiazolidinyl, dithiazolyl, dithiazolidinyl, tetrahydrofuryl, and benzofused derivatives thereof. In certain embodiments, a "substituted heterocycle, or heterocycloalkyl or heterocyclic "group is utilized and as used herein, refers to a heterocycle, or heterocycloalkyl or heterocyclic group, as defined above, substituted by the independent replacement of one, two or three of the hydrogen atoms thereon with but are not limited to aliphatic; alicyclic ; heteroaliphatic; heterocyclic; aromatic; heteroaromatic; aryl; heteroaryl; alkylaryl ; heteroalkylaryl; alkylheteroaryl ; heteroalkylheteroaryl; alkoxy; aryloxy; heteroalkoxy; heteroaryloxy; alkylthio; arylthio ; heteroalkylthio; heteroarylthio; F; Cl; Br; I; -OH; -NO₂; -CN; -CF₃; -CH₂CF₃; -CHCl₂; -CH₂OH; -CH₂CH₂OH; - CH₂NH₂; -CH₂SO₂CH₃; -C(=O)Rₓ; -C(=O)N(Rₓ)₂; -OC(=O)Rₓ; -OCO₂Rₓ; -OC(=O)N(Rₓ)₂; -N(Rₓ)₂; -S(O)₂Rₓ; - NRₓ(CO)Rₓ wherein each occurrence of Rₓ independently includes, but is not limited to, aliphatic, alicyclic, heteroaliphatic, heterocyclic, aromatic, heteroaromatic, aryl, heteroaryl, alkylaryl, alkylheteroaryl, heteroalkylaryl or heteroalkylheteroaryl, wherein any of the aliphatic, alicyclic, heteroaliphatic, heterocyclic, alkylaryl, or alkylheteroaryl substituents described above and herein may be substituted or unsubstituted, branched or unbranched, saturated or unsaturated, and wherein any of the aromatic, heteroaromatic, aryl or heteroaryl described above and herein may be substituted or unsubstituted. Additionally, it will be appreciated that any of the alicyclic or heterocyclic moieties described above and herein may comprise an aryl or heteroaryl moiety fused thereto.

The terms "halo" and "halogen" used herein refer to an atom selected from fluorine, chlorine, bromine and iodine.

The term "haloalkyl" denotes an alkyl group, as defined above, having one, two, or three halogen atoms attached thereto and is exemplified by such groups as chloromethyl, bromoethyl, and trifluoromethyl.

The term "amino" as used herein, refers to a primary (-NH₂), secondary (-NHRₓ), tertiary (-NRₓR_{y}), or quaternary amine (-N⁺RₓR_{y}R_{z}), where R_{y} and R_{z} are independently an aliphatic, alicyclic, heteroaliphatic, heterocyclic, aromatic or heteroaromatic moiety, as defined herein. Examples of amino groups include, but are not limited to, methylamino, dimethylamino, ethylamino, diethylamino, diethylaminocarbonyl, iso-propylamino, piperidino, trimethylamino, and propylamino.

The term "acyl", as used herein, refers to a group having the general formula -C(=O)R, where R is an aliphatic, alicyclic, heteroaliphatic, heterocyclic, aromatic or heteroaromatic moiety, as defined herein.

The term "sulfonamido" as used herein, refers to a group of the general formula -SO2NRxRy where Rx and Ry are independently hydrogen, or an aliphatic, alicyclic, heteroaliphatic, heterocyclic, aromatic, heteroaromatic or acyl moiety, as defined herein.

The term "benzamido", as used herein, refers to a group of the general formula PhNRx, where Rx is hydrogen, or an aliphatic, alicyclic, heteroaliphatic, heterocyclic, aromatic, heteroaromatic or acyl moiety, as defined herein.

As used herein, the terms "aliphatic", "heteroaliphatic", "alkyl", "alkenyl", "alkynyl", "heteroalkyl" ,"heteroalkenyl", and "heteroalkynyl", encompass substituted and unsubstituted, saturated and unsaturated, and linear and branched groups. Similarly, the terms, "alicyclic", "heterocyclic", heterocycloalkyl", and "heterocycle", encompass substituted and unsubstituted, and saturated and unsaturated groups. Additionally, the terms "cycloalkyl", cycloalkenyl", cycloalkynyl", "heterocycloalkyl", "heterocycloalkenyl", "heterocycloalkynyl", "aromatic", "heteroaromatic", "aryl", and "heteroaryl" encompass both substituted and unsubstituted groups.

The term "natural amino acid" as used herein refers to any one of the common, naturally occurring L-amino acids found in naturally occurring proteins : glycine (Gly), alanine (Ala), valine (Val), leucine (Leu), isoleucine (Ile), lysine (Lys), arginine (Arg), histidine (His), proline (Pro), serine (Ser), threonine (Thr), phenylalanine (Phe), tyrosine (Tyr), tryptophan (Trp), aspartic acid (Asp), glutamic acid (Glu), asparagine (Asn), glutamine (Gln), cysteine (Cys) and methionine (Met).

The term "unnatural amino acid"as used herein refers to all amino acids which are not natural amino acids. This includes, for example, α-, β-, D-, L-amino acid residues, and compounds of the general formula: 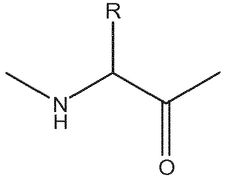 wherein the side chain R is other than the amino acid side chains occurring in nature.

More generally, the term "amino acid", as used herein, encompasses natural amino acids and unnatural amino acids.

The present invention provides formulated LFA-1 antagonists or pharmaceutically acceptable salts thereof that are suitable for topical delivery. In particular, the LFA-1 antagonists are particularly well suited for localized treatment by having a rapid systemic clearance rate. The invention also encompasses LFA-1 topical formulations of the present invention for use in methods of treatment and prevention of immune related disorders using said LFA-1 topical formulations of the present invention. Advantages of localized LFA-1 antagonist therapy delivered topically include delivery of a higher concentration of active compound to the site of interest, rapid delivery of the active compound and decreased systemic effects due to lower systemic circulating levels.

Various aspects of the invention are described in further detail in the following subsections.

### LFA-1 Antagonist compositions for localized topical treatment

The present invention includes formulations for localized treatment of immune related disorders. The formulations comprise an LFA-1 antagonist in a composition suitable for topical delivery to a subject. Compositions may include gel, cream, lotion, solution, suspension, emulsion, ointment, powder, crystalline forms, spray, foam, salve, paste, plaster, paint, microparticle, nanoparticle, and bioadhesive. Formulations may further include additional ingredients such as ingredients to facilitate delivery of the active compounds, enhance the therapeutic effect, have a secondary effect or minimize side effects. The formulations of the present invention are more fully described below.

The topical formulations of the present invention contain an LFA-1 antagonist as a therapeutic agent. In a preferred embodiment of the disclosure, the LFA-1 antagonists of the present disclosure have a rapid systemic clearance rate. LFA-1 interaction with ICAMs exert various systemic effects throughout the body. Treatment of a disorder using an LFA-1 antagonist may result in unwanted effects due to LFA-1 antagonist activity in unwanted locations, for example, other than at the site of administration. The present invention utilizes LFA-1 antagonists which are cleared quickly from systemic circulation. By utilizing topical delivery to the site of an inflammatory or immune disorder, unwanted systemic effects are minimized while still allowing for localized treatment. The LFA-1 antagonists of the present disclosure typically have minimal systemic LFA-1 antagonist activity. In some embodiments, the LFA-1 antagonists of the present disclosure may have undetectable systemic LFA-1 antagonist activity.

The systemic clearance rate can be calculated by various means known in the art. For example, the clearance rate for a drug may be calculated from an analysis of the drug concentration time profile for the drug concentration time profile for the rate of disappearance of a drug from the plasma following administration of the formulation, for example after a single intravenous injection. One of skill in the art could use a variety of methods to calculate and determine systemic clearance rates. For example, the rate of disappearance may be measured by analysis of the absorption, distribution, metabolism and excretion of a radiolabelled form of a drug or other means of measuring the level of drug in plasma, such as gas chromatography (Sapirstein et al., 1955, Am. Jour. Physiol., Vol. 181, pp. 330; U.S. Patent No. 4,908,202), liquid chromatography-mass spectrometry methods (LCMS) or HPLC methods. As another example, the clearance rate may be calculated by introducing the formulation to the subject by continuous intravenous infusion until an equilibrium is reached at which the plasma level of the substance (as determined by analysis of plasma samples) is steady, at which point the infusion rate is equal to the rate of clearance from plasma (Earle et al., 1946, Proc. Soc. Exp. Biol. Med., Vol. 62, pp. 262 ff.)

Rapid systemic clearance may be through clearance or metabolism in the liver, kidney or other organs. Data for rate of clearance through the liver in rats is given for selected compounds in **Figure 1** (see also Example 11). Where clearance occurs in a particular organ, the clearance rate is related to the blood flow to that particular organ. By knowing the mechanism in which a compound is cleared for a particular species, the clearance rate for other animals may be calculated by allometric scaling. For example, a compound of the present disclosure, Compound 12, is known to be cleared through the liver in rats. Based on the rate of clearance calculated in rat, the clearance of the compound may be scaled for various animals based on the known blood flow in rats compared to other animals (see Davies and Morris, "Physiological Parameters in Laboratory Animals and Humans" Pharmaceutical Research (1993) 10:1093-5). An LFA-1 antagonist of the present disclosure may have a systemic clearance rate approaching cardiac output, hepatic blood flow or kidney blood flow when scaled to a human. The scaling may be based on percent of cardiac output, hepatic blood flow or kidney blood. For example, 100% of rat hepatic blood flow would be approximately 55 mL/min/Kg while 100% of human hepatic blood flow would be approximately 20 mL/min/kg. In some embodiments, the compositions of the invention have a clearance rate of at least 5% of hepatic blood flow. In humans, this would mean a clearance rate of 1 mL/min/kg. In other embodiments, the LFA-1 antagonist has a clearance rate of at least about 10%, 20%, 30%, 40%, 50%, 60%, 70%, 80%, 90% or 100% of hepatic blood flow rate in humans (which would be a clearance rate in human liver of 20 mL/min/kg). In yet other embodiments, the LFA-1 antagonist has a clearance rate of at least about 110%, 120%, 130%, 140%, 150%, 175%, 200%, 220%, 240%, 260%, 280%, 300%, 320%, 340%, 360%, 380%, 400%, 420%, 440%, 460%, 480%, or 500% of hepatic blood flow rate in humans.

The clearance rates of the present invention may include clearance rates scaled to humans of approximately 1-500 mL/min/kg. In some embodiments, the LFA-1 antagonist may have a systemic clearance rate of approximately 1 mL/min/kg or greater. In other embodiments, the LFA-1 antagonist may have a systemic clearance rate of approximately 2 mL/min/kg or greater. In other embodiments, the LFA-1 antagonist may have a systemic clearance rate of approximately 3 mL/min/kg or greater. In other embodiments, the LFA-1 antagonist may have a systemic clearance rate of approximately 5 mL/min/kg or greater. In other embodiments, the LFA-1 antagonist may have a systemic clearance rate of approximately 7 mL/min/kg or greater. In some embodiments, the LFA-1 antagonist may have a systemic clearance rate of approximately 10 mL/min/kg or greater. In other embodiments, the LFA-1 antagonist may have a systemic clearance rate of approximately 15 mL/min/kg or greater. In other embodiments, the LFA-1 antagonist may have a systemic clearance rate of approximately 20 mL/min/kg or greater. In other embodiments, the LFA-1 antagonist may have a systemic clearance rate of approximately 25 mL/min/kg or greater. In some embodiments, the LFA-1 antagonist may have a systemic clearance rate of approximately 30 mL/min/kg or greater. In some embodiments, the LFA-1 antagonist may have a systemic clearance rate of approximately 40 mL/min/kg or greater. In other embodiments, the LFA-1 antagonist may have a systemic clearance rate of approximately 50 mL/min/kg or greater. In yet other embodiments, the LFA-1 antagonist may have a systemic clearance rate of at least about 60, 65, 70, 75, 80, 85, 90, 95, or 100 mL/min/kg.

In another aspect of the disclosure, the LFA-1 antagonist of the present disclosure has an inhibitory effect on LFA-1 binding to ICAM-1. The inhibitory effect of the LFA-1 antagonists of the present disclosure may be tested using any of a variety of known binding assays in the art, including direct cell binding to ICAM-1 coated plates, enzyme-linked immunoadsorbant assay (ELISA), radioimmunoassay (RIA) or the use of biosensors. The inhibitory effect of a drug is typically measured as an IC50 value, which measures how much compound is required to inhibit 50% of a biological process. Alternatively, the inhibitory effect may be calculated as an EC50 value, which measures the effective concentration by which the drug functions to achieve 50% of the desired effect. For example, the EC50 value could be measured to calculate inhibition of LFA-1 expressing T-cells from binding to ICAM-1. For example, T-cell lines known to express LFA-1 maybe used to calculate an IC50 value by inhibition of binding to ICAM-1 coated plates. As an example, the T-cell line HuT78 (ATCC TIB-161) maybe bound to ICAM-1 coated plates in the presence of increasing concentrations of an LFA-1 antagonist (see Example 1). In some embodiments, the LFA-1 antagonist is a directly competitive inhibitor of the interaction between LFA-1 and ICAM-1. Examples of competitive binding experiments for LFA-1 antagonists are described in the art, for example, U.S. Patent Application No. 2005/0148588 and US Provisional Application No. 60/999,571; Gadek et al. Science 295, 1086-1089, 2002, and Keating et al Protein Science, 15, 290-303, 2006. The EC50 or IC50 may be used in embodiments described below. Such assays can be used to identify inhibitors that are directly competitive inhibitors.

In some embodiments, the LFA-1 antagonist inhibits HuT78 cellular binding to ICAM-1 coated plates with an EC50 of 10 µM or less. In other embodiments, the LFA-1 antagonist inhibits HuT78 or Jurkat cellular binding to ICAM-1 coated plates with an EC50 of 1 µM or less. Alternatively, the LFA-1 antagonist inhibits HuT78 or Jurkat cellular binding to ICAM-1 coated plates with an EC50 of 100 nM or less. In some other embodiments, the LFA-1 antagonist inhibits HuT78 or Jurkat cellular binding to ICAM-1 coated plates with an EC50 of 10, 5 or 1 nM or less. Data for the inhibition of HuT78 cellular binding to ICAM-1 for selected LFA-1 antagonists of Formula I and Formula II are shown in **Figure 1****.**

The inhibitory effect of the LFA-1 antagonists of the present disclosure may also be tested using known downstream events following binding of LFA-1 to ICAM-1. For example, it is known that IL-2 is released from human T-cells in primary culture following stimulation by the superantigen staph enterotoxin B (SEB) or other inflammatory stimuli.

In one embodiment, the LFA-1 antagonist inhibits IL-2 release from peripheral blood mononuclear cells (PBMCs) in primary culture stimulated with SEB with an IC50 or EC50 of 10 mM or less. In another embodiment, the LFA-1 antagonist inhibits IL-2 release from peripheral blood mononuclear cells (PBMCs) in primary culture stimulated with SEB with an IC50 or EC50 of 1 mM or less. In yet other embodiments, the LFA-1 antagonist inhibits IL-2 release from peripheral blood mononuclear cells (PBMCs) in primary culture stimulated with SEB with an IC50 or EC50 of 100 µM or less. In some embodiments, the LFA-1 antagonist inhibits IL-2 release from peripheral blood mononuclear cells (PBMCs) in primary culture stimulated with SEB with an IC50 or EC50 of 10 µM or less. The disclosure provides other embodiments wherein the LFA-1 antagonist inhibits IL-2 release from peripheral blood mononuclear cells (PBMCs) in primary culture stimulated with SEB with an IC50 or EC50 of 1 µM, 100nM, 10nM or 1 nM or less. In some embodiments, the LFA-1 antagonist simultaneously inhibits the release of two or more inflammatory cytokines with IC50 or EC50's of 1 µM or less when PBMC's are stimulated with SEB. The LFA-1 antagonist may also simultaneously inhibit the release of two or more cytokines with IC50 or EC50's of 100 nM or less when PBMC's are stimulated with SEB. In further embodiments, the LFA-1 antagonist simultaneously inhibits the release of IL-2 and IL-4 with IC50 or EC50's of 500 nM or less when PBMC's are stimulated with SEB. This is particularly important since IL-2 and IL-4 release play important roles in Th1 and Th2 lymphocyte mediated inflammatory diseases. In yet another embodiment, the LFA-1 antagonist simultaneously inhibits the release of IL-1 (alpha), IL-1 (beta), IL-2, IL-4, IL-5, IL-10, IL-13, Interferon gamma, MIP 1(alpha), MCP-1, TNF(alpha) and GM-CSF with IC50 or EC50's of 1 µM or less when PBMC's are stimulated with SEB.

The LFA-1 antagonist is delivered such that a local therapeutically effective concentration is achieved. For example, the therapeutically effective concentration may be achieved with a local tissue concentration of LFA-1 of greater than about 1 nM. In another embodiment, the local therapeutically effective concentration may be achieved with a local tissue concentration of LFA-1 of greater than about 10 nM. In some other embodiments, the local therapeutically effective concentration may be achieved with a local tissue concentration of LFA-1 of greater than about 100 nM. In yet another embodiment, the local therapeutically effective concentration maybe achieved with a local tissue concentration of LFA-1 of greater than about 1 µM. In other embodiments, the local therapeutically effective concentration may be achieved with a local tissue concentration of LFA-1 of greater than about 10 µM. In another embodiment, the local therapeutically effective concentration of is achieved while maintaining a low systemic level. For example, in some embodiments, a local therapeutically effective concentration of about 1 nM, about 10 nM, about 100 nM, about 1 µM, or about 10 µM is achieved while maintaining a systemic drug concentration of less than 1 µM. In other embodiments, a local therapeutically effective concentration of about 1 nM, about 10 nM, about 100 nM, about 1 µM, or about 10 µM is achieved while maintaining a systemic drug concentration of less than 100 nM. In yet other embodiments, a therapeutically effective concentration of about 1 nM, about 10 nM, about 100 nM, about 1 µM, or about 10 µM is achieved while maintaining a systemic drug concentration of less than 10 nM. The invention provides other embodiments wherein a therapeutically effective concentration of about 1 nM, about 10 nM, about 100 nM, about 1 µM, or about 10 µM is achieved with a systemic drug concentration of less than 1 nM. The systemic drug concentration may be measured by blood plasma concentration using any of a variety of methods known in the art and as disclosed above.

In another aspect of the invention, the local tissue concentration of LFA-1 antagonist is maintained at therapeutically effective levels for an extended period of time. In some embodiments, it may be desired that local tissue concentrations of an LFA-1 antagonist is maintained at therapeutically effective levels for a certain amount of time or between doses. By selecting for LFA-1 antagonists that can maintain local therapeutically effective levels for extended periods, the subject may achieve a therapeutic effect without administration of multiple doses per day. For example, LFA-1 antagonists of the present disclosure, when delivered to the eye in an approximately 1% solution, may be present at local tissue concentration levels above about 1 µM for 16- 24 hours post dose, a timeperiod considered sufficient for a claim of once daily administration of an ophthalmic drug. A local administration of an LFA-1 antagonist of the present disclosure when delivered to the skin as an approximately 1% solution, gel, ointment, or cream can provide local tissue concentration levels in the epidermis and dermis above 1
µM for 24 hours. The local tissue concentration level may be measured by any of a variety of methods known in the art, such as radiolabelled analysis.

In some embodiments, the LFA-1 antagonist has a local tissue concentration of greater than about 1 µM for at least about 2 hours, about 4 hours, about 6 hours, about 8 hours, about 10 hours, about 12 hours, about 14 hours, about 16 hours, about 18 hours, about 20 hours, about 22 hours, or about 24 hours following administration to a subject.

In other embodiments, the LFA-1 antagonist has a local tissue concentration of greater than about 100 nM for at least about 2 hours, about 4 hours, about 6 hours, about 8 hours, about 10 hours, about 12 hours, about 14 hours, about 16 hours, about 18 hours, about 20 hours, about 22 hours, or about 24 hours following administration to a subject.

In other embodiments, the LFA-1 antagonist has a local tissue concentration of greater than about 100 nM for at least about 2 hours, about 4 hours, about 6 hours, about 8 hours, about 10 hours, about 12 hours, about 14 hours, about 16 hours, about 18 hours, about 20 hours, about 22 hours, or about 24 hours following administration to a subject. In other embodiments, the LFA-1 antagonist is maintained at a local tissue concentration level greater than about 100 nM for up to about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, or about 24 hours.

In yet other embodiments, the LFA-1 antagonist has a local tissue concentration of greater than about 10 nM for at least about 2 hours, about 4 hours, about 6 hours, about 8 hours, about 10 hours, about 12 hours, about 14 hours, about 16 hours, about 18 hours, about 20 hours, about 22 hours, or about 24 hours following administration to a subject. In other embodiments, the LFA-1 antagonist is maintained at a local tissue concentration level greater than about 10 nM for up to about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours, about 21 hours, about 22 hours, about 23 hours, or about 24 hours.

The disclosure also provides embodiments wherein the LFA-1 antagonist has a local tissue concentration of greater than about 1 nM for at least about 2 hours, about 4 hours, about 6 hours, about 8 hours, about 10 hours, about 12 hours, about 14 hours, about 16 hours, about 18 hours, about 20 hours, about 22 hours, or about 24 hours following administration to a subject.

### LFA-1 Antagonist compounds

Specific LFA-1 antagonist compounds have been previously described in the art and may be used in the present disclosure. For example, LFA-1 antagonists have been described in US Patent No. 7,314,938, US Patent Application Publication No. 2006/0281739, U.S. Application Serial No. 12/288,330, and co-pending US Applications WSGR Docket Numbers 32411-712.201, 32411-709.201, and 32411-710.201. The compounds can be synthesized as described in these references.

In some embodiments, the LFA-1 antagonist is a directly competitive inhibitor of the interaction of LFA-1 and ICAM-1.

In some embodiments, the LFA-1 antagonist of the present disclosure has a structure of Formula (I) or (II):

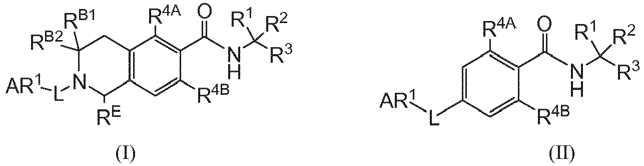

Wherein R¹ and R² are each independently hydrogen, an amino acid side chain, -(CH₂)ₘOH, -(CH2)ₘaryl, - (CH2)ₘheteroaryl, wherein m is 0-6,- CH(R^{1A})(OR^{1B}), -CH(R^{1A})(NHR^{1B}), U-T-Q, or an aliphatic, alicyclic, heteroaliphatic or heteroalicyclic moiety optionally substituted with U-T-Q,

wherein U is absent, -O-, -S(O)₀₋₂-, -SO₂N(R^{1A}), -N(R^{1A})-, -N(R^{1A})C(=O)-, -N(R^{1A})C(=O)-O-, -N(R^{1A})C(=O)-N(R^{1B})-, -N(R^{1A})-SO₂-, -C(=O)-, -C(=O)-O-, -O-C(=O)-, aryl, heteroaryl, alkylaryl, alkylheteroaryl,-C(=O)-N(R^{1A})-, - OC(=O)N(R^{1A})-, -C(=N-R^{1E})-, -C(=N-R^{1E})-O-, -C(=N-R^{1E})-N(R^{1A})-, -O-C(=N-R^{1E})-N(R^{1A})-, -N(R^{1A})C(=N-R^{1E})-, - N(R^{1A})C(=N-R^{1E})-O-, -N(R^{1A})C(=N-R^{1E})-N(R^{1B})-, -P(=O)(OR^{1A})-O-, or -P(=O)(R^{1A})-O-;

T is absent, an aliphatic, heteroaliphatic, aryl, heteroaryl, alkylaryl or alkylheteroaryl moiety; and

Q is hydrogen, halogen, cyano, isocyanate, -OR^{1B}; -SR^{1B}; -N(R^{1B})₂, -NHC(=O)OR^{1B}, -NHC(=O)N(R^{1B})₂, -NHC (=O)R^{1B}, -NHSO₂R^{1B}, NHSO₂N(R^{1B})₂, -NHSO₂NHC(=O)OR^{1B}, -NHC(=O)NHSO₂R^{1B}, -C(=O)NHC(=O)OR^{1B}, C(=O)NHC(=O)R^{1B}, -C(=O)NHC(=O) N(R^{1B})₂, -C(=O)NHSO₂R^{1B}, -C(=O)NHSO₂N(R^{1B})₂, C(=S)N(R^{1B})₂, -SO₂R^{1B}, - SO₂OR^{1B}, -SO₂N(R^{1B})₂, -SO₂-NHC(=O)OR^{1B}, -OC(=O)-N(R^{1B})₂, -OC(=O)R^{1B}, -OC(=O)NHC(=O)R^{1B}, - OC(=O)NHSO₂R^{1B}, -OSO₂R^{1B}, or an aliphatic heteroaliphatic, aryl or heteroaryl moiety, or wherein R¹ and R² taken together are an alicyclic or heterocyclic moiety, or together are

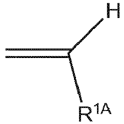

wherein each occurrence of R^{1A} and R^{1B} is independently hydrogen, an aliphatic, alicyclic, heteroaliphatic, heterocyclic, aryl, heteroaryl, alkylaryl or alkylheteroaryl moiety, -C(=O)R^{1C}, or -C(=O)NR^{1C}R^{1D}; wherein each occurrence of R^{1C} and R^{1D} is independently hydrogen, hydroxyl, or an aliphatic, heteroaliphatic, aryl, heteroaryl, alkylaryl or alkylheteroaryl moiety; and R^{1E} is hydrogen, an aliphatic, alicyclic, heteroaliphatic, heterocyclic, aryl, heteroaryl, alkylaryl or alkylheteroaryl moiety, -CN, -OR^{1C},-NR^{1C} R^{1D} or -SO₂R^{1C};

R³ is -C(=O)OR^{3A}, -C(=O)H, -CH₂OR^{3A}, -CH₂OC(=O)-alkyl, OC(=O)NH(R^{3A}). -CH₂X⁰; wherein each occurrence of R^{3A} is independently hydrogen, a protecting group, an aliphatic, alicyclic, heteroaliphatic, heteroalicyclic, aryl, heteroaryl, alkylaryl, alkylheteroaryl, heteroalkylaryl heteroalkylheteroaryl moiety, or pharmaceutically acceptable salt or ester, or R^{3A}, taken together with R¹ and R², forms a heterocyclic moiety; wherein X⁰ is a halogen selected from F, Br or I;

wherein R^{4A} and R^{4B} are independently a halogen selected from F, Cl, Br or I; and R^{B1}, R^{B2} and R^{E} are independently hydrogen or substituted or unsubstituted lower alkyl;

AR¹ is a monocyclic or polycyclic aryl, heteroaryl, alkylaryl, alkylheteroaryl, alicyclic or heterocyclic moiety; and,

L is absent or is V-W-X-Y-Z, wherein each occurrence of V, W, X, Y and Z is independently absent, C=O, NR^{L1}, - O-, -C(R^{L1})=, =C(R^{L1})-, -C(R^{L1})(R^{L2}), C(=N-O R^{L1}), C(=NR^{L1}), -N=, S(O)₀₋₂; a substituted or unsubstituted C₁₋₆ alkenylidene or C₂₋₆ alkenylidine chain wherein up to two non-adjacent methylene units are independently optionally replaced by-C(=O)-, -CO₂-, -C(=O)C(=O)-, -C(C=O)NR^{L3}-, -OC(=O)-, -OC(=O)NR^{L3}-, -NR^{L3}NR^{L4}-, -NR^{L3}NR ^{L4}C(=O)-, -NR^{L3}C(=O)-, NR^{L3}CO₂-, NR^{L3}C(=O)NR^{L4}-, -S(=O)-, -SO₂-, -NR^{L3}SO₂-, -SO₂NR^{L3}, -NR^{L3}SO₂NR^{L4}, -O-, -S-, or -NR^{L3}-; wherein each occurrence of R^{L3} and R^{L4} is independently hydrogen, alkyl, heteroalkyl, aryl, heteroaryl or acyl; or an aliphatic, alicyclic, heteroaliphatic, heteroalicyclic, aryl, heteroaryl, alkylaryl or alkylheteroaryl moiety; and each occurrence of R^{L1} and R^{L2} is independently hydrogen, hydroxyl, protected hydroxyl, amino, protected amino, thio, protected thio, halogen, cyano, isocyanate, carboxy, carboxyalkyl, formyl, formyloxy, azido, nitro, ureido, thioureido, thiocyanato, alkoxy, aryloxy, mercapto, sulfonamido, benzamido, tosyl, or an aliphatic, alicyclic, heteroaliphatic, heteroalicyclic, aryl, heteroaryl, alkylaryl or alkylheteroaryl moiety, or wherein one or more occurrences of R^{L1} and R^{L2}, taken together, or taken together with one of V, W, X, Y or Z form an alicyclic or heterocyclic moiety or form an aryl or heteroaryl moiety, and/or its pharmaceutically acceptable salts or esters.

Compounds of the present disclosure include the following: 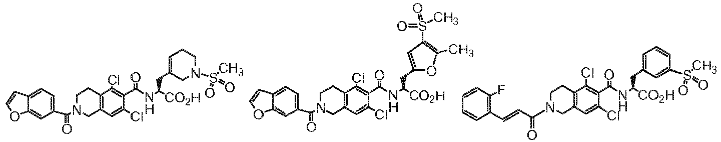 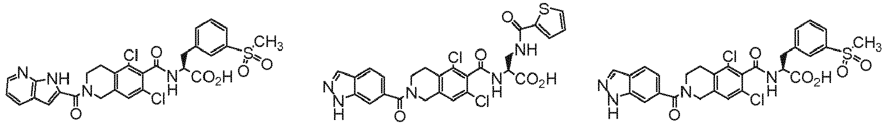 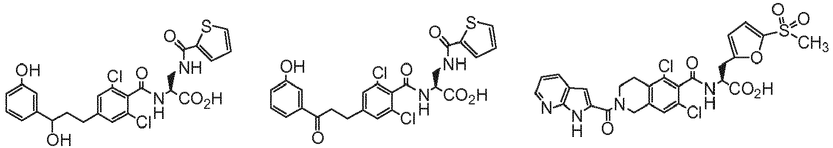 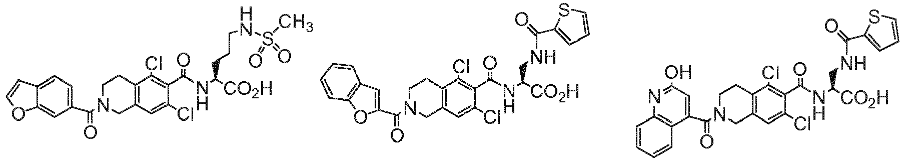 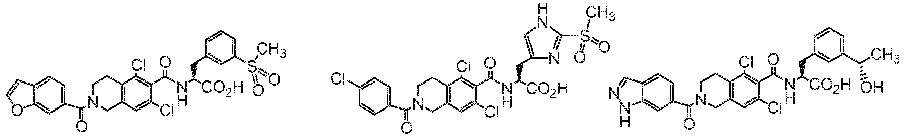 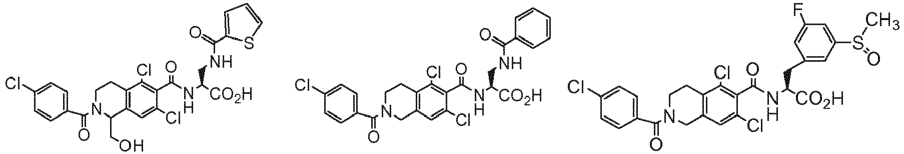 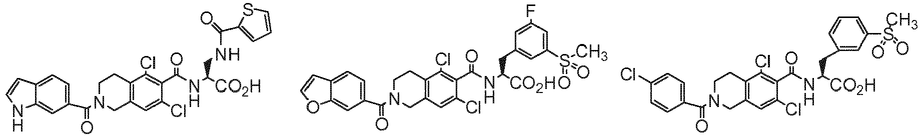 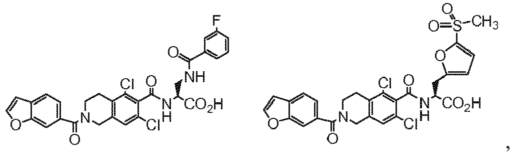 and their pharmaceutically acceptable salts and esters.

It is envisioned additionally, that the LFA-1 antagonist may be used in amorphous form or the LFA-1 antagonist may be any of the crystalline forms described in co-pending application docket number 32411-712.201. In some embodiments of the disclosure, the compound of Formula (I) is Form A of Compound 12, which comprises an X-ray powder diffraction pattern having characteristic peaks at a reflection angle 2θ of about 18.2, 21.4, and 22.7 degrees; Form B of Compound 12, which comprises an X-ray powder diffraction pattern having characteristic peaks at a reflection angle 2θ of about 12.1, 17.1, and 18.5 degrees; Form C of Compound 12, which comprises an X-ray powder diffraction pattern having characteristic peaks at a reflection angle 2θ of about 4.8, 17.8, and 21.5 degrees; Form D of Compound 12, which comprises an X-ray powder diffraction pattern having characteristic peaks at a reflection angle 2θ of about 17.6,21.7, and 24.8 degrees; Form E of Compound 12, which comprises an X-ray powder diffraction pattern having characteristic peaks at a reflection angle 2θ of about 5.12, 8.26, and 17.8 degrees; an amorphous form of Compound 12, which comprises greater than 90% purity; or any combination thereof.

In some embodiments, the LFA-1 antagonist of Formula I or Formula II is a salt. Representative alkali or alkaline earth metal salts include but are not limited to sodium, lithium, potassium, calcium, and magnesium. Further pharmaceutically acceptable salts include, when appropriate, nontoxic ammonium, quaternary ammonium, and amine cations formed by direct reacton with the drug carboxylic acid or by using counterions such as halide, hydroxide, carboxylate, sulfate, phosphate, nitrate, sulfonate and aryl sulfonate. In one embodiment, the LFA-1 antagonist is used in the methods of the disclosure, as the sodium salt of the carboxylic acid.

Antibodies specific for binding to LFA-1 may be used in the present disclosure. Blocking of the CAMs, such as for example ICAM-1, or the leukointegrins, such as LFA-1, by antibodies directed against either or both of these molecules can inhibit inflammatory response. Previous studies have investigated the effects of anti-CD 11a MAbs on many T-cell-dependent immune functions in vitro and a number of immune responses in vivo. In vitro, anti-CD11a MAbs inhibit T-cell activation (See Kuypers T.W., Roos D. 1989 "Leukocyte membrane adhesion proteins LFA-1, CR3 and p150,95: a review of functional and regulatory aspects" Res. Immunol., 140:461-465;
Fischer A, Durandy A, Sterkers G, Griscelli C. 1986 "Role of the LFA-1 molecule in cellular interactions required for antibody production in humans" J. Immunol., 136, 3198; target cell lysis by cytotoxic T-lymphocytes (Krensky et al., supra), formation of immune conjugates (Sanders VM, Snyder JM, Uhr JW, Vitetta ES., "Characterization of the physical interaction between antigen-specific B and T cells". J. Immunol., 137:2395 (1986); Mentzer SJ, Gromkowski SH, Krensky AM, Burakoff SJ, Martz E. 1985 "LFA-1 membrane molecule in the regulation of homotypic adhesions of human B lymphocytes" J. Immunol., 135:9), and the adhesion of T-cells to vascular endothelium (Lo SK, Van Seventer GA, Levin SM, Wright SD., Two leukocyte receptors (CD11a/CD18 and CD11b/CD18) mediate transient adhesion to endothelium by binding to different ligands., J. Immunol., 143:3325 (1989)). Two anti-CD11a MAbs, HI 111, and G43-25B are available from Pharmingen/BD Biosciences. The anti-murine monoclonal antibody M17 has been studied for treatment of LFA-1 mediated disorders in mouse models of human disease and therapy (US Pat. No. 5,622,700). Additionally, a study including F8.8, CBR LFA 1/9, BL5, May.035, TS1/11, TS1/12, TS 1/22, TS2/14, 25-3-1, MHM2 and efalizumab evaluated the range of binding sites on LFA-1 these antibodies occupied in blocking ICAM binding an leukocyte function. See Lu, C; Shimaoka, M.; Salas, A.; Springer, T.A. 2004, "The Binding Sites for Competitive Antagonistic, Allosteric Antagonistic, and Agonistic Antibodies to the I Domain of Integrin LFA-1" J. Immun. 173: 3972-3978 and references therein. In particular, it has been shown that >90% occupancy of LFA-1 with efalizumab led to a greater than 50% clinical improvement in PASI score in a clinical trial demonstrating the efficacy of efalizumab (see D.L. Mortenson et al. J Clin Pharmacol 2005;45:286-298. "Pharmacokinetics and Pharmacodynamics of Multiple Weekly Subcutaneous Efalizumab Doses in Patients With Plaque Psoriasis").

Peptides have also been investigated for use in reducing the interaction of LFA-1 with ICAM-1 and may be used in the present disclosure. Polypeptides that do not contain an Fc region of an IgG are described in U.S. Patent No. 5,747,035, which can be used to treat LFA-1 mediated disorders, in particular diabetic retinopathy. Use of dual peptides, the first a modulator of ICAM-1 and the second a blocking peptide with a sequence obtained from LFA-1 is described in U.S. Patent No. 5,843,885 to reduce the interactions between LFA-1 and ICAM-1. Cyclic peptides have been described in U.S. Patent No. 6,630,447 as inhibitors of the LFA-1:ICAM-1 interaction.

Small molecule antagonists may be used in the present disclosure, for example, statins which bind to the CD11a domain of LFA-1. See Kallen, J., Welzenbach, K., Ramage, P. Geyl, D. Kriwacki, R., Legge, G., Cottens, S., Weitz-Schmidt, G., and Hommel, U. 1999. "Structural basis for LFA-1 inhibition upon lovastatin binding to the CD11a I-domain", J. Mol. Biol., 292: 1-9; and Weitz-Schmidt, G., Welzenbach, K., Brinkmann, V., Kamata, T., Kallen, J., Bruns, C., Cottens, S., Takada, Y., and Hommel, U. 2001. Statins selectively inhibit leukocyte function antigen-1 by binding to a novel regulatory integrin site, Nature Med., 7: 687-692; and Frenette, P. S. 2001. "Locking a leukocyte integrin with statins", N. Engl. J. Med., 345: 1419-1421. Molecules derived from the mevinolin/compactin motif also show activity against LFA-1. See Welzenbach, K., Hommel, U., and Weitz-Schmidt,G. 2002. "Small molecule inhibitors induce conformational changes in the I domain and the I-like domain of Lymphocyte Function-Associated Antigen-1", J. Biol. Chem., 277: 10590-10598, and U.S. Patent No. 6,630,492.

Additionally, other known LFA-1 antagonists recognized in the art may be used in the present disclosure. For example, a family of hydantoin-based inhibitors can be used as LFA-1 antagonists. See Kelly, T. A., Jeanfavre, D. D., McNeil, D. W., Woska, J. R. Jr., Reilly, P. L., Mainolfi, E. A., Kishimoto, K. M., Nabozny, G. H., Zinter, R., Bormann, B.-J., and Rothlein, R. 1999. "Cutting edge: a small molecule antagonist of LFA-1-mediated cell adhesion", J. Immunol., 163: 5173-5177. These compounds are believed to be allosteric inhibitors of LFA-1. As another example, a family of novel p-arylthio cinnamides can act as antagonists of LFA-1. See Liu, G.; Link, J.T.; Pei, Z.; Reilly, E.B.; Nguyen, B.; Marsh, K.C.; Okasinski, G.F.; von Geldern, T.W.; Ormes, M.; Fowler, K.; Gallatin, M. 2000 "Discovery of novel p-arylthio cinnamides as antagonists of leukocyte function-associated antigen-1/intracellular adhesion molecule-1 interaction. 1. Identification of an additional binding pocket based on an anilino diaryl sulfide lead." J. Med. Chem. 43, 4015-4030.

Other families of small molecule inhibitors are disclosed in publications (See Gadek, T. R., Burdick, D. J., McDowell, R. S., Stanley, M. S., Marsters, J. C. Jr., Paris, K. J., Oare, D. A., Reynolds, M. E., Ladner, C., Zioncheck, K. A., Lee, W. P., Gribling, P., Dennis, M. S., Skelton, N. J., Tumas, D. B., Clark, K. R., Keating, S. M., Beresini, M. H., Tilley, J. W., Presta, L. G., and Bodary, S. C. 2002. "Generation of an LFA-1 antagonist by the transfer of the ICAM-1 immunoregulatory epitope to a small molecule" Science, 295: 1086-1089 and online supplementary material.) and in patents, including U.S. Patent No. 6,872, 735, U.S. Patent No. 6,667,318, U.S. Patent No. 6,803,384, U.S. Patent No. 6,515,124, U.S. Patent No. 6,331,640, and patent applications, including: U.S. 2002/0119994. U.S. 2004/0058968, U.S. 2005/0080119, WO99/49856, WO00/21920, WO01/58853, WO02/59114, WO05/044817, and others.

### Topical formulations of LFA-1 antagonists

The invention is suitable for localized treatment of immune related disorders. The formulations contain an LFA-1 antagonist in a composition suitable for topical delivery to a subject. Compositions may include gel, cream, lotion, solution, suspension, emulsion, ointment, powder, crystalline forms, spray, foam, salve, paste, plaster, paint, and bioadhesive. Formulations may further include additional ingredients such as ingredients to facilitate delivery of the active compounds, enhance the therapeutic effect, have a secondary effect or minimize side effects. Such formulations allow for efficacious delivery of LFA-1 antagonists to the site of administration, such as but not limited to the eye, skin, mouth, nose, vaginal mucosa and anal mucosa.

The particular combination of active agent or agents and excipients may be determined in large part by chemical compatibility. That is, each active agent may coexist in the topical pharmaceutical formulation together with the base and any other active agent without reacting or otherwise interacting with each other or with other components of the formulation in a way that would diminish therapeutic efficacy or increase the likelihood of toxic or other adverse effects. Thus, for example, direct contact between a strong inorganic base, such as potassium hydroxide, and an acid, such as salicylic acid, should be avoided, as such compounds may react with each other in deleterious ways. Even such reactive pairs of compounds may, however, be combined in an effective topical formulation if, for example, the active agent is protected (e.g. the active agent is contained within liposomes, micelles, microspheres, or similar structures), so that it is released after permeation into the skin and after the base has dissipated sufficiently to avoid significant reaction with the active agent.

It is envisioned additionally, that the LFA-1 antagonist may be used in amorphous form or any of the crystalline forms described in co-pending U.S. application docket number 32411-712.101. Any of the forms of LFA-1 may also be milled to provide more suitable properties for formulation. Milling may provide smaller particle size with greater surface area exposure, which can provide faster solubilization in-vivo or during formulation. Alternatively, milling to a smaller particle size may provide the capacity to pass through biological barriers, such as the skin or gut wall, directly, without initial solubilization, permitting use as a solid in the formulation, which may provide additional benefits of temperature stability, shelf life, ease of transport, and ease of use by the subject. Furthermore, one skilled in the art would be able to determine which form of the LFA-1 antagonist, or a combination of forms thereof, may be attached releasably to biocompatible polymers for use in sustained release formulations. The controlled release from a biocompatible polymer may be utilized with a water soluble polymer to form an instillable formulation, as well. Any suitable biodegradable and biocompatible polymer may be used.

In yet another aspect, LFA-1 antagonist compounds of the present disclosure may be topically administered alone, for example, in a dry powder form. Dry powder formulations will typically comprise the formulation in a dry, usually lyophilized, form with a particle size within a preferred range for deposition within the alveolar region of the lung, typically from 0.5 µm to 5 µm.

### Excipients

The pharmaceutical compositions may include one or more inert excipients, which include water, buffered aqueous solutions, surfactants, volatile liquids, starches, polyols, granulating agents, microcrystalline cellulose, diluents, lubricants, acids, bases, salts, emulsions, such as oil/water emulsions, oils such as mineral oil and vegetable oil, wetting agents, chelating agents, antioxidants, sterile solutions, complexing agents, and disintegrating agents. The CTFA Cosmetic Ingredient Handbook, Seventh Edition, 1997 and the Eighth Edition, 2000, describes a wide variety of cosmetic and pharmaceutical ingredients commonly used in skin care compositions, which are suitable for use in the compositions of the present invention. Examples of these functional classes disclosed in this reference include: absorbents, abrasives, anticaking agents, antifoaming agents, antimicrobial agents, antioxidants, binders, biological additives, buffering agents, bulking agents, chelating agents, chemical additives, colorants, cosmetic astringents, cosmetic biocides, denaturants, drug astringents, external analgesics, film formers, fragrance components, humectants, opacifying agents, pH adjusters, plasticizers, preservatives, reducing agents, skin bleaching agents, skin-conditioning agents (emollient, humectants, miscellaneous, and occlusive), skin protectants, solvents, foam boosters, hydrotropes, solubilizing agents, steroidal anti-inflammatory agents, surfactants/emulsifying agents, suspending agents (nonsurfactant), sunscreen agents, topical analgesics, ultraviolet light absorbers, SPF boosters, thickening agents, waterproofing agents, and viscosity increasing agents (aqueous and nonaqueous).

Surfactants which can be used to form pharmaceutical compositions and dosage forms of the invention include, but are not limited to, hydrophilic surfactants, lipophilic surfactants, and mixtures thereof. That is, a mixture of hydrophilic surfactants may be employed, a mixture of lipophilic surfactants may be employed, or a mixture of at least one hydrophilic surfactant and at least one lipophilic surfactant may be employed.

One surfactant may be the sodium salt form of the compound, which may include the monosodium salt form. Suitable sodium salt surfactants may be selected based on desirable properties, including high speed of polymerization, small resultant particle sizes suitable for delivery, good polymerization yields, stability including freeze-thaw and shelf-life stability, improved surface tension properties, and lubrication properties.

The surfactant may be any suitable, non-toxic compound that is non-reactive with the medicament and that substantially reduces the surface tension between the medicament, the excipient and the site of administration. The surfactants include but are not limited to: oleic acid available under the tradenames Mednique 6322 and Emersol 6321 (from Cognis Corp., Cincinnati, Ohio); cetylpyridinium chloride (from Arrow Chemical, Inc. Westwood, N.J.); soya lecithin available under the tradename Epikuron 200 (from Lucas Meyer Decatur, Ill.); polyoxyethylene(20) sorbitan monolaurate available under the tradename Tween 20 (from ICI Specialty Chemicals, Wilmington, Del.); polyoxyethylene(20) sorbitan monostearate available under the tradename Tween 60 (from ICI); polyoxyethylene(20) sorbitan monooleate available under the tradename Tween 80 (from ICI); polyoxyethylene (10) stearyl ether available under the tradename Brij 76 (from ICI); polyoxyethylene (2) oleyl ether available under the tradename Brij 92 (frown ICI); Polyoxyethylene-polyoxypropylene-ethylenediamine block copolymer available under the tradename Tetronic 150 R1 (from BASF); polyoxypropylene-polyoxyethylene block copolymers available under the tradenames Pluronic L-92, Pluronic L-121 end Pluronic F 68 (from BASF); castor oil ethoxylate available under the tradename Alkasurf CO-40 (from Rhone-Poulenc Mississauga Ontario, Canada); and mixtures thereof.

A suitable hydrophilic surfactant may generally have an HLB value of at least 10, while suitable lipophilic surfactants may generally have an HLB value of or less than about 10. An empirical parameter used to characterize the relative hydrophilicity and hydrophobicity of non-ionic amphiphilic compounds is the hydrophilic-lipophilic balance (" HLB" value). Surfactants with lower HLB values are more lipophilic or hydrophobic, and have greater solubility in oils, while surfactants with higher HLB values are more hydrophilic, and have greater solubility in aqueous solutions. Hydrophilic surfactants are generally considered to be those compounds having an HLB value greater than about 10, as well as anionic, cationic, or zwitterionic compounds for which the HLB scale is not generally applicable. Similarly, lipophilic (i.e., hydrophobic) surfactants are compounds having an HLB value equal to or less than about 10. However, HLB value of a surfactant is merely a rough guide generally used to enable formulation of industrial, pharmaceutical and cosmetic emulsions.

Hydrophilic surfactants may be either ionic or non-ionic. Suitable ionic surfactants include, but are not limited to, alkylammonium salts; fusidic acid salts; fatty acid derivatives of amino acids, oligopeptides, and polypeptides; glyceride derivatives of amino acids, oligopeptides, and polypeptides; lecithins and hydrogenated lecithins; lysolecithins and hydrogenated lysolecithins; phospholipids and derivatives thereof; lysophospholipids and derivatives thereof; carnitine fatty acid ester salts; salts of alkylsulfates; fatty acid salts; sodium docusate; acyl lactylates; mono- and di-acetylated tartaric acid esters of mono- and di-glycerides; succinylated mono- and di-glycerides; citric acid esters of mono- and di-glycerides; and mixtures thereof.

Within the aforementioned group, ionic surfactants include, by way of example: lecithins, lysolecithin, phospholipids, lysophospholipids and derivatives thereof; carnitine fatty acid ester salts; salts of alkylsulfates; fatty acid salts; sodium docusate; acyl lactylates; mono- and di-acetylated tartaric acid esters of mono- and di-glycerides; succinylated mono- and di-glycerides; citric acid esters of mono- and di-glycerides; and mixtures thereof.

Ionic surfactants may be the ionized forms of lecithin, lysolecithin, phosphatidylcholine, phosphatidylethanolamine, phosphatidylglycerol, phosphatidic acid, phosphatidylserine, lysophosphatidylcholine, lysophosphatidylethanolamine, lysophosphatidylglycerol, lysophosphatidic acid, lysophosphatidylserine, PEG-phosphatidylethanolamine, PVP-phosphatidylethanolamine, lactylic esters of fatty acids, stearoyl-2-lactylate, stearoyl lactylate, succinylated monoglycerides, mono/diacetylated tartaric acid esters of mono/diglycerides, citric acid esters of mono/diglycerides, cholylsarcosine, caproate, caprylate, caprate, laurate, myristate, palmitate, oleate, ricinoleate, linoleate, linolenate, stearate, lauryl sulfate, teracecyl sulfate, docusate, lauroyl carnitines, palmitoyl carnitines, myristoyl carnitines, and salts and mixtures thereof.

Hydrophilic non-ionic surfactants may include, but not limited to, alkylglucosides; alkylmaltosides; alkylthioglucosides; lauryl macrogolglycerides; polyoxyalkylene alkyl ethers such as polyethylene glycol alkyl ethers; polyoxyalkylene alkylphenols such as polyethylene glycol alkyl phenols; polyoxyalkylene alkyl phenol fatty acid esters such as polyethylene glycol fatty acids monoesters and polyethylene glycol fatty acids diesters; polyethylene glycol glycerol fatty acid esters; polyglycerol fatty acid esters; polyoxyalkylene sorbitan fatty acid esters such as polyethylene glycol sorbitan fatty acid esters; hydrophilic transesterification products of a polyol with at least one member of the group consisting of glycerides, vegetable oils, hydrogenated vegetable oils, fatty acids, and sterols; polyoxyethylene sterols, derivatives, and analogues thereof; polyoxyethylated vitamins and derivatives thereof; polyoxyethylene-polyoxypropylene block copolymers; and mixtures thereof; polyethylene glycol sorbitan fatty acid esters and hydrophilic transesterification products of a polyol with at least one member of the group consisting of triglycerides, vegetable oils, and hydrogenated vegetable oils. The polyol may be glycerol, ethylene glycol, polyethylene glycol, sorbitol, propylene glycol, pentaerythritol, or a saccharide.

Other hydrophilic-non-ionic surfactants include, without limitation, PEG-10 laurate, PEG-12 laurate, PEG-20 laurate, PEG-32 laurate, PEG-32 dilaurate, PEG-12 oleate, PEG-15 oleate, PEG-20 oleate, PEG-20 dioleate, PEG-32 oleate, PEG-200 oleate, PEG-400 oleate, PEG-15 stearate, PEG-32 distearate, PEG-40 stearate, PEG-100 stearate, PEG-20 dilaurate, PEG-25 glyceryl trioleate, PEG-32 dioleate, PEG-20 glyceryl laurate, PEG-30 glyceryl laurate, PEG-20 glyceryl stearate, PEG-20 glyceryl oleate, PEG-30 glyceryl oleate, PEG-30 glyceryl laurate, PEG-40 glyceryl laurate, PEG-40 palm kernel oil, PEG-50 hydrogenated castor oil, PEG-40 castor oil, PEG-35 castor oil, PEG-60 castor oil, PEG-40 hydrogenated castor oil, PEG-60 hydrogenated castor oil, PEG-60 corn oil, PEG-6 caprate/caprylate glycerides, PEG-8 caprate/caprylate glycerides, polyglyceryl-10 laurate, PEG-30 cholesterol, PEG-25 phyto sterol, PEG-30 soya sterol, PEG-20 trioleate, PEG-40 sorbitan oleate, PEG-80 sorbitan laurate, polysorbate 20, polysorbate 80, POE-9 lauryl ether, POE-23 lauryl ether, POE-10 oleyl ether, POE-20 oleyl ether, POE-20 stearyl ether, tocopheryl PEG-100 succinate, PEG-24 cholesterol, polyglyceryl-lOoleate, Tween 40, Tween 60, sucrose monostearate, sucrose monolaurate, sucrose monopalmitate, PEG 10-100 nonyl phenol series, PEG 15-100 octyl phenol series, and poloxamers.

Suitable lipophilic surfactants include, by way of example only: fatty alcohols; glycerol fatty acid esters; acetylated glycerol fatty acid esters; lower alcohol fatty acids esters; propylene glycol fatty acid esters; sorbitan fatty acid esters; polyethylene glycol sorbitan fatty acid esters; sterols and sterol derivatives; polyoxyethylated sterols and sterol derivatives; polyethylene glycol alkyl ethers; sugar esters; sugar ethers; lactic acid derivatives of mono- and di-glycerides; hydrophobic transesterification products of a polyol with at least one member of the group consisting of glycerides, vegetable oils, hydrogenated vegetable oils, fatty acids and sterols; oil-soluble vitamins/vitamin derivatives; and mixtures thereof. Within this group, lipophilic surfactants include glycerol fatty acid esters, propylene glycol fatty acid esters, and mixtures thereof, or are hydrophobic transesterification products of a polyol with at least one member of the group consisting of vegetable oils, hydrogenated vegetable oils, and triglycerides.

Surfactants may be used in any formulation of the invention where its use is not otherwise contradicted. In some embodiments of the invention, the use of no surfactants or limited classes of surfactants is desirable. The topical formulations according to the invention can contain no, or substantially no surfactant, i.e. contain less than approximately 0.0001% by weight of surface-active agents. This is particularly the case if one employs a cromone as described above. If desired, however, the formulations can contain surface-active agents conventionally employed in topical formulations, such as oleic acid, lecithin, sorbitan trioleate, cetylpyridinium chloride, benzalkonium chloride, polyoxyethylene (20) sorbitan monolaurate, polyoxyethylene (20) sorbitan monostearate, polyoxyethylene (20) sorbitan mono-oleate, polyoxypropylene/polyoxyethylene block copolymers, polyoxypropylene/polyoxyethylene/ethylenediamine block copolymers, and ethoxylated castor oil, where the proportion of surface-active agents, if present, can be about 0.0001 to 1% by weight, in particular about 0.001 to 0.1% by weight, based on the total formulation. Other suitable surfactant/emulsifying agents would be known to one of skill in the art and are listed in the CTFA International Cosmetic Ingredient Dictionary and Handbook, Vol. 2, 7th Edition (1997).

Other suitable aqueous vehicles include, but are not limited to, Ringer's solution and isotonic sodium chloride. Aqueous suspensions may include suspending agents such as cellulose derivatives, sodium alginate, polyvinyl-pyrrolidone and gum tragacanth, and a wetting agent such as lecithin. Suitable preservatives for aqueous suspensions include ethyl and n-propyl p-hydroxybenzoate.

Chelating agents which can be used to form pharmaceutical compositions and dosage forms of the invention include, but are not limited to, ethylene diaminetetraacetic acid (EDTA), EDTA disodium, calcium disodium edetate, EDTA trisodium, albumin, transferrin, desferoxamine, desferal, desferoxamine mesylate, EDTA tetrasodium and EDTA dipotassium, sodium metasilicate or combinations of any of these. In some embodiments, up to about 0.1% W/V of a chelating agent, such as EDTA or its salts, is added to the formulations of the invention.

Preservatives which can be used to form pharmaceutical compositions and dosage forms of the invention include, but are not limited to, purite, peroxides, perborates, imidazolidinyl urea, diazolidinyl urea, phenoxyethanol, alkonium chlorides including benzalkonium chlorides, methylparaben, ethylparaben and propylparaben. In other embodiments, suitable preservatives for the compositions of the invention include: benzalkonium chloride, purite, peroxides, perborates, thimerosal, chlorobutanol, methyl paraben, propyl paraben, phenylethyl alcohol, edetate disodium, sorbic acid, Onamer M, or other agents known to those skilled in the art. In some embodiments of the invention, such preservatives may be employed at a level of from 0.004% to 0.02%W/V. In some compositions of the present application the preservative, for example, benzalkonium chloride, methyl paraben, and/or propyl paraben, may be employed at a level of from about 0.001% to less than about 0.01%, e.g. from about 0.001% to about 0.008%, or about 0.005% W/V. It has been found that a concentration of benzalkonium chloride of about 0.005% may be sufficient to preserve the compositions of the present invention from microbial attack. One of skill in the art could determine the proper concentration of ingredients as well as combinations of various ingredients for generating a suitable topical formulation. For example, ophthalmic drops or formulations for application to skin may use a mixture of methyl and propyl parabens at about 0.02%W/V and about 0.04%W/V respectively. In some embodiments, these formulations use methyl paraben and/or propyl paraben in amounts up to about 0.02% W/V and up to about 0.04% W/V respectively, which encompasses the embodiments where no methyl paraben or no propyl paraben is used.

Lubricants which can be used to form pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, or mixtures thereof.

Thickening agents which can be used to form pharmaceutical compositions and dosage forms of the invention include, but are not limited to, isopropyl myristate, isopropyl palmitate, isodecyl neopentanoate, squalene, mineral oil, C₁₂-C₁₅ benzoate and hydrogenated polyisobutene. Those agents which would not disrupt other compounds of the final product, such as non-ionic thickening agents may be desirable. The selection of additional thickening agents is well within the skill of one in the art.

Skin conditioning agents can be emollients, humectants and moisturizers. A humectant is a moistening agent that promotes retention of water due to its hygroscopic properties. Suitable skin conditioning agents include urea; guanidine; aloe vera; glycolic acid and glycolate salts such as ammonium and quaternary alkyl ammonium; lactic acid and lactate salts such as sodium lactate, ammonium lactate and quaternary alkyl ammonium lactate; polyhydroxy alcohols such as sorbitol, glycerol, mannitol, xylitol, hexanetriol, propylene glycol, butylene glycol, hexylene glycol, polymeric glycols such as polyethylene glycol and polypropylene glycol; carbohydrates such as alkoxylated glucose; starches; starch derivatives; glycerin; pyrrolidone carboxylic acid (PCA); lactamide monoethanolamine; acetamide monoethanolamine; volatile silicone oils; nonvolatile silicone oils; and mixtures thereof. Suitable silicone oils can be polydialkylsiloxanes, polydiarylsiloxanes, polyalkarylsiloxanes and cyclomethicones having 3 to 9 silicon atoms.

An emollient is an oleaginous or oily substance which helps to smooth and soften the skin, and may also reduce its roughness, cracking or irritation. Typical suitable emollients include mineral oil having a viscosity in the range of 50 to 500 centipoise (cps), lanolin oil, coconut oil, cocoa butter, olive oil, almond oil, macadamia nut oil, aloe extracts such as aloe vera lipoquinone, synthetic jojoba oils, natural sonora jojoba oils, safflower oil, corn oil, liquid lanolin, cottonseed oil and peanut oil. In some embodiments, the emollient is a cocoglyceride, which is a mixture of mono, di and triglycerides of cocoa oil, sold under the trade name of Myritol 331 from Henkel KGaA, or Dicaprylyl Ether available under the trade name Cetiol OE from Henkel KGaA or a C₁₂-C₁₅ Alkyl Benzoate sold under the trade name Finsolv TN from Finetex. Another suitable emollient is DC 200 Fluid 350, a silicone fluid, available from Dow Corning Corp.

Other suitable emollients include squalane, castor oil, polybutene, sweet almond oil, avocado oil, calophyllum oil, ricin oil, vitamin E acetate, olive oil, silicone oils such as dimethylopolysiloxane and cyclomethicone, linolenic alcohol, oleyl alcohol, the oil of cereal germs such as the oil of wheat germ, isopropyl palmitate, octyl palmitate, isopropyl myristate, hexadecyl stearate, butyl stearate, decyl oleate, acetyl glycerides, the octanoates and benzoates of (C₁₂-C₁₅) alcohols, the octanoates and decanoates of alcohols and polyalcohols such as those of glycol and glyceryl, ricinoleates esters such as isopropyl adipate, hexyl laurate and octyl dodecanoate, dicaprylyl maleate, hydrogenated vegetable oil, phenyltrimethicone, jojoba oil and aloe vera extract.

Other suitable emollients which are solids or semi-solids at ambient temperatures may be used. Such solid or semi-solid cosmetic emollients include glyceryl dilaurate, hydrogenated lanolin, hydroxylated lanolin, acetylated lanolin, petrolatum, isopropyl lanolate, butyl myristate, cetyl myristate, myristyl myristate, myristyl lactate, cetyl alcohol, isostearyl alcohol and isocetyl lanolate. One or more emollients can optionally be included in the formulation.

Anti-oxidants which can be used to form pharmaceutical compositions and dosage forms of the invention include, but are not limited to, propyl, octyl and dodecyl esters of gallic acid, butylated hydroxyanisole (BHA, usually purchased as a mixture of ortho and meta isomers), green tea extract, uric acid, cysteine, pyruvate, nordihydroguaiaretic acid, ascorbic acid, salts of ascorbic acid such as ascorbyl palmitate and sodium ascorbate, ascorbyl glucosamine, vitamin E (i.e., tocopherols such as a-tocopherol), derivatives of vitamin E (e.g., tocopheryl acetate), retinoids such as retinoic acid, retinol, trans-retinol, cis-retinol, mixtures of trans-retinol and cis-retinol, 3-dehydroretinol and derivatives of vitamin A (e.g., retinyl acetate, retinal and retinyl palmitate, also known as tetinyl palmitate), sodium citrate, sodium sulfite, lycopene, anthocyanids, bioflavinoids (e.g., hesperitin, naringen, rutin and quercetin), superoxide dismutase, glutathione peroxidase, butylated hydroxytoluene (BHT), indole-3-carbinol, pycnogenol, melatonin, sulforaphane, pregnenolone, lipoic acid and 4-hydroxy-5-methyl-3[2H]-furanone.

Skin protecting agents are agents that protect the skin against chemical irritants and/or physical irritants, e.g., UV light, including sunscreens, anti-acne additives, anti-wrinkle and anti-skin atrophy agents. Suitable sunscreens as skin protecting agents include 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropy dibenzoylmethane, 3-benzylidene camphor, 3-(4-methylbenzylidene) camphor, anthanilates, ultrafine titanium dioxide, zinc oxide, iron oxide, silica, 4-N,N-(2-ethylhexyl)methylaminobenzoic acid ester of 2,4-dihydroxybenzophenone, 4-N,N-(2-ethylhexyl)-methylaminobenzoic acid ester with 4-hydroxydibenzoylmethane, 4-N,N-(2-ethylhexyl)-methylaminobenzoic acid ester of 2-hydroxy-4-(2-hydroxyethoxy)benzophenone and 4-N,N(2-ethylhexyl)-methylaminobenzoic acid ester of 4-(2-hydroxyethoxy)dibenzoylmethane. Suitable anti-acne agents include salicylic acid; 5-octanoyl salicylic acid; resorcinol; retinoids such as retinoic acid and its derivatives; sulfur-containing D and L amino acids other than cysteine; lipoic acid; antibiotics and antimicrobials such as benzoyl peroxide, octopirox, tetracycline, 2,4,4'-trichloro-2'-hydroxydiphenyl ether, 3,4,4'-trichlorobanilide, azelaic acid, phenoxyethanol, phenoxypropanol, phenoxisopropanol, ethyl acetate, clindamycin and melclocycline; flavonoids; and bile salts such as scymnol sulfate, deoxycholate and cholate. Examples of anti-wrinkle and anti-skin atrophy agents are retinoic acid and its derivatives, retinol, retinyl esters, salicylic acid and its derivatives, sulfur-containing D and L amino acids except cysteine, alpha-hydroxy acids (e.g., glycolic acid and lactic acid), phytic acid, lipoic acid and lysophosphatidic acid.

The formulations may also contain irritation-mitigating additives to minimize or eliminate the possibility of skin irritation or skin damage resulting from the permeation-enhancing base or other components of the composition. Suitable irritation-mitigating additives include, for example: alpha-tocopherol; monoamine oxidase inhibitors, particularly phenyl alcohols such as 2-phenyl-1-ethanol; glycerin; salicylic acids and salicylates; ascorbic acids and ascorbates; ionophores such as monensin; amphiphilic amines; ammonium chloride; N-acetylcysteine; cis-urocanic acid; capsaicin; and chloroquine. The irritant-mitigating additive, if present, may be incorporated into the present formulations at a concentration effective to mitigate irritation or skin damage, typically representing not more than about 20 wt. %, more typically not more than about 5 wt. %, of the composition.

A dry-feel modifier is an agent which when added to an emulsion, imparts a "dry feel" to the skin when the emulsion dries. Dry feel modifiers can include talc, kaolin, chalk, zinc oxide, silicone fluids, inorganic salts such as barium sulfate, surface treated silica, precipitated silica, fumed silica such as an Aerosil available from Degussa Inc. of New York, N.Y. U.S.A. Another dry feel modifier is an epichlorohydrin cross-linked glyceryl starch of the type that is disclosed in U.S. Pat. No. 6,488,916.

Other agents may also be added, such as antimicrobial agents, to prevent spoilage upon storage, i.e., to inhibit growth of microbes such as yeasts and molds. Suitable antimicrobial agents are typically selected from the group consisting of the methyl and propyl esters of p-hydroxybenzoic acid (i.e., methyl and propyl paraben), sodium benzoate, sorbic acid, imidurea, purite, peroxides, perborates and combinations thereof.

The formulation may also contain an aesthetic agent. Examples of aesthetic agents include fragrances, pigments, colorants, essential oils, skin sensates and astringents. Suitable aesthetic agents include clove oil, menthol, camphor, eucalyptus oil, eugenol, methyl lactate, bisabolol, witch hazel distillate and green tea extract.

Fragrances are aromatic substances which can impart an aesthetically pleasing aroma. Typical fragrances include aromatic materials extracted from botanical sources (i.e., rose petals, gardenia blossoms, jasmine flowers, etc.) which can be used alone or in any combination to create essential oils. Alternatively, alcoholic extracts may be prepared for compounding fragrances. However, due to the relatively high costs of obtaining fragrances from natural substances, the modern trend is to use synthetically prepared fragrances, particularly in high-volume products. One or more fragrances can optionally be included in the sunscreen composition in an amount ranging from about 0.001 to about 5 weight percent, p orabout 0.01 to about 0.5 percent by weight. Additional preservatives may also be used if desired and include well known preservative compositions such as benzyl alcohol, phenyl ethyl alcohol and benzoic acid, diazolydinyl, urea, chlorphenesin, iodopropynyl and butyl carbamate, among others.

### Topical penetration enhancers

The delivery of drugs topically to the skin provides many advantages. For the patient, it is comfortable, convenient, and noninvasive. The variable rates of absorption and metabolism possibly encountered in oral treatment may be avoided, and other inherent inconveniences (e.g., gastrointestinal irritation, the need for administration with food in some cases or without food in other cases) are eliminated. Such localized treatment avoids incurring high systemic drug levels and possible adverse effects that could follow, i.e. inhibition of LFA-1 in other biological processes.

The topical delivery of drugs into the skin, however, is commonly challenging. Skin is a structurally complex, relatively thick membrane. Molecules moving from the environment into and through intact skin must first penetrate the stratum corneum and any material on its surface. The stratum corneum is a layer approximately 10-15 micrometers thick over most of the body that consists of dense, highly keratinized cells. The high degree of keratinization within these cells, as well as their dense packing, are believed to be the factors most responsible for creating, in most cases, a substantially impermeable barrier to drug penetration. With many drugs, the rate of penetration through the skin is extremely low without the use of some means to enhance the skin's permeability. As the stratum corneum of many inflammatory dermatoses is commonly thicker than that of normal skin, the penetration of topical drugs into the affected areas of skin is particularly difficult to achieve.

**In** order to increase the degree and rate at which a drug penetrates the skin, various approaches have been followed, each of which involves the use of either a chemical penetration enhancer or a physical penetration enhancer. Physical enhancements of skin permeation include, for example, electrophoretic techniques such as iontophoresis. The use of ultrasound (or "phonophoresis") as a physical penetration enhancer has also been researched. Chemical penetration enhancers are more commonly used. These are compounds that are topically administered along with a drug (or, in some cases, prior to drug administration) in order to increase the permeability of the stratum corneum, and thereby provide for enhanced penetration of the drug through the skin. Ideally, such chemical penetration enhancers (or "permeation enhancers," as the compounds are referred to herein) are compounds that are innocuous and serve merely to facilitate diffusion of the drug through the stratum corneum.

Various compounds for enhancing the permeability of skin are known in the art and are described in the pertinent texts and literature. Compounds that have been used to enhance skin permeability include: sulfoxides such as dimethylsulfoxide (DMSO) and decylmethylsulfoxide (C₁₀MSO); ethers such as diethylene glycol monoethyl ether (available commercially as Transcutol®) and diethylene glycol monomethyl ether; surfactants such as sodium laurate, sodium lauryl sulfate, cetyltrimethylammonium bromide, benzalkonium chloride, Poloxamer (231, 182, 184), Tween (20, 40, 60, 80), and lecithin (U.S. Pat. No. 4,783,450); the 1-substituted azacycloheptan-2-ones, particularly 1-n-dodecylcyclazacycloheptan-2-one (available under the trademark Azone.RTM. from Nelson Research & Development Co., Irvine, Calif.; see U.S. Pat. Nos. 3,989,816, 4,316,893, 4,405,616, and 4,557,934); alcohols such as ethanol, propanol, octanol, and benzyl alcohol; fatty acids such as lauric acid, oleic acid and valeric acid; fatty acid esters such as isopropyl myristate, isopropyl palmitate, methylpropionate, and ethyl oleate; polyols and esters thereof such as propylene glycol, ethylene glycol, glycerol, butanediol, polyethylene glycol, and polyethylene glycol monolaurate (PEGML; see, e.g., U.S. Pat. No. 4,568,343); amides and other nitrogenous compounds such as urea, dimethylacetamide (DMA), dimethylformamide (DMF), 2-pyrrolidone, 1-methyl-2-pyrrolidone, ethanolamine, diethanolamine and triethanolamine; terpenes; alkanones; and organic acids, particularly salicylic acid and salicylates, citric acid, and succinic acid. The book Percutaneous Penetration Enhancers (Smith et al., editors, CRC Press, 1995) provides an excellent overview of the field and further background information on a number of chemical and physical enhancers.

It has long been thought that strong bases, such as NaOH, were not suitable as permeation enhancers because they would damage skin. It has been now been discovered that the skin permeability of various drugs could be enhanced without skin damage by exposing the skin to a base or basic solution, in a skin contacting formulation or patch. The desired pH of the solution on the skin can be obtained using a variety of bases or base concentrations. Accordingly, the pH is selected so as to be low enough so as to not cause skin damage, but high enough to enhance skin permeation to various active agents. As such, it is important that the amount of base in any patch or formulation is optimized so as to increase the flux of the drug through the body surface while minimizing any possibility of skin damage. In general, this means that the pH at the body surface in contact with a formulation or drug delivery system of the invention may be in the range of approximately pH 8.0 to about pH 13.0, about pH 8.0 to about pH 11.5, about pH 8.5 to about pH 11.5, or about pH 8.5 to about pH 10.5. In some embodiments, the pH is in the range of about pH 9.5 to about pH 11.5, or about pH 10.0 to about pH 11.5.

In one embodiment, the pH at the skin surface is the primary design consideration, i.e., the composition or system is designed so as to provide the desired pH at the skin surface. Anhydrous formulations and transdermal systems may not have a measurable pH, and the formulation or system can be designed so as to provide a target pH at the skin surface. Moisture from the body surface can migrate into the formulation or system, dissolve the base and thus release the base into solution, which will then provide the desired target pH at body surface. In those instances, a hydrophilic composition may be desirable. In addition, when using aqueous formulations, the pH of the formulation may change over time after it is applied on the skin. For example, gels, solutions, ointments, etc., may experience a net loss of moisture after being applied to the body surface, i.e., the amount of water lost is greater than the amount of water received from the body surface. In that case, the pH of the formulation may be different than its pH when manufactured. This problem can be easily remedied by designing the aqueous formulations to provide a target pH at the body surface.

In other embodiments of the invention, the pH of the formulation or the drug composition contained within a delivery system will be in the range of approximately pH 8.0 to about pH 13.0, about pH 8.0 to about pH 11.5, about pH 8.5 to about pH 11.5, or about pH 8.5 to about pH 10.5. In some embodiments, the pH will be in the range of about pH 9.5 to about pH 11.5, or about pH 10.0 to about pH 11.5. In one embodiment of the invention the pH of the formulation is higher than the pH at the body surface. For example, if an aqueous formulation is used, moisture from the body surface can dilute the formulation, and thus provide for a different pH at the body surface, which will typically be lower than that of the formulation itself.

In one embodiment, the body surface is exposed to a base or basic solution for a sufficient period of time so as to provide a high pH at the skin surface, thus creating channels in the skin or mucosa for the drug to go through. It is expected that drug flux is proportional to the strength of the solution and the duration of exposure. However, it is desirable to balance the maximization of drug flux with the minimization of skin damage. This can be done in numerous ways. For example, the skin damage may be minimized by selecting a lower pH within the 8.0 to 13.0 range, by exposing the skin to the formulation or system for a shorter period of time, or by including at least one irritation-mitigating additive. Alternatively, the patient can be advised to change the location of application with each subsequent administration.

While certain amounts are set forth below, it is understood that, for all of the inorganic and organic bases described herein, the optimum amount of any such base will depend on the strength or weakness of the base and its molecular weight, and other factors such as the number of ionizable sites in the active agent being administered and whether there are any acidic species present in the formulation or patch. One skilled in the art may readily determine the optimum amount for any particular base such that the degree of enhancement is optimized while the possibility of damage to the body surface is eliminated or at least substantially minimized.

Exemplary inorganic bases are inorganic hydroxides, inorganic oxides, inorganic salts of weak acids, and combinations thereof. Some inorganic bases are those whose aqueous solutions have a high pH, and are acceptable as food or pharmaceutical additives. Examples of such inorganic bases include ammonium hydroxide, sodium hydroxide, potassium hydroxide, calcium hydroxide, magnesium hydroxide, magnesium oxide, calcium oxide, Ca(OH)₂, sodium acetate, sodium borate, sodium metaborate, sodium carbonate, sodium bicarbonate, sodium phosphate, potassium carbonate, potassium bicarbonate, potassium citrate, potassium acetate, potassium phosphate and ammonium phosphate and combinations thereof.

Inorganic hydroxides include, for example, ammonium hydroxide, alkali metal hydroxide and alkaline earth metal hydroxides, and mixtures thereof. Some inorganic hydroxides include ammonium hydroxide; monovalent alkali metal hydroxides such as sodium hydroxide and potassium hydroxide; divalent alkali earth metal hydroxides such as calcium hydroxide and magnesium hydroxide; and combinations thereof.

The amount of inorganic hydroxide included in the compositions and systems of the invention will typically represent about 0.3-7.0 W/V %, about 0.5-4.0 W/V %, about 0.5-3.0 W/V %, or about 0.75-2.0 W/V % of a topically applied formulation or of a drug reservoir of a drug delivery system, or patch.

Inorganic oxides include, for example, magnesium oxide, and calcium oxide.

The amount of inorganic oxide included in the compositions and systems of the invention may be substantially higher than the numbers set forth above for the inorganic hydroxide, and may be as high as 20 wt %, in some cases as high as 25 wt % or higher, but will generally be in the range of about 2-20 wt %. These amounts may be adjusted to take into consideration the presence of any base-neutralizable species.

Inorganic salts of weak acids include, ammonium phosphate (dibasic); alkali metal salts of weak acids such as sodium acetate, sodium borate, sodium metaborate, sodium carbonate, sodium bicarbonate, sodium phosphate (tribasic), sodium phosphate (dibasic), potassium carbonate, potassium bicarbonate, potassium citrate, potassium acetate, potassium phosphate (dibasic), potassium phosphate (tribasic); alkaline earth metal salts of weak acids such as magnesium phosphate and calcium phosphate; and combinations thereof.

Organic bases suitable for use in the invention are compounds having an amino group, amido group, an oxime, a cyano group, an aromatic or non-aromatic nitrogen-containing heterocycle, a urea group, and combinations thereof. More specifically, examples of suitable organic bases are nitrogenous bases, which include, but are not limited to, primary amines, secondary amines, tertiary amines, amidines, guanidines, hydroxylamines, cyano guanidines, cyanoamidines, oximes, cyano (--CN) containing groups, aromatic and non-aromatic nitrogen-containing heterocycles, urea, and mixtures thereof. In some embodiments, the organic bases are primary amines, secondary amines, tertiary amines, aromatic and non-aromatic nitrogen-containing heterocycles, and mixtures thereof.

For all permeation-enhancing bases herein, the optimum amount of any particular agent will depend on the strength or weakness of the base, the molecular weight of the base, and other factors such as the number of ionizable sites in the drug administered and any other acidic species in the formulation or patch. One skilled in the art may readily determine the optimum amount for any particular agent by ensuring that a formulation is effective to provide a pH at the skin surface, upon application of the formulation, in the range of about pH 7.5 to about pH 13.0, about pH 8.0 to about pH 11.5, or about pH 8.5 to about pH 10.5. In some embodiments, the pH will be in the range of about pH 9.5 to about pH 11.5, or about pH 10.0 to about pH 11.5. This in turn ensures that the degree of treatment is maximized while the possibility of damage to the body surface is eliminated or at least substantially minimized.

In the case of intranasal administration, such solutions or suspensions may be isotonic relative to nasal secretions and of about the same pH, ranging e.g., from about pH 4.0 to about pH 7.4 or from about pH 6.0 to about pH 7.0. Buffers should be physiologically compatible and include, simply by way of example, phosphate buffers. For example, a representative nasal decongestant is described as being buffered to a pH of about 6.2 (Remington's Pharmaceutical Sciences 16th edition, Ed. Arthur Osol, page 1445 (1980)). One skilled in the art can readily determine a suitable saline content and pH for an innocuous aqueous solution for nasal and/or upper respiratory administration. An example of a suitable formulation for intranasal administration, is an aqueous solution buffered to a pH of about 6.0 to about 8.0 with Sodium Phosphate, Monobasic,comprising about 1% W/V of the LFA-1 antagonist, up to about 0.1% W/V EDTA, and, optionally, up to about 0.4% w/w Methylparaben and up to about 0.02% w/w Propylparaben.

Additional permeation enhancers will be known to those of ordinary skill in the art of topical drug delivery, and/or are described in the pertinent texts and literature. See, e.g., Percutaneous Penetration Enhancers, Smith et al., eds. (CRC Press, 1995).

### LFA-1 antagonists with other active agents

In one embodiment, the methods of the disclosure involve the administration of one or more additional drugs for the treatment of immune related disorders. Combinations of agents can be used to treat LFA-1 mediated disorders or to modulate the side-effects of one or more agents in the combination. In some instances, pathological events in this disease state are marked by a combination of impaired autoregulation, apoptosis, ischemia, neovascularization, and inflammatory stimuli, it may be desirable to administer the LFA-1 antagonists of the disclosure in combination with other therapeutic agents to additionally or synergistically intervene. In some embodiments, the second therapeutic agent is an antioxidant, antiinflammatory agent, antimicrobial including antibacterial, antihistamine, mast cell stabilizer, antiviral and antifungal agents, antiangiogenic agent, anti-apoptotic agent, lubricant, and/or secretagogue. In some embodiments of the disclosure, in addition to administering a compound which directly competes for binding to LFA-1, an additional therapeutic agent may be administered which is an allosteric, but not a directly competitive, anatagonist of LFA-1 as discussed above, potentially resulting in synergistic efficacy. An example of such allosteric antagonist is the class of hydantoin inhibitors of LFA-1. (See for example, Keating et al., Protein Science, 15, 290-303, (2006)).

A class of therapeutic agents which may be useful to administer in combination, prior to, after, or concomitantly with the LFA-1 antagonists of the disclosure is the group of drugs which inhibit Vascular Endothelial Growth Factor and thus may target another route of initiation of neovascularization. Any VEGF inhibitor may be of use in the compositions of the invention, for example: 1) neutralizing monoclonal antibodies against VEGF or its receptor, 2) small molecule tyrosine kinase inhibitors of VEGF receptors, 3) soluble VEGF receptors which act as decoy receptors for VEGF, and 4) ribozymes which specifically target VEGF. Some examples of antibodies which are active against VEGF are, for example, Lucentis (ranibizumab), and Avastin (bevacizumab). An example of an oligonucleotide drug is, e.g., Macugen (pegaptanib sodium injection). Small molecule tyrosine kinase inhibitors include, for example, pazopanib, sorafenib, and sutent.

Inflammation is induced by the process of leukocyte adhesion and neovascularization. Therefore, other anti-inflammatory agents may be administered in combination, prior to, after, or concomitantly with the LFA-1 antagonists of the disclosure. The anti-inflammatory agents can be chosen from corticosteroid related drugs including but not limited to dexamethasone, fluoromethalone, medrysone, betamethasone, triamcinolone, triamcinolone acetonide, prednisone, prednisolone, hydrocortisone, rimexolone, and pharmaceutically acceptable salts thereof, prednicarbate, deflazacort, halomethasone, tixocortol, prednylidene , prednival, paramethasone, methylprednisolone, meprednisone, mazipredone, isoflupredone, halopredone acetate, halcinonide, formocortal, flurandrenolide, fluprednisolone, fluprednidine acetate, fluperolone acetate, fluocortolone, fluocortin butyl, fluocinonide, fluocinolone acetonide, flunisolide, flumethasone, fludrocortisone, fluclorinide, enoxolone, difluprednate, diflucortolone, diflorasone diacetate, desoximetasone (desoxymethasone), desonide, descinolone, cortivazol, corticosterone, cortisone, cloprednol, clocortolone, clobetasone, clobetasol, chloroprednisone, cafestol, budesonide, beclomethasone, amcinonide, allopregnane acetonide, alclometasone, 21-acetoxypregnenolone, tralonide, diflorasone acetate, deacylcortivazol, RU-26988, budesonide, and deacylcortivazol. Additionally anti-inflammatory agents include 5-aminosalicylate (5-ASA) compounds, such as sulfasalzine (Azulfidine), osalazine (Dipentum), and mesalamine (examples include Pentasa, Asacol, Dipentum, Colazal, Rowasa enema, and Canasa suppository). Similarly, the anti-inflammatory agents can be chosen from cyclosporine related drugs (e.g. calcineurin antagonist) including but not limited to members of the cyclosporine family, and other related calcineurin antagonists including sirolimus, tacorlimus and pimecrolimus. Alternatively, the antiinflammatory agents can be chosen from the group of NSAIDs including but not limited to acetaminophen, acemetacin, aceclofenac, alminoprofen, amfenac, bendazac, benoxaprofen, bromfenac, bucloxic acid, butibufen, carprofen, celecoxib, cinmetacin, clopirac, diclofenac, etodolac, etoricoxib, felbinac, fenclozic acid, fenbufen, fenoprofen, fentiazac, flunoxaprofen, flurbiprofen, ibufenac, ibuprofen, indomethacin, isofezolac, isoxicam, isoxepac, indoprofen, ketoprofen, lonazolac, loxoprofen, mefenamic acid, meclofenamic acid, meloxicam, metiazinic acid, mofezolac, miroprofen, naproxen, niflumic, oxaprozin, pirozolac, pirprofen, pranoprofen, protizinic acid, rofecoxib , salicylic acid and its derivatives(i.e. for example, aspirin), sulindac, suprofen, suxibuzone, triaprofenic acid, tolmetin, valdecoxib, xenbucin, ximoprofen, zaltoprofen, zomepirac, aspirin, acemetcin, bumadizon, carprofenac, clidanac, diflunisal, enfenamic acid, fendosal, flufenamic acid, flunixin, gentisic acid, ketorolac, mesalamine, and prodrugs thereof. Additionally, immunomodulators such as 6-mercaptopurine (6-MP), azathioprine (Imuran), methotrexate (Rheumatrex, Trexall), infliximab (Remicade), and adalimumab (Humira) may be used.

A class of therapeutic agents which may be useful to administer in combination, prior to, after, or concomitantly with the LFA-1 antagonists of the disclosure is antihistamines, including akylamine, ethanolamine and phenothiazine classes, such as, for example, chlorpheniramine maleate, chlorphenamiramine tannate, diphenhydramine hydrochloride, promethazine hydrochloride, acrivastine, azatadine maleate, azelastine hydrochloride, brompheniramine maleate, carbinoxamine maleate, cetirizine hydrochloride, clemastine fumarate, cyproheptadine hydrochloride, desloratadine, dexbrompheniramine maleate, dexchlorpheniramine maleate, dimenhydriunate, diphenhydramine hydrochloride, emedastine difumarate, fexofenadine hydrochloride, hydroxyzine hydrochloride, ketotifen fumarate, loratadine, meclizine hydrochloride, olopatadine hydrochloride, phenindamine tartrate, quetiapine, tripelennamine citrate, tripelennamine hydrochloride, and triprolidine hydrochloride. In some embodiments of the disclosure, the formulations administered nasally or to the eye include one or more antihistamines.

A class of therapeutic agents which may be useful to administer in combination, prior to, after, or concomitantly with the LFA-1 antagonists of the disclosure is mast cell stabilizers such as cromolyn sodium and nedocromil.

Oxidative stress may be induced in cells with impaired autoregulatory and ischemic processes induced by LFA-1 mediated immune disorders. Therefore, anti-oxidants may be useful to administer in combination, prior to, after, or concomitantly with the LFA-1 antagonists of the disclosure. Examples of suitable anti-oxidants useful in the methods of the disclosure include, but are not limited to, ascorbic acid, tocopherols, tocotrienols, carotinoids, glutathione, alpha-lipoic acid, ubiquinols, bioflavonoids, carnitine, and superoxide dismutase mimetics, such as, for example, 2,2,6,6-tetramethyl-1-piperidinyloxy (TEMPO), DOXYL, PROXYL nitroxide compounds; 4-hydroxy-2,2,6,6-tetramethyl-1-piperidinyloxy (Tempol), M-40401, M-40403, M-40407, M-40419,M-40484, M-40587, and M-40588.

In some embodiments of the disclosure, methods are provided wherein anti-apoptotic therapeutic agents may be administered in combination, prior to, after, or concomitantly with the LFA-1 antagonists of the disclosure. Examples of suitable anti-apoptotic agents are, for example, inhibitors of caspases, cathepsins, and TNF-α.

Another class of therapeutic agents which may be useful to administer in combination, prior to, after, or concomitantly with the LFA-1 antagonists of the disclosure are antimicrobial agents. Suitable antimicrobial compounds, include, but are not limited to, penicillins, such as, for example, amoxicillin, ampicillin, azlocillin, carbenicillin, cloxacillin, dicloxacillin, flucloxacillin, mezlocillin, nafcillin, penicillin, piperacillin, and ticarcillin; beta-lactamase inhibitors; carbapenems, such as, for example, ertapenem, imipenem, and meropenem; cephalosporins, such as, for example, cefaclor, cefamandole, cefoxitin, cefprozil, cefuroxime, cefixime, cefdinir, cefditoren, cefoperazone, cefotaxime, cefpodoxime, cefadroxil, ceftazidime, ceftibuten, ceftizoxime, ceffiriaxone, cefazolin, cefixime, cephalexin, and cefepime; quinolones, such as, for example, ciprofloxacin, enoxacin, gatifloxacin, levofloxacin, lomefloxacin, morifloxacin, norfloxacin, ofloxacin, and trovafloxacin, ; macrolides, such as, for example, azithromycin, clarithromycin, dirithromycin, erythromycin, milbemycin, and troleandomycin; monbactams, such as, for example, LFA-1 antagonist; tetracyclins, such as, for example, demeclocyclin, doxycycline, minocycline, oxytetracyclin, and tetracycline; aminoglycosides, such as, for example, amikacin, gentamicin, kanamycin, neomycin, netilmicin, paromomycin, streptomycin, and tobramycin; carbacephem, such as, for example, loracarbef; streptogramins; sulfonamides, such as, for example, mefanide, prontosil, sulfacetamide, sulfamethizole, sulfanilamide, sulfasalazine, sulfisoxazole, trimethoprim, and trimethoprim-sultamethoxazole; other antimicrobials such as metronidazole; and the combination drugs such as for example, sulfamethoxazole and trimethoprim.

Other antimicrobial agents include the class of antiviral agents. Antiviral agents include, but are not limited to therapeutic agents such as entry inhibitors, reverse transcriptase inhibitors, nucleoside or nucleotide analogs, protease inhibitors, and inhibitors of viral release from host cells. Some illustrative therapeutic agents of this group, include, but are not limited to abacavir, acyclovir, adefovir, amantadine, amprenavir, arbidol, atazanavir, atripla, brivudine, cidofovir, combivir, darunavir, delavirdine, didanosine, docosanol, edoxudine, efavirenz, emtricitabine, enfuvirtide, entecavir, famciclovir, fomivirsen, foscarnet, fosfonet, ganciclovir, gardasil, ibacitabine, imunovir, idoxuridine, imiquimod, indinavir, inosine, interferon type III, interferon type II, interferon type I, interferon, lamivudine, lopinavir, loviride, maraviroc, moroxydine, nelfinavir, neviapine, nexavir, oseltamivir, penciclovir, peramivir, pleconaril, podophyllotoxin, raltegravir, ribavirin, rimantadine, ritonavir, saquinavir, stavudine, tenofovir, tenofovir disoproxil, tipranavir, trifluridine, trizivir, tromantadine, truvada, valaciclovir, valganciclovir, vicriviroc, vidarabine, viramidine, zalcitabine, zanamivir, and zidovudine.

In some of the embodiments of the invention, the formulations administered to the skin comprise one or more antimicrobial or antibiotic agents.

Secretagogues may also be administered in combination, prior to, concomitantly with, or subsequent to administration of the LFA-1 antagonist. Increasing mucin or other fluid production in the eye may be beneficial. Examples include but are not limited to Diquafasol, Rebamipide, and Eicosanoid 15-(S)-HETE.

Additionally, lubricants may be be administered in combination, prior to, concomitantly with, or subsequent to ocular administration of the LFA-1 antagonist. Examples include but are not limited to Refresh Dry Eye Therapy® and other lubricating eye drops.

### Composition forms

The formulation may be in any form suitable for application to the body surface, such as a cream, lotion, solution, gel, ointment, paste, plaster, paint, or bioadhesive, and/or may be prepared so as to contain liposomes, micelles, and/or microspheres. Formulations for topical use of the pharmaceutical compositions of the present invention can be provided as a topical composition wherein the pharmacologically active ingredients are mixed with excipients to form a semisolid consistency. Examples of such topical pharmaceutical compositions include, but are not limited to, a gel, cream, lotion, suspension, emulsion, ointment, foam, and paste. Alternatively, the topical pharmaceutical compositions of the present invention can be formulated in a semi-liquid formulation. Examples of such topical pharmaceutical compositions include, but are not limited to, a topical solution, spray, mist, and drops. Alternatively, the topical pharmaceutical compositions of the present disclosure can be formulated in a dry powder form. The pharmaceutical compositions can also be administered by a transdermal patch.

Ointments, as is well known in the art of pharmaceutical formulation, are semi-solid preparations that are typically based on petrolatum or other petroleum derivatives. As an ointment, the composition has a consistency suitable for uniform dermal application. Additionally, the ointment may be substantially viscous to remain in contact with the skin regardless of perspiration, excess moisture or environmental conditions. The specific ointment base to be used, as will be appreciated by those skilled in the art, is one that will provide for optimum drug delivery, and, will provide for other desired characteristics as well, e.g., emolliency. As with other carriers or vehicles, an ointment base should be inert, stable, nonirritating and nonsensitizing. As explained in Remington: The Science and Practice of Pharmacy, 19th Ed. (Easton, Pa.: Mack Publishing Co., 1995), at pages 1399-1404, ointment bases may be grouped in four classes: oleaginous bases; emulsifiable-bases; emulsion bases; and water-soluble bases. Oleaginous ointment bases include, for example, vegetable oils, fats obtained from animals, and semisolid hydrocarbons obtained from petroleum. Emulsifiable ointment bases, also known as absorbent ointment bases, contain little or no water and include, for example, hydroxystearin sulfate, anhydrous lanolin and hydrophilic petrolatum. Emulsion ointment bases are either water-in-oil (W/O) emulsions or oil-in-water (O/W) emulsions, and include, for example, cetyl alcohol, glyceryl monostearate, lanolin, and stearic acid. Some water-soluble ointment bases are prepared from polyethylene glycols of varying molecular weight; again, see Remington: The Science and Practice of Pharmacy for further information.

Creams, as also well known in the art, are viscous liquids or semi-solid emulsions, either oil-in-water or water-in-oil. Cream bases are water-washable, and contain an oil phase, an emulsifier, and an aqueous phase. The oil phase, also called the "internal" phase, is generally comprised of petrolatum and a fatty alcohol such as cetyl or stearyl alcohol. The aqueous phase usually, although not necessarily, exceeds the oil phase in volume, and generally contains a humectant. The emulsifier in a cream formulation is generally a nonionic, anionic, cationic, or amphoteric surfactant.

Gels are semi-solid, suspension-type systems and are well known in the art. Gel forming agent for use herein can be any gelling agent typically used in the pharmaceutical art for topical semi solid dosage forms. Single-phase gels contain organic macromolecules distributed substantially uniformly throughout the carrier liquid, which is typically aqueous, but also can contain an alcohol and optionally an oil. In order to prepare a uniform gel, dispersing agents such as alcohol or glycerin can be added, or the gelling agent can be dispersed by tritration, mechanical mixing or stirring, or combinations thereof. The amount of gelling agents varies widely and will ordinarily range from about 0.1% to about 2.0% by weight, based on the total weight of the composition. The gel forming agent also works by the principle of copolymerization. Under alkaline pH, carbomer in presence of water undergoes cross linking and forms a gel like structure. The degree of polymerization is dependent upon the pH. At a threshold pH, the viscosities achieved by the polymer grade are the maximum.

Lotions, are preparations to be applied to the skin surface without friction, and are typically semi-liquid preparations in which solid particles, including the active agent, are present in a water or alcohol base. Lotions are usually suspensions of solids, and for the present purpose, comprise a liquid oily emulsion of the oil-in-water type. Lotions may be desirable formulations herein for treating large body areas, because of the ease of applying a more fluid composition. It is generally necessary that the insoluble matter in a lotion be finely divided. Lotions will typically contain suspending agents to produce better dispersions as well as compounds useful for localizing and holding the active agent in contact with the skin, e.g., methylcellulose, or sodium carboxymethyl-cellulose.

Pastes are semi-solid dosage forms in which the active agent is suspended in a suitable base. Depending on the nature of the base, pastes are divided between fatty pastes or those made from a single-phase aqueous gels. The base in a fatty paste is generally petrolatum or hydrophilic petrolatum. The pastes made from single-phase aqueous gels generally incorporate carboxymethylcellulose as a base.

Plasters are comprised of a pasty mixture that is spread on the body, either directly or after being saturated into a base material such as cloth. Medications, including the pharmacologically active bases of the disclosure, may be dissolved or dispersed within the plaster to make a medicated plaster.

Bioadhesives are preparations that adhere to surfaces of body tissues. Polymeric bioadhesive formulations are well known in the art; see, for example, Heller et al., "Biodegradable polymers as drug delivery systems", in Chasin, M. and Langer, R., eds.: Dekker, N.Y., pp.121-161 (1990); and U.S. Pat. No. 6,201,065. Suitable non-polymeric bioadhesives are also known in the art, including certain fatty acid esters (U.S. Pat. No. 6,228,383).

### Methods of treatment using topically formulated LFA-1 antagonists

Compounds of the disclosure are therapeutically and/or prophylactically useful for treating diseases or conditions mediated by LFA-1 activity. Accordingly, in one aspect, a method is provided for treatment of an inflammatory or immune related disorder in a subject comprising topically administering to said subject in need thereof a formulation comprising an LFA-1 antagonist or a pharmaceutically acceptable salt or ester thereof, and a pharmaceutically acceptable excipient, wherein the LFA-1 antagonist has a systemic clearance rate greater than about 2 mL/min/kg when administered to a subject.

The benefits of topical administration include localized delivery of the therapeutic agent and minimal systemic side effects due to low systemic bioavailability. For example, topical formulations of the invention may be administered directly to the skin, eye, mouth, nose, vaginal mucosa or anal mucosa. The methods of topical delivery of the formulations of the present invention are particularly well suited for localized administration of the formulation. Suitable formulations and additional carriers are discussed herein and, additionally, described in Remington "The Science and Practice of Pharmacy" (20th Ed., Lippincott Williams & Wilkins, Baltimore MD).

One advantage of the therapeutic composition according to the invention is that topical application is particularly convenient for treating and preventing a variety of dermal conditions. Therapeutic compositions may be noninvasively applied directly to the site of interest. Other disorders conveniently addressed by topical administration include allergic conditions of the nasal passageway, eye, and oral cavity. Localized delivery of LFA-1 antagonist to the eye can be acheived via drop or spray into eye or tears. The drug then distributes either via peri-ocular soft tissue or via distribution through the sclera or across corneal epithelium, gastointestinal disorders such as IBD and Crohn's Disease may also be usefully treated by localized treatment according to the methods of the disclosure. In treating such disease, the LFA-1 antagonist is administered orally, but is delivered only in the GI tract where the formulation permits the drug to dissolve in GI fluid. The LFA-1 antagonist is then distributed to the surface of the GI mucosa, whereupon the LFA-1 antagonist penetrates through intestinal epithelium to local adjacent tissue. The fluids in the GI tract having high levels of drug will travel down the GI tract with normal GI motility and gastric flow and coat the effected surface of GI along the way. Additionally, LFA-1 antagonist that does distribute out of local intestinal tissue and into the vasculature is swept to the liver and delivered via bile into the lower GI tract.

In some embodiments, therapeutic agents of the disclosure have a rapid systemic clearance such that any drug that gets absorbed systemically is quickly cleared. In some embodiments, the LFA-1 antagonist may have a
systemic clearance rate of greater than about 1 mL/min/kg , about 2 mL/min/kg, about 3 mL/min/kg, about 4 mL/min/kg, about 5 mL/min/kg, about 6 mL/min/kg, about 7 mL/min/kg, about 8 mL/min/kg, about 9 mL/min/kg, about 10 mL/min/kg, about 11 mL/min/kg, about 12 mL/min/kg, about 13 mL/min/kg, about 14 mL/min/kg, about 15 mL/min/kg, about 16 mL/min/kg, about 17 mL/min/kg, about 18 mL/min/kg, about 19 mL/min/kg, about 20 mL/min/kg, about 25 mL/min/kg, about 30 mL/min/kg, about 35 mL/min/kg, about 40 mL/min/kg, about 45 mL/min/kg, about 50 mL/min/kg, about 60 mL/min/kg, about 65 mL/min/kg, about 70 mL/min/kg, about 75 mL/min/kg, about 80 mL/min/kg, about 85 mL/min/kg, about 90 mL/min/kg, about 95 mL/min/kg, or about 100 mL/min/kg.

It is known that LFA-1 interacts with several ligands which could result in several unwanted side effects. Thus in some embodiments, the local concentration of therapeutic agent is about 2X, 3X, 4X, 5X, 10X, 25X, 50X, or 100X greater than the systemic concentration. In another embodiment of the current disclosure, local concentration of LFA-1 antagonist is 1000x greater than the systemic concentration. In one embodiment, the local concentration is about 10,000x or more greater than the systemic concentration at the same time point. The concentration of therapeutic agent may be measured using any known method in the art. For example, radiolabelled therapeutic drug may be used and measurements taken from the local site of administration compared to systemic levels (e.g. plasma level concentrations).

The compositions may be delivered with a pharmacokinetic profile that results in the delivery of an effective dose of the LFA-1 antagonist. The actual effective amounts of drug can vary according to the specific drug or combination thereof being utilized, the particular composition formulated, the mode of administration, and the age, weight, condition of the patient, and severity of the symptoms or condition being treated. Dosages for a particular patient can be determined by one of ordinary skill in the art using conventional considerations, (e.g. by means of an appropriate, conventional pharmacological protocol).

In some embodiments, the LFA-1 antagonist achieves a local tissue concentration of greater than about 1 µM within about 4 hours following administration to a subject. In other embodiments, the LFA-1 antagonist achieves a local tissue concentration of greater than about 1 µM within about 3 hours following administration to a subject. In other embodiments, the LFA-1 antagonist achieves a local tissue concentration of greater than about 1 µM within about 2 hours following administration to a subject. In other embodiments, the LFA-1 antagonist achieves a local tissue concentration of greater than about 1 µM within about 1 hour following administration to a subject. In other embodiments, the LFA-1 antagonist achieves a local tissue concentration of greater than about 1 µM within about 50 min, about 40 min, about 30 min, about 20 min, about 10 min, about 5 min, or about 3 minutes following administration to a subject. In some embodiments, the LFA-1 antagonist achieves a local tissue concentration in skin of greater than about 1 µM within about 4 hours following administration to a subject. In other embodiments, the LFA-1 antagonist achieves a local tissue concentration in skin of greater than about 1 µM within about 6 hours, about 5.5 hours, about 5 hours, about 4.5 hours, about 3.5 hours, about 3.0 hours, or about 2.5 hours following administration to a subject, In some embodiments, the LFA-1 antagonist achieves a local retina and/or intraocular tissue concentration of greater than about 1 µM within about 180 min, about 170 min, about 160-min, about 150 min, about 140 min, about 130 min, about 120 min, about 110 min, about 100 min, about 90 min, about 80 min, about 70 min, about 60 min, about 50 min, about 40 min, about 30 min or about 20 min following administration to a subject. In some embodiments, the LFA-1 antagonist is administered to the eye as an eyedrop to deliver LFA-1 antagonist to the retina and/or intraocular tissue. In other embodiments, the LFA-1 antagonist
achieves a local tear and/or corneal surface concentration of greater than about 1 µM within about 60 min, about 50 min, about 40 min, about 30 min, about 20 min, about 19 min, about 18min, about 17min, about 16 min, about 15 min, about 14 min, about 13 min, about 12 min, about 11 min, about 10 min, about 9 min, about 8 min, about 7min, about 6 min, about 5 min, about 4 min, about 3 min, about 2 min or about 1 min following administration to a subject. In some embodiments, the LFA-1 antagonist is administered to the eye as an eyedrop to deliver LFA-1 antagonist to the tears and/or corneal surface.

After the formulation of the invention is topically administered as described above, the LFA-1 antagonist distributes to local tissue and is present in a therapeutically effective concentration within about 1 mm of an epithelial surface to which the the formulation is applied. In some embodiments wherein the formulation is topically administered, the LFA-1 antagonist is present in a therapeutically effective concentration within about 2 mm, about 3 mm, about 4 mm, about 5 mm, about 6 mm, about 7 mm, about 8 mm, about 9 mm, about 10 mm, about 12 mm, about 14 mm, about 16 mm, about 18 mm, about 20 mm, about 30 mm, about 40 mm, or about 50 mm of an epithelial surface to which the formulation is applied. In embodiments, wherein the formulations of the invention are orally administered, the LFA-1 antagonist is released in the GI tract and is present in a therapeutically effective concentration within about 1 mm of an epithelial surface to which the LFA-1 antagonist is distributed from the GI tract. In some other embodiments, wherein the formulations of the invention are orally administered, the LFA-1 antagonist is released in the GI tract and is present in a therapeutically effective concentration within about 2 mm, about 3 mm, about 4 mm, about 5 mm, about 6 mm, about 7 mm, about 8 mm, about 9 mm, about 10 mm, about 12 mm, about 14 mm, about 16 mm, about 18 mm, about 20 mm, about 30 mm, about 40 mm, or about 50 mm of an epithelial surface to which the LFA-1 antagonist is distributed from the GI tract.

In some embodiments, the LFA-1 antagonist has a local tissue concentration of greater than about 10 nM within about 4 hours following administration to the subject. In other embodiments, the LFA-1 antagonist has a local tissue concentration of greater than about 20 nM, about 30 nM, about 40 nM, about 50 nM, about 75 nM, about 100 nM, about 150 nM, about 200 nM, about 150 nM, about 300 nM, about 400 nM, about 500 nM, about 600 nM, about 700 nM, about 800 nM, about 900 nM, about 1 µM, about 2 µM, about 3µM, about 4µM, about 5 µM, about 6µM, about 7µM, about 8µM, about 9µM, or about 10µM within about 4 hours following administration to the subject. In yet other embodiments, the LFA-1 antagonist has a local tissue concentration of greater than about than about 10 nM, about 20 nM, about 30 nM, about 40 nM, about 50 nM, about 75 nM, about 100 nM, about 150 nM, about 200 nM, about 150 nM, about 300 nM, about 400 nM, about 500 nM, about 600 nM, about 700 nM, about 800 nM, about 900 nM, about 1 µM, about 2 µM, about 3µM, about 4µM, about 5 µM, about 6µM, about 7µM, about 8µM, about 9µM, or about 10µM within about 5 hours following administration to the subject. The disclosure also provides methods wherein the LFA-1 antagonist has a local tissue concentration of greater than about than about 10 nM, about 20 nM, about 30 nM, about 40 nM, about 50 nM, about 75 nM, about 100 nM, about 150 nM, about 200 nM, about 150 nM, about 300 nM, about 400 nM, about 500 nM, about 600 nM, about 700 nM, about 800 nM, about 900 nM, about 1 µM, about 2 µM, about 3µM, about 4µM, about 5 µM, about 6µM, about 7µM, about 8µM, about 9µM, or about 10µM within about 3 hours following administration to the subject. The LFA-1 antagonist may also have a local tissue concentration of greater than about than about 10 nM, about 20 nM, about 30 nM, about 40 nM, about 50 nM, about 75 nM, about 100 nM, about 150 nM, about 200 nM, about 150 nM, about 300 nM, about 400 nM, about 500 nM, about 600 nM, about 700 nM, about 800 nM, about 900 nM, about 1 µM, about 2 µM, about 3µM, about 4µM, about 5 µM, about 6µM, about 7µM, about 8µM, about 9µM, or
about 10µM within about 2 hours following administration to the subject. In other embodiments, the LFA-1 antagonist has a local tissue concentration of greater than about than about 10 nM, about 20 nM, about 30 nM, about 40 nM, about 50 nM, about 75 nM, about 100 nM, about 150 nM, about 200 nM, about 150 nM, about 300 nM, about 400 nM, about 500 nM, about 600 nM, about 700 nM, about 800 nM, about 900 nM, about 1 µM, about 2 µM, about 3µM, about 4µM, about 5 µM, about 6µM, about 7µM, about 8µM, about 9µM, or about 10µM within about 1 hour following administration to the subject. In some other embodiments, the LFA-1 antagonist has a local tissue concentration of greater than about than about 10 nM, about 20 nM, about 30 nM, about 40 nM, about 50 nM, about 75 nM, about 100 nM, about 150 nM, about 200 nM, about 150 nM, about 300 nM, about 400 nM, about 500 nM, about 600 nM, about 700 nM, about 800 nM, about 900 nM, about 1 µM, about 2 µM, about 3µM, about 4µM, about 5 µM, about 6µM, about 7µM, about 8µM, about 9µM, or about 10µM within about 50 min, about 40 min, about 30 min, abourt 20 min, about 10 min, about 9 min, about 8 min, about 7 min, about 6 min, about 5 min, about 4 min, about 3 min, about 2 min, or about 1 min following administration to the subject.

In some of the methods of the disclosure, the LFA-1 antagonist maintains a local tissue concentration of greater than about 10 nM for at least about 8 hours following administration. In other embodiments, the LFA-1 antagonist maintains a local tissue concentration of greater than about 10 nM, about 20 nM, about 30 nM, about 40 nM, about 50 nM, about 75 nM, about 100 nM, about 150 nM, about 200 nM, about 150 nM, about 300 nM, about 400 nM, about 500 nM, about 600 nM, about 700 nM, about 800 nM, about 900 nM, or about 1 µM, for at least about 8 hours following administration. In other embodiments, the LFA-1 antagonist maintains a local tissue concentration of greater than about 10 nM, about 20 nM, about 30 nM, about 40 nM, about 50 nM, about 75 nM, about 100 nM, about 150 nM, about 200 nM, about 150 nM, about 300 nM, about 400 nM, about 500 nM, about 600 nM, about 700 nM, about 800 nM, about 900 nM, or about 1 µM, for at least about 10 hours, about 9 hours, about 8 hours, about 7 hours, about 6 hours, about 5 hours, about 4 hours, about 3 hours, about 2 hours, or about 1 hour following administration.

In some of the methods of the disclosure, the LFA-1 antagonist has a local tissue concentration of greater than about 1 µM and a systemic concentration as measured in plasma of less than about 100 nM, within about 4 hrs following administration. In other embodiments, the LFA-1 antagonist has a local tissue concentration of greater than about 1 µM and a systemic concentration as measured in plasma of less than about 80 nM, about 70 nM, about 60 nM or about 50 nM, within about 4 hours, about 3 hours, about 2 hours, about 1 hour, about 50 min, about 40 min, about 30 min, about 20 min, about 10 min, or about 5 min following administration.

Additionally, in some of the methods of the disclosure, the LFA-1 antagonist is present in a therapeutically effective concentration within about 1mm of an epithelial surface to which the formulation is applied and is present in blood plasma below a therapeutically effective level, within about 4 hrs following administration. In other embodiments, the LFA-1 antagonist is present in a therapeutically effective concentration within about 2 mm, about 3 mm, about 4 mm, about 5 mm, about 6 mm, about 7 mm, about 8 mm, about 9 mm, about 10 mm, about 12 mm, about 14 mm, about 16 mm, about 18 mm, about 20 mm, about 30 mm, about 40 mm, or about 50 mm of an epithelial surface to which the formulation is applied and is present in blood plasma below a therapeutically effective level, within about 4 hrs following administration. Alternatively, the LFA-1 antagonist may be present in a therapeutically effective concentration within about 1mm of an epithelial surface to which the formulation is applied and is present in blood plasma below a therapeutically effective level, within about 6 hours, about 5 hours, about 3 hours, about 2 hours, about 1 hour, about 50 min, about 40 min, about 30 min, about 20 min, about 10 min or about 5 min following administration.

The disclosure provides methods for the treatment of the inflammatory component of immune and other disorders in a subject. In particular, the methods described herein are useful for the treatment of leukocyte mediated inflammation. The formulations of the invention are potent inhibitors of LFA-1 and inhibit cytokines released by Th1 T-cells and Th2 T-cells. Leukocyte mediated inflammation plays a role in initiating and advancing inflammation in selected diseases, such as T cell inflammatory responses. The methods generally involve the administration of one or more drugs for the treatment of one or more diseases. Combinations of agents can be used to treat one disease or multiple diseases or to modulate the side-effects of one or more agents in the combination.

The compounds described herein can be used in combination with other agents such as agents to treat immune related disorders. Also, the compounds of the disclosure can be used in conjunction with other drugs in order to counteract certain effects, e.g. LFA-1 antagonists may be administered with drugs that cause dry eye as a side effect.

### Uses of the invention

The LFA-1 antagonists of the present disclosure may be used to treat a variety of immune related disorders. LFA-1 has been implicated in a number of immune related disorders. In particular, the methods described herein are useful for the treatment of leukocyte mediated inflammation. Leukocyte mediated inflammation plays a role in initiating and advancing inflammation in selected diseases, such as T cell inflammatory responses. Local administration of LFA-1 antagonists may be particularly effective in disease states where systemic administration of anti-LFA-1 monoclonal antibodies has proven effective (see Raptiva clinical trials at www.clinicaltrials.gov. Raptiva has shown effect in psoriasis, eczema, kidney and islet cell transplant.).

Immune related disorders involving LFA-1 include eye disorders, such as intraocular, periocular and ocular surface inflammation: Keratoconjunctivitis, keratoconjunctivitis sicca (KCS, aka Dry Eye), KCS in patients with Sjogren's syndrome, allergic conjunctivitis, uveitis; inflammation of the eye, the cornea and periocular tissue from contact lens wear; inflammation of the eye following surgery including lasik; intraocular inflammation including inflammation of the retina and the anterior and posterior segments of the eye, inflammation of the meibomian gland, age related macular degeneration (AMD), uveitis, edema and retinopathies including diabetic macular edema and diabetic retinopathy; corneal inflammation including rejection of corneal transplants, Graves ophthalmopathy, age-related dry eye, Stevens-Johnson syndrome, congenital alachrima, pharmacological side effects, infection, Riley-Day syndrome, conjunctival fibrosis, eye stress, glandular and tissue destruction, ocular cicatrical pemphogoid, blepharitis, autoimmune and other immunodeficient disorders, allergies, lacrimal gland deficiency, lupus, rheumatoid arthritis, rosacea, environmental exposure to excessively dry air, airborne particulates, smoke, and smog and inability to blink, amongst others. Other immune related disorders include allergic diseases such as allergic conjunctivitis, allergic asthma, dermatitis such as atopic dermatitis, eczema, allergic rhinitis, allergic conjunctivitis, food hypersensitivity and allergic contact dermatitis. Other immune related disorders include inflammatory diseases such as skin hypersensitivity reactions (including poison ivy and poison oak). Other immune related disorders include dermatologic inflammatory diseases such as eczema, atopic dermatitis, psoriasis, bullous skin diseases, irritant contact dermatitis and further eczematous dermatitises, seborrhoeic dermatitis and cutaneous manifestations of immunologically-mediated disorders. Other immune related disorders include autoimmune diseases such as Sjogren's syndrome including Dry Eye, Dry Mouth and other local
inflammations associated with Sjogren's syndrome and rheumatoid arthritis. Other immune related disorders include transplantation related disorders such as acute or chronic rejection of cell, tissue or organ allo- or xenografts or delayed graft function, graft versus host disease. Examples of cell, tissue or solid organ transplants include e.g. corneal tissue. Other immune related disorders include, but are not limited to alopecia areata, diabetic retinopathy, chronic obstructive pulmonary disease (COPD), atopic dermatitis, inflammation from kidney transplant, asthma, hidradentis supporativa, rheumatoid arthritis, psoriatic arthritis, Sjogren's Syndrome, uveitis, Graft vs. Host disease (GVHD), Oral Lichen Planus, arthralgia or Islet Cell Transplant inflammation, and post surgical inflammation of the eye.

The present disclosure is also useful in treating inflammatory bowel disease, or IBD. IBD refers to any of a variety of diseases typically characterized by inflammation of all or part of the intestines. Examples of inflammatory bowel disease include, but are not limited to, Crohn's disease, ulcerative colitis, irritable bowel syndrome, mucositis, radiation induced enteritis, short bowel syndrome, celiac disease, colitis, stomach ulcers, diverticulitis, pouchitis, proctitis, and chronic diarrhea. Reference to IBD is exemplary of gastrointestinal inflammatory conditions, and is not meant to be limiting.

Another embodiment of this disclosure is for the treatment of eye disorders. The topical formulations of the present disclosure may be applied directly to the eye. For example, the methods of the present disclosure are useful for treatment of intraocular, periocular and ocular surface inflammation: Keratoconjunctivitis, keratoconjunctivitis sicca (KCS, aka Dry Eye), KCS in patients with Sjogren's syndrome, allergic conjunctivitis, uveitis; inflammation of the eye, the cornea and periocular tissue from contact lens wear; inflammation of the eye following surgery including lasik; intraocular inflammation including inflammation of the retina and the anterior and posterior segments of the eye, inflammation of the meibomian gland, meibomian gland dysfunction, age related macular degeneration (AMD), uveitis, edema and retinopathies including diabetic macular edema and diabetic retinopathy; corneal inflammation including rejection of corneal transplants, Graves ophthalmopathy, age-related dry eye, Stevens-Johnson syndrome, congenital alachrima, pharmacological side effects, infection, Riley-Day syndrome, conjunctival fibrosis, eye stress, glandular and tissue destruction, ocular cicatrical pemphogoid, blepharitis, autoimmune and other immunodeficient disorders, allergies, diabetes, lacrimal gland deficiency, lupus, Parkinson's disease, rheumatoid arthritis, rosacea, environmental exposure to excessively dry air, airborne particulates, smoke, and smog and inability to blink, amongst others.

Diabetes affects nearly 200 million persons worldwide and 20 million in the United States. Diabetic retinopathy, the microvascular complications of diabetes, is the leading cause of blindness in working-aged persons in the U.S. The prevalence of DR increases with duration of disease. After 20 years, approximately 100% of Type I patients develop DR and approximately 60% of Type II patients develop DR. DR can be classified into 2 stages: *non-proliferative* and *proliferative.* Diabetic macular edema (DME), a manifestation of DR, can occur at any stage and is the principal cause of vision loss. DME is characterized by increased vascular permeability and hard exudates.

For topical administration, all the formulations for topical ocular administration used in the field of ophthalmology (e.g., eye drops, inserts, eye packs, impregnated contact lenses, pump delivery systems, dimethylsulfoxide (DMSO)-based solutions suspensions, liposomes, and eye ointment) and all the formulations for external use in the fields of dermatology and otolaryngology (e.g., ointment, cream, gel, powder, salve, lotion, crystalline forms, foam, and spray) may be utilized as is known in the art. Additionally all suitable formulations for
topical administration to skin and mucus membranes of the nasal passages may be utilized to deliver the compounds of the disclosure. The pharmaceutical compositions of the present invention may be a liposomal formulation for topical or oral administration, any of which are known in the art to be suitable for the purpose of this invention.

Another embodiment is treatment of allergic diseases. The formulations of the present invention may be applied topically directly, for example, to the eyes, nose, mouth, skin, vaginal mucosa or anal mucosa. The methods of the present disclosure are useful for treatment of allergic conjunctivitis, vernal conjunctivitis, allergic asthma, atopic dermatitis, eczema, allergic rhinitis, allergic conjunctivitis and allergic contact dermatitis.

Allergic conjunctivitis is predominantly a disease of young adults that is characterized by ocular itching, redness, conjunctival edema, eyelid swelling, and watery discharge from eyes and nasal passages. Although not vision-threatening, patients suffering from allergic conjunctivitis tend to have impaired social functioning and emotional well-being and increased utilization of healthcare resources (Blaiss, 2006, Allergy Asthma Proc.). Ocular allergy is estimated to affect approximately 20% of the US population and the incidence is increasing (Abelson, 2003, Ocul Surf).

The conjunctiva is a mucosal surface that is highly exposed to environmental allergens and is often the first site of contact with airborne allergens in atopic individuals. Following antigen exposure, conjunctival mast cells degranulate, triggered by the antigen cross-linking of IgE antibodies on the cell surface (Bielory, 2005, Drugs). Mast cells release newly formed and pre-existing inflammatory mediators. Histamine is a primary preformed mediator responsible for the typical early phase reaction (EPR) that triggers itching (ocular pruritus), vasodilation, and vascular leak leading to ocular hyperemia, chemosis, and blepharitis. The EPR occurs within minutes to hours upon allergen exposure. Mast cells also synthesize and release cytokines IL-4, IL-5, PAF, and TNFα. The release of cytokines, chemokines, and growth factors initiates a cascade of inflammatory events including increased expression of ICAM-1 on the surface of epithelial cells, leading to a late phase reaction (LPR) with LFA-1/ICAM-1-macrophages into the conjunctival tissues (Ciprandi, 1993, J Allery Clin Immunol), (Bacon, 2000, J Allergy Clin Immunol). Allergic subjects (but not normal subjects) express ICAM-1 on conjuctival epithelium within 30 minutes after allergen challenge, which increases 3-fold over the first 24 hours.

While currently approved treatments (e.g., anti-histamines, MCS) for ocular allergy are centered primarily at reducing signs or symptoms of the EPR, there is emerging evidence to suggest that many patients exhibit clinical evidence of persistent LPR (Choi, 2008, Curr Opin Allergy Clin Immunol). Manifestations of the LPR occur approximately 6-24 hours after allergen exposure and are characterized by the prolongation of ocular signs and symptoms as well as the histologic influx of acute inflammatory cells, particularly eosinophils, into the conjunctiva. Topical steroids have been used to manage chronic ocular inflammation and refractory disease that is not adequately controlled with anti-histamines/MCS. However, only short courses of steroid therapy can be used due to the increased risk of potential side effects (e.g., cataract formation, glaucoma).

Compound 12 may be instrumental in blocking the LFA-1/ICAM-1 interaction and provide an alternative therapy for reducing ocular inflammation, treating LPR, and avoiding the safety issues associated with topical steroid administration. In murine conjunctival allergen challenge models, significant reductions in both the clinical signs and eosinophil/neutrophil infiltration into the conjunctiva have been demonstrated when animals received prophylactic treatment with systemically administered anti-ICAM-1 and/or anti-LFA-1 antibodies (Whitcup, 1999, Clin Immunol). Furthermore, mast cells appear to require LFA-1/ICAM-1-mediated contact with activated T-cells for degranulation. In vitro studies have shown that the degree of activated T-cell adhesion to mast cells decreases when T-cells are pre-treated with anti-LFA-1 antibody (Mekori, 1999, J Allergy Clin Immunol), (Brill, 2004, Clin Exp Allergy).

Yet another embodiment is treatment of dermatologic inflammatory diseases. The topical formulations of the present invention may be applied directly, for example, to the skin, eye, mouth, nose, vaginal mucosa or anal mucosa. For example, the methods of the present disclosure are useful for treatment of eczema, atopic dermatitis, psoriasis, irritant contact dermatitis and further eczematous dermatitises, seborrhoeic dermatitis and cutaneous manifestations of immunologically-mediated disorders.

Not intending to limit the mechanism of action, the methods of the present disclosure involve the inhibition of initiation and progression of inflammation related disease by inhibiting the interaction between LFA-1 and ICAM-1. LFA-1 and ICAM-1 are molecules with extracellular receptor domains which are involved in the process of lymphocyte/leukocyte migration and proliferation, leading to a cascade of inflammatory responses. In some embodiments, such methods provide anti-inflammatory effects *in-vitro* and *in-vivo*, e.g., as described in more detail below, and are useful in the treatment of inflammation mediated diseases, for example, asthma, eczema or dry eye disease.

Human blood contains white blood cells (leukocytes) which are further classified as neutrophils, lymphocytes (with B- and T- subtypes), monocytes, eosinophils, and basophils. Several of these classes of leukocytes, neutrophils, eosinophils, basophils and lymphocytes, are involved in inflammatory disorders. LFA-1 is one of a group of leukointegrins which are expressed on most leukocytes, and is considered to be the lymphoid integrin which interacts with a number of ICAMs as ligands. Disrupting these interactions, and thus the immune/inflammatory response provides for reduction of inflammation, for example, asthma, eczema or inflammation of the eye.

For example, ICAM-1 (CD54) is a member of the ICAM family of adhesion receptors (ICAM-1, ICAM-2, ICAM-3, ICAM-4) in the immunoglobulin protein super family, and is expressed on activated leukocytes, dermal fibroblasts, and endothelial cells. See Krensky, A.M.; Sanchez-Madrid, F.; Robbins, E.; Nagy, J.A.; Springer, T.A. Burakoff, S.J. "The functional significance, distribution, and structure of LFA-1, LFA-2, and LFA-3: cell surface antigens associated with CTL-target interactions." 1983 J. Immunol. 131, 611-616. It is normally expressed on the endothelial cells lining the vasculature, and is upregulated upon exposure to cytokines or compounds which induce cytokine release such as IL-1, LPS, SEB and TNF during immune/inflammatory initiation.

Research conducted over the last decade has helped elucidate the molecular events involved in the movement and activation of cells in the immune system, focusing on cell-to-cell triggering interactions within the cascade. See Springer, T.A. "Adhesion receptors of the immune system." Nature, 1990, 346, 425-434. The interaction of Intercellular Adhesion Molecules (ICAMs) with leukointegrins plays a role in the functioning of the immune system. It is believed that immune processes such as antigen presentation, T-cell mediated cytotoxicity and leukocyte transendothelial migration (diapedesis) require cellular adhesion mediated by ICAMs interacting with leukointegrins. See Kishimoto, T. K.; Rothlein; R. R. "Integrins, ICAMs, and selectins: role and regulation of adhesion molecules in neutrophil recruitment to inflammatory sites." Adv. Pharmacol. 1994, 25, 117-138 and Diamond, M.; Springer, T.A."The dynamic regulation of integrin adhesiveness." Current Biology, 1994, 4, 506-532.

The interaction of ICAM-1 and LFA-1 (also referred to as α_{L}β₂ and CD11a/CD18) has been shown to be involved in the processes of adhesion, leukocyte transendothelial migration, migration to sites of injury, and proliferation of lymphocytes at the activated target site. For example, it is presently believed that prior to leukocyte transendothelial migration, a component of the inflammatory response, the presence of cytokines/chemokines activate integrins constitutively expressed on leukocytes. Blood vessel endothelial cells also upregulate ICAM-1 in response to the presence of the same cytokines/chemokines. As rolling leukocytes approach activated endothelial cells, their progress is first slowed by these upregulated ICAM-1 receptors. This is followed by a ligand/receptor interaction between LFA-1 and ICAM-1, expressed on blood vessel endothelial cell surfaces, which arrests the lymphocyte from rolling further. The lymphocyte then flattens, and transvasation takes place. This process is of importance both in lymphocyte transmigration through vascular endothelial as well as lymphocyte trafficking from peripheral blood to lymph nodes.

LFA-1 plays a role in creating and maintaining the immunological synapse, which may be defined as the physical structure of the interacting surfaces of T cells and Antigen Presenting Cells (APCs). LFA-1 stabilizes T-cell engagement with the APC, and thus leads to activation of T cells. The interaction of LFA-1 and ICAM-1 also appears to provide co-stimulatory signals to resting T cells. CD4+ T-cell proliferation and cytokine synthesis are mediated by this interaction as part of the inflammatory response.

Given the role that the interaction of ICAM-1 and LFA-1 plays in immune/inflammatory response, it is desirable to modulate these interactions to achieve a desired therapeutic result (e.g., inhibition of the interaction in the event of an overactive inflammatory response). It has been demonstrated that the antagonism of the interaction between ICAMs and leukointegrins can be realized by agents directed against either component, particularly with monoclonal antibodies.

Also, since LFA-1 has several ligand partners within the ICAM family (ICAM-1, ICAM-2 and ICAM-3), involving a number of signaling pathways, in some embodiments of the invention, it is desirable to modulate these interactions selectively.

The methods and compositions described herein can modulate one or more components of the pathways described herein. In addition to inhibiting interaction between LFA-1 and ICAM-1, the methods and compositions of the present disclosure may also intervene in either earlier or later portions of the inflammatory process as well. For example, upregulation of ICAM-1 or LFA-1 (activation) on endothelial cells or leukocytes, prior to tethering and transendothelial migration, may be modulated by the methods and compositions described herein. The present disclosure may be useful in modulating the expression of cytokines or chemokines that activate ICAM-1 and LFA-1 in the course of leukocyte trafficking, in modulating the transport of the cytokines or chemokines, in preventing transvasation of the arrested leukocyte, in modulating signalling via other mechanisms that are involved in leukocyte proliferation at the site of injury or inflammation.

### Administration

The method of delivery of the pharmaceutically active composition may vary, but necessarily involves application of a formulation of the invention to an area of body surface affected with an inflammatory dermatosis. In the methods of the disclosure, the formulation is topically applied to skin, eyes, mouth, nose, vaginal mucosa or anal mucosa. A cream, ointment, paste, plaster, or lotion may be spread on the affected area of skin and gently rubbed in. Similarly, a polymeric or other bioadhesive formulation may be spread or dabbed on the affected area of skin. A solution may be applied in the same ways, but more typically will be applied with a dropper, or swab, and carefully applied to the affected area of skin. Petrolatum may be spread on the skin surrounding the affected area of skin to protect it from possible irritation during treatment.

The dosing regimen will depend on a number of factors that may readily be determined, such as the size of the affected area, the severity of the dermatosis, and the responsiveness of the inflammatory dermatosis to treatment, but will normally be one or more doses per day, with a course of treatment lasting from several days to several months, or until a cure is effected or a significant diminution in the size and/or severity of the inflammatory dermatosis is achieved. Local administration of an LFA-1 antagonist that is rapidly cleared from the systemic circulation may have particular benefit for patitients with inflammatory diseases affecting large areas. In this scenario, patients may be able to treat large areas without significant immunosuppression and risk of side effects due to systemic exposure to drug. One of ordinary skill may readily determine optimum dosages, dosing methodologies, and repetition rates. In general, it is contemplated that the formulation will be applied one to four times daily. With a skin patch, the device is generally maintained in place on the body surface throughout a drug delivery period, typically in the range of 8 to 72 hours, and replaced as necessary.

In some embodiments, the LFA-1 antagonist is present in an amount sufficient to exert a therapeutic effect to reduce symptoms of an immune related disorder by an average of at least about 5, 10, 15, 20, 25, 30, 40, 50, 60, 70, 80, 90, more than 90%, or substantially eliminate symptoms of the immune related disorder. For many inflammatory diseases, there are well recognized clinical assessments of therapeutic effect (e.g. PASI score for psoriasis and EASI score for eczema)

In some embodiments, the LFA-1 antagonist is present in an amount sufficient to decrease neovascularization and erythema in a treated individual by an average of at least about 5, 10, 15, 20, 25, 30, 40, 50, 60, 70, 80, 90, more than 90%, or substantially eliminate neovascularization.

In some embodiments, the LFA-1 antagonist is present in an amount sufficient to decrease fibrovascular growth of an individual by an average of at least about 5, 10, 15, 20, 25, 30, 40, 50, 60, 70, 80, 90, more than 90%, or substantially eliminate fibrovascular growth.

In some embodiments, an effective amount of the LFA-1 antagonist is a dose of about 1x 10⁻¹¹, 1x 10⁻¹⁰, 1x 10⁻⁹, 1x 10⁻⁸, 1x 10⁻⁷, 1x 10⁻⁶, 1x 10⁻⁵, 1x 10⁻⁴, 1x 10⁻³, 1x 10⁻², 1x 10⁻¹, 1, 1x 10¹ or 1x 10² grams.

A method for treatment of immune system disorders comprises administration of the formulations of the present invention in topical form.

The total daily doses of the medicaments contemplated for use with this invention, and consequently the concentrations by weight of the medicaments in the respective compositions, may vary widely, but are within the typical skill of the routine practitioner.

In some embodiments, the LFA-1 antagonist is administered in a single dose. A single dose of a LFA-1 antagonist may also be used when it is co-administered with another substance (e.g., an analgesic) for treatment of an acute condition.

In some embodiments, the LFA-1 antagonist (by itself or in combination with other drugs) is administered in multiple doses. Dosing may be about once, twice, three times, four times, five times, six times, seven times, eight times, nine times, ten times or more than ten times per day. Dosing may be about once a year, twice a year, every six months, every 4 months, every 3 months, every 60 days, once a month, once every two weeks, once a week, or once every other day. In one embodiment the drug is an analgesic. In another embodiment the LFA-1 antagonist and another therapeutic substance are administered together about once per day to about 10 times per day. In another embodiment, an additional therapeutic substance is administered concurrent with, prior to, or subsequent to administering the LFA-1 antagonist.In another embodiment the administration of the LFA-1 antagonist and another therapeutic substance continues for less than about 7 days. In yet another embodiment the co-administration continues for more than about 6, 10, 14, 28 days, two months, six months, or one year. In some cases, co-administered dosing is maintained as long as necessary, e.g., dosing for chronic inflammation.

Administration of the compositions of the invention may continue as long as necessary. In some embodiments, a composition of the invention is administered for more than 1, 2, 3, 4, 5, 6, 7, 14, or 28 days. In some embodiments, a composition of the invention is administered for less than 28, 14, 7, 6, 5, 4, 3, 2, or 1 day. In some embodiments, a composition of the invention is administered chronically on an ongoing basis, e.g., for the treatment of chronic pain.

Dosing for the LFA-1 antagonist in the method of the disclosure may be found by routine experimentation. The daily dose can range from about 1x 10⁻⁷g to 5000mg. Daily dose range may depend on the form of LFA-1 antagonist e.g., the esters or salts used, and/or route of administration, as described herein. For example, for systemic administration, , typical daily dose ranges are, e.g. about 1-5000 mg, or about 1-3000 mg, or about 1-2000 mg, or about 1-1000 mg, or about 1-500 mg, or about 1-100 mg, or about 10-5000 mg, or about 10-3000 mg, or about 10-2000 mg, or about 10-1000 mg, or about 10-500 mg, or about 10-200 mg, or about 10-100 mg, or about 20-2000 mg or about 20-1500 mg or about 20-1000 mg or about 20-500 mg, or about 20-100 mg, or about 50-5000 mg, or about 50-4000 mg, or about 50-3000 mg, or about 50-2000 mg, or about 50-1000 mg, or about 50-500 mg, or about 50-100 mg, about 100-5000 mg, or about 100-4000 mg, or about 100-3000 mg, or about 100-2000 mg, or about 100-1000 mg, or about 100-500 mg. In some embodiments, the daily dose of LFA-1 antagonist is about 100, 200, 300, 400, 500, 600, 700, 800,900, or 1000 mg. In some embodiments, the daily dose of the LFA-1 antagonist is 0.1 mg. In some embodiments, the daily dose of the LFA-1 antagonist is 1.0 mg. In some embodiments, the daily dose of the LFA-1 antagonist is 10 mg. In some embodiments, the daily dose of the LFA-1 antagonist is 100 mg. In some embodiments, the daily dose of LFA-1 antagonist is 500 mg. In some embodiments, the daily dose of LFA-1 antagonist is 1000 mg.

The typical daily dose ranges are, e.g. about 1x10⁻⁷g to 5.0g, or about 1x10⁻⁷g to 2.5g, or about 1x10⁻⁷g to 1.00g, or about 1x10⁻⁷g to 0.5g, or about 1x10⁻⁷g to 0.25g, or about 1x10⁻⁷g to 0.1g, or about 1x10⁻⁷g to 0.05g, or about 1x10⁻⁷g to 0.025g, or about 1x10⁻⁷g to 1x 10⁻²g, or about 1x10⁻⁷g to 5x 10⁻³g, or about 1x10⁻⁷g to 2.5x 10⁻³g, or about 1x10⁻⁷g to 1x 10⁻³g, or about 1x10⁻⁷g to 5x 10⁻⁴g, or about 1x10⁻⁶g to 5.0g, or about 1x10⁻⁶g to 2.5g, or about 1x10⁻⁶g to 1g, or about 1x 10⁻⁶g to 0.5g, or about 1x10⁻⁶g to 0.25g, or about 1x10⁻⁶g to 0.1g, or about 1x10⁻⁶g to 5x10⁻²g, or about 1x10⁻⁶g to 5x10⁻²g, or about 1x10⁻⁶g to 2.5x10⁻²g, or about 1x10⁻⁶g to 1x10⁻²g, or about 1x10⁻⁶g to 5x10⁻³g, or about 1x10⁻⁶g to 2.5x10⁻³g, or about 1x10⁻⁶g to 1x10⁻³g, or about 1x10⁻⁶g to 5x10⁻⁴g, or about 1x10⁻⁵g to 5g, or about 1x10⁻⁵g to 2.5g, or about 1x10⁻⁵g to 1g, or about 1x10⁻⁵g to 0.5g, or about 1x10⁻⁵g to 0.25g, or about 1x10⁻⁵g to 0.1g, or about 1x10⁻⁵g to 0.05g, or about 1x10⁻⁵g to 2.5 x10⁻²g, or about 1x10⁻⁵g to 1 x10⁻²g, or about 1x10⁻⁵g to 5 x10⁻³g, or about 1x10⁻⁵g to 2.5 x10⁻³g, or about 1x10⁻⁵g to 1 x10^{- 3}g, or about 1x10⁻⁵g to 5 x10⁻⁴g. In some embodiments, the daily dose of LFA-1 antagonist is about 1x 10⁻⁷, 1x 10⁻⁶, 1x 10⁻⁵, 1x 10⁻⁴, 1x 10⁻³g, 1x 10⁻²g, 1x 10¹g,or 1g. In some embodiments, the daily dose of the LFA-1 antagonist is 1x 10⁻⁷g. In some embodiments, the daily dose of the LFA-1 antagonist is 1x 10⁻⁵g. In some embodiments, the daily dose of LFA-1 antagonist is 1x 10⁻³g. In some embodiments, the daily dose of LFA-1 antagonist is 1x 10⁻²g. In some embodiments the individual dose ranges from about 1x10⁻⁷g to 5.0g, or about 1x10⁻⁷g to 2.5g, or about 1x10⁻⁷g to 1.00g, or about 1x10⁻⁷g to 0.5g, or about 1x10⁻⁷g to 0.25g, or about 1x10⁻⁷g to 0.1g, or about 1x10⁻⁷g to 0.05g, or about 1x10⁻⁷g to 0.025g, or about 1x10⁻⁷g to 1x 10⁻²g, or about 1x10⁻⁷g to 5x 10⁻³g, or about 1x10⁻⁷g to 2.5x 10⁻³g, or about 1x10⁻⁷g to 1x 10⁻³g,
or about 1x10⁻⁷g to 5x 10⁻⁴g, or about 1x10⁻⁶g to 5.0g, or about 1x10⁻⁶g to 2.5g, or about 1x10⁻⁶g to 1g, or about 1x10⁻⁶g to 0.5g, or about 1x10⁻⁶g to 0.25g, or about 1x10⁻⁶g to 0.1g, or about 1x10⁻⁶g to 5x10⁻²g, or about 1x10⁻⁶g to 5x10⁻²g, or about 1x10⁻⁶g to 2.5x10⁻²g, or about 1x10⁻⁶g to 1x10⁻²g, or about 1x10⁻⁶g to 5x10⁻³g, or about 1x10⁻⁶g to 2.5x10⁻³g, or about 1x10⁻⁶g to 1x10⁻³g, or about 1x10⁻⁶g to 5x10⁻⁴g, or about 1x10⁻⁵g to 5g, or about 1x10⁻⁵g to 2.5g, or about 1x10⁻⁵g to 1g, or about 1x10⁻⁵g to 0.5g, or about 1x10⁻⁵g to 0.25g, or about 1x10⁻⁵g to 0.1g, or about 1x10⁻⁵g to 0.05g, or about 1x10⁻⁵g to 2.5 x10⁻²g, or about 1x10⁻⁵g to 1 x10⁻²g, or about 1x10⁻⁵g to 5 x10⁻³g, or about 1x10⁻⁵g to 2.5 x10⁻³g, or about 1x10⁻⁵g to 1 x10⁻³g, or about 1x10⁻⁵g to 5 x10⁻⁴g. In some embodiments, the individual doses as described above, is repeated 1, 2, 3, 4, 5, 6,7, 8, 9, or 10 times per day.

The compositions of the invention may be packaged in multidose form. Preservatives may be preferred to prevent microbial contamination during use. The composition of the invention can be formulated as a sterile unit dose type containing no preservatives. Alternatively, preservatives may be used.

Suitable preservatives for the compositions of the invention include: benzalkonium chloride, purite, peroxides, perborates, thimerosal, chlorobutanol, methyl paraben, propyl paraben, phenylethyl alcohol, edetate disodium, sorbic acid, Onamer M, or other agents known to those skilled in the art. In some embodiments of the invention, such preservatives may be employed at a level of from 0.004% to 0.02%W/V. In some compositions of the present application the preservative, for example, benzalkonium chloride, methyl paraben, and/or propyl paraben,, may be employed at a level of from about 0.001% to less than about 0.01%, e.g. from about 0.001% to about 0.008%, or about 0.005% W/V. It has been found that a concentration of benzalkonium chloride of about 0.005% may be sufficient to preserve the compositions of the present invention from microbial attack. One of skill in the art could determine the proper concentration of ingredients as well as combinations of various ingredients for generating a suitable topical formulation. For example, ophthalmic drops or formulations for application to skin may use a mixture of methyl and propyl parabens at about 0.02%W/V and about 0.04%W/V respectively. In some embodiments, these formulations use methyl paraben and/or propyl paraben in amounts up to about 0.02% W/V and up to about 0.04% W/V respectively, which encompasses the embodiments where no methyl paraben or no propyl paraben is used.

The amount of administration and the number of administrations of the active ingredient used in the present invention vary according to sex, age and body weight of patient, symptoms to be treated, desirable therapeutic effects, administration routes and period of treatment. For delivery to the eye of an adult, the formulations containing the compounds of the disclosure may range in concentration from about 0.0001 to 10.0 W/V %, about 0.005 to 10.0 W/V %, about 0.01 to 10.0 W/V %, about 0.05 to 10.0 W/V %, about 0.1 to 10.0 W/V %, about 0.5 to 10.0 W/V %, about 1.0 to 10.0 W/V %, about 20 to 10.0 W/V %, about 3.0 to 10.0 W/V %, about 4.0 to 10.0 W/V %, or about 5.0 to 10.0 W/V %. One embodiment of the disclosure has a formulation of about 1.0 to 10.0 W/V % of the compounds of the disclosure. One embodiment of the disclosure has a formulation of about 0.01 to 10.0 W/V % of the compounds of the disclosure. One embodiment of the disclosure has a formulation of about 5.0 to 10.0 W/V % of the compounds of the disclosure. The administration may be administered several times a day per eye, one to ten times, one to four times, or once a day.

When used in the above compositions, a therapeutically effective amount of a medicament of the present disclosure may be employed in pure form or, where such forms exist, in pharmaceutically acceptable salt, ester or prodrug form. By a "therapeutically effective amount" of a medicament is meant a sufficient amount of the compound to obtain the intended therapeutic benefit, at a reasonable benefit/risk ratio applicable to any medical treatment. Local administration of LFA-1 antagonists rapidly cleared from the systemic circulation may be particularly beneficial in this regard where the local to systemic exposure ratio may be 10 to 10,000 fold or more. In dogs and rats, systemic bioavailability of Compound 12 from 1% ophthalmic drops has been measured at 6-30%, yet drug levels in tear are >1000x the level in plasma. It will be understood, however, that the total daily usage of the medicaments and compositions of the present invention will be decided by the attending physician within the scope of sound medical judgment. The specific therapeutically effective dose level for any particular patient and medicament will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific medicament employed; the duration of the treatment; drugs used in combination or coincidental with the specific compound employed; and like factors well known in the medical arts. For example, it is well within the skill of the art to start doses at levels lower than required to achieve the desired therapeutic effect and to gradually increase the dosage until the desired effect is achieved.

### EXAMPLES

In the following examples, compounds 5, 7, 9, 13, 15, 17, 19, 20, 23, 25, 27, 28, 30, 31, 34, 35, 38, 41, 44, and 48 relate to the invention. The remaining compounds are provided as reference examples.

### Example 1: Human T-Cell Adhesion Assay

The T-cell adhesion assay was performed using the human T-lymphoid cell line HuT 78 (ATCC TIB-161). Goat anti-HuIgG(Fc) was diluted to 2 µg/ml in PBS and 96-well plates were coated with 50 µl/well at 37°C for 1 h. Plates were washed with PBS and blocked for 1 h at room temperature with 1% BSA in PBS. 5 domain ICAM-Ig was diluted to 100 ng/ml in PBS and 50 µl/well was added to the plates O/N at 4°C. HuT 78 cells were centrifuged at 100 g and the cell pellet was treated with 5 mM EDTA for ∼5' at 37°C in a 5% CO₂ incubator. Cells were washed in 0.14 M NaCl, 0.02 M Hepes, 0.2% glucose and 0.1 mM MnCl₂ (assay buffer) and centrifuged. The cells were resuspended in assay buffer to 3.0X10⁶ c/ml. Inhibitors were diluted in assay buffer to a 2X final concentration and pre-incubated with HuT78 cells for 30' at room temperature. 100 µl/well of cells and inhibitors were added to the plates and incubated at room temperature for 1 h. 100 µl/well PBS was added and the plates were sealed and centrifuged inverted at 100 g for 5'. Unattached cells were flicked out of the plate and excess PBS was blotted on a paper towel. 60 µl/well p-nitrophenyl n-acetyl-β-D-glucosaminide (0.257 g to 100 ml citrate buffer) was added to the plate and incubated for 1.5 h at 37°C. The enzyme reaction was stopped with 90 µl/well 50 mM glycine/5 mM EDTA and read on a platereader at 405 nM. HUT 78 cell adhesion to 5dICAM-Ig was measured using the p-nitrophenyl n-acetyl-β-D-glucosaminide method of Landegren, U. (1984). J. Immunol. Methods 57, 379-388. The results are shown in **Figure 1**.

### Example 2: LFA-1 :ICAM-1 Receptor Binding Assay Using Forward Format Assay

Competitive inhibition of the LFA-1 :ICAM-1 interaction is quantitated by adding known amounts of inhibitors.

Purified full length recombinant human LFA-1 protein is diluted to 2.5 µg/ml in 0.02 M Hepes, 0.15M NaCl, and 1 mM MnCl₂ and 96-well plates (50 µl/well) are coated overnight at 4°C. The plates are washed with wash buffer (0.05% Tween in PBS) and blocked for 1 h at room temperature with 1% BSA in 0.02M Hepes, 0.15 M NaCl, and 1 mM MnCl₂. Plates are washed. 50 µl/well inhibitors, appropriately diluted in assay buffer (0.5% BSA in 0.02M Hepes, 0.15M NaCl, and 1 mM MnCl₂), are added to a 2X final concentration and incubated for 1 h at room temperature. 50 µl/well of purified recombinant human 5 domain ICAM-Ig, diluted to 50 ng/ml in assay buffer, is added and incubated 2 h at room temperature. Plates are washed and bound ICAM-Ig is detected with Goat anti-HuIgG(Fc)-HRP for 1 h at room temperature. Plates are washed and developed with 100 µl/well TMB substrate for 10-30' at room temperature. Colorimetric development is stopped with 100 µl/well 1M H₂PO₄ and read at 450 nM on a platereader.

### Example 3: In-Vitro Inhibition of Antigen Stimulated Release of Cytokines From Human Peripheral Blood Monocytes (PBMC)

One form of the LFA-1 antagonist of Formula I, Compound 12, was evaluated for its ability to inhibit release of inflammatory cytokines, in human mononuclear cells (PBMC) stimulated with staphylococcal enterotoxin B (SEB). Stock solutions of Compound 12, Rebamipide (a mucosal protective agent), and Cyclosporin A (CsA) were prepared in culture media and dilutions were prepared by addition of culture media to achieve the desired concentration. Negative controls were prepared without SEB stimulation. SEB stimulation with vehicle (0.25% DMSO/media) was used as the positive control.

Human PBMC, frozen in cryopreservation media were thawed, washed with RPMI culture media containing 10% FBS in growth media and seeded onto a 96 well plate at 20,000 cells/well containing 180 µl culture media. Cells were incubated in the presence of Compound 12, Rebamipide or CsA at 37°C for 1 hour prior to stimulation with SEB. SEB was added at 1 ng/ml and cell supernatants were harvested at 6, 16, and 48 hours. Cytokine levels in the assay supernatants were determined using a Luminex multiplex assay.

Compound 12 demonstrated potent inhibition of the release of inflammatory cytokines, particularly the T-cell regulating cytokines, IL-2 and IL-4, with increasing dose. The results are shown in Tables 1, 2, and 3. Additionally, in vitro inhibition of IL-2 release for various LFA-1 antagonists is shown in **Figure 1****.** The pattern of cytokine release inhibited by more than 50% with Compound 12 is similar to that seen in comparison with CsA. The exceptions to this similarity include IL-3, 11-6, and IL-12p40.

**Table 1. EC50 Concentrations for Inhibition of IL-2, IFNγ, MIP-1α, and TNF-α.**

| | EC50 µM Cytokine Release | | | |
|---|---|---|---|---|
| | IL-2 | IFNγ | MIP-1α | TNF-α |
| Compound 12 | 0.0018 | 0.0016 | 0.020 | 0.076 |
| Rebamipide | >1000 | >1000 | >1000 | >1000 |
| Cyclosporine A | 0.00094 | 0.00050 | 0.0011 | 0.00049 |

**Table 2. EC50 Concentrations for Inhibition of IL-4, IL-10, IP-10 GM-CSF and MCP-1.**

| | EC50 µM Cytokine Release | | | | |
|---|---|---|---|---|---|
| | IL-4 | IL-10 | IP-10 | GM-CSF | MCP-1 |
| Compound 12 | 0.143 | 0.147 | 1.158 | 0.545 | 0.0050 |
| Rebamipide | >1000 | >1000 | >1000 | >1000 | >1000 |
| Cyclosporine A | 0.0063 | 0.0292 | 0.167 | 0.0202 | 0.0926 |

**Table 3. EC50 Concentrations for Inhibition of IL-1α, IL-1β, IL-3, IL-5, IL-6, IL-12p40, and IL-13.**

| | EC50 µM Cytokine Release | | | | | | |
|---|---|---|---|---|---|---|---|
| | IL-1 α | IL-1β | IL-3 | IL-5 | IL-6 | IL-12p40 | IL-13 |
| Compound 12 | 0.24 | 0.36 | 52.23 | 0.11 | 43.51 | >1000 | 0.36 |
| Rebamipide | >1000 | >1000 | >1000 | >1000 | >1000 | >1000 | >1000 |
| Cyclosporine A | 0.002 | 0.003 | 0.002 | 0.073 | 0.001 | 0.002 | 0.074 |

### Example 4: Formulations of LFA-1 Antagonist

One compound of Formula I (Compound 12) was formulated in several compositions for administration as gels, lotions, ointments, and solutions, for administration by varying routes, including but not limited to topical, via instillation, aerosol, transdermal patch, via insert, or oral administration.

**Table 4. Gel Formulations 1 and 2 of Compound 12.**

| **Formulation 1 (% w/w)** | **Formulation 2 (% w/w)** |
|---|---|
| 1% Form A of Compound 12 | 1% Form A of Compound 12 |
| 15% Dimethyl Isosorbide | 15% Dimethyl Isosorbide |
| 25% Transcutol | 25% Transcutol |
| 12% Hexylene glycol | 12% Hexylene glycol |
| 5% Propylene Glycol | 5% Propylene Glycol |
| 0.15% Methylparaben | 0.15% Methylparaben |
| 0.05% Propylparaben | 0.05% Propylparaben |
| 0.01% EDTA | 0.01% EDTA |
| 0.5% Penmulen TR-1 | 1% Hydroxyethyl Cellulose |
| q.s. pH 6.0 25% Trolamine | q.s. pH 4.5 25% Trolamine |
| q.s. 100 Water | q.s. 100 Water |

**Table 5. Lotion Formulations 3 and 4 of Compound 12.**

| **Formulation 3 (% w/w)** | **Formulation 4 (% w/w)** |
|---|---|
| 1% Form A | 1% Form A |
| 13% Dimethyl Isosorbide | 13% Dimethyl Isosorbide |
| 20% Transcutol | 20% Transcutol |
| 10% Hexylene glycol | 10% Hexylene glycol |
| 4% Propylene Glycol | 4% Propylene Glycol |
| 0.15% Methylparaben | 0.15% Methylparaben |
| 0.05% Propylparaben | 0.05% Propylparaben |
| 0.01% EDTA | 0.01% EDTA |
| 0.5% Carbopol Ultrez 10 | 0.3% Carbopol Ultrez 10 |
| 0.2% Penmulen TR-1 | 0.2% Penmulen TR-1 |
| 3% Isopropyl Myristate | 2% Cetyl Alcohol |
| 5% Olelyl Alcohol | 5.5% Light Mineral Oil |
| 5% White Petrolatum | 5% Oleic Acid |
| 0.02% Butylated Hydroxytoluene | 0.02% Butylated Hydroxytoluene |
| q.s. pH 6.0 25% Trolamine | q.s. pH 6.0 25% Trolamine |
| q.s. 100 Water | q.s. 100 Water |

**Table 6. Ointment Formulations 5 and 6 of Compound 12.**

| **Formulation 5 (% w/w)** | **Formulation 6 (% w/w)** |
|---|---|
| 1% Form A | 1% Form A |
| 15% PEG 400 | 10% Dimethyl Isosorbide |
| 0.02% Butylated Hydroxytoluene | 0.02% Butylated Hydroxytoluene |
| 2% Span 80 | 2% Span 80 |
| 10% White Wax | 10% White Wax |
| 71.98% White Petrolatum | 76.98% White Petrolatum |

**Table 7. Solution Formulations 7, 8, and 9 of Compound 12.**

| **Formulation 7 (% w/w)** | **Formulation 8 (% w/w)** | **Formulation 9 (% w/w)** |
|---|---|---|
| 1% Form A | 1% Form A | 1% Form A |
| 15% Dimethyl Isosorbide | 15% Dimethyl Isosorbide | 99% Dimethyl Sulfoxide |
| 25% Transcutol | 25% Transcutol | |
| 12% Hexylene glycol | 12% Hexylene glycol | |
| 5% Propylene Glycol | 5% Propylene Glycol | |
| q.s. pH 4.5 25% Trolamine | q.s. pH 6.0 25% Trolamine | |
| q.s. 100 Water | q.s. 100 Water | |

**Table 8. Solution Formulations 10, 11, 12, 13 and 14 of Compound 12.**

| **W/W%** | **Formulation 10** | **Formulation 11** | **Formulation 12** | **Formulation 13** | **Formulation 14** |
|---|---|---|---|---|---|
| Form A | 0.1% | 0.3% | 1% | 3% | 5% |
| Sodium Bicarbonate | 0.015% | 0.046% | 0.15% | 0.46% | 0.77% |
| | 0.1% EDTA | | | | |
| | 0.12% Sodium Phosphate, Monobasic | | | | |
| | 0.4% Methylparaben | | | | |
| | 0.02% Propylparaben | | | | |
| | q.s. Osmolality 270, Sodium Chloride | | | | |
| | q.s. pH 7.01% Sodium Hydroxide | | | | |
| | q.s. pH 7.0 1% HCl | | | | |
| | q.s. Water | | | | |

**Table 9. Solution Formulation 15 of Compound 12.**

| **Formulation 15** |
|---|
| 1 ml of a solution of Compound 12 10%W/W in water, plus 0.158mmol sodium bicarbonate |
| 9 ml PBS |

Compound 12 can be supplied as a sterile, clear, colorless liquid solution containing 0.1%, 1.0%, and 5.0% (w/w) Active Pharmaceutical Ingredient (API) concentrations (pH 7.0). Each mL of a 1% solution contains 10 mg of the active ingredient. In addition to Compound 12, other components of a drug product solution, their functions, and their compendial grade can include propylparaben (preservative; National Formulary (NF)), methylparaben (preservative, NF), EDTA (antioxidant, United States Pharmacopeia (USP)), sodium bicarbonate (buffering agent, USP), monobasic sodium phosphate (buffering agent, USP), dibasic sodium phosphate (buffering agent, USP), and sterile water (diluent, USP). All excipients can be of compendial grade and of non-human or non-animal origin.

Formulated drug product solution can be packaged under aseptic conditions into sterile 7.0 mL High Density Polyethylene (HDPE) bottles equipped with a dropper tip that delivers an approximate per drop volume of 0.35 µL and a protective cap. The dropper bottle can have a 40 µL tip. Unpreserved study drug (no methyl or propylparabens in the formulation) can be provided in 0.5 mL unit dose Low Density Polyethylene (LDPE) containers manufactured using a blow fill seal process and stored in aluminum foil pouches.

Drug solutions can be stored refrigerated (2-8°C). The stability of the drug at 5°C and 25°C can be out to 9 months or longer.

### Example 5: In-Vitro Percutaneous Absorption of the Compound of Formula I Following Topical Application.

Bioavailability following topical application in-vivo was assessed using in-vito percutaneous absorption test methods, using procedures adapted from Skelly et al., Pharmaceutical Research 1987 4(3): 265-276, "FDA and AAPS Report of the Workshop on Principles and Practices of In-Vitro Percutaneous Penetration Studies: Relevance to Bioavailability and Bioequivalence".

Formulations 1-9 were applied to dermatomed human skin tissue excised from a single donor in a single clinically relevant dose of 5mg/cm², which is equivalent to a 30-35µg dose. The thickness of the tissue ranges form 0.023 to 0.039 inches (0.584 to 0.991 mm) with a mean +/- standard deviation in thickness of 0.030 +/- 0.004 inches (0.773 +/- 0.111mm) and a coefficient of variation of 14.4%. The tissue samples were mounted in Bronaugh flow-through diffusion cells. The cells were maintained at a constant temperature of 32°C using recirculating water baths. The cells have a nominal diffusion area of 0.64 cm². PBS, at pH 7.4, with 0.1% sodium azide and 4% Bovine Serum Albumin was used as the receptor phase below the mounted tissue. Fresh receptor phase was continuously pumped under the tissue at a flow rate of nominally 1.0 ml/hr and collected in 6 hour intervals. The receptor phases were collected for analysis.

The tissue samples were exposed to Formulations 1-9 for 24 hours. The excess formulation residing on the strateum corneum at that timepoint was removed by tape-stripping with CuDerm D-Squame stripping discs. The tape strips were discarded. The epidermis and dermis were separated by blunt dissection. Epidermis, dermis and receptor phase were analyzed for content of Compound 12. The results are represented in Table 10.

Tissue permeation levels (the receptor phase) of Compound 12 for all formulations except for Formulation 9, which contained 99% DMSO, were below the limits of quantitation, which was 0.54ng/ml (which is equivalent to 0.013% of the applied dose). Formulation 9, in contrast, provided 1.4% of the applied dose, permeating through all the layers of the skin tissue over the exposure period of 24 hours.

Epidermal deposition of Compound 12 over the 24 hour exposure period was very high and consistent with a large percentage of the applied dose being retained in the upper layers of the epidermis. The levels reported in Table 10 were obtained from small volume samples, which could not be re-assayed, and thus are considered underestimates of the amount of drug present in the epidermis.

Analytical data for the dermis fell within the linearity range established for Compound 12, and are quantitative. Dermal deposition of Compound 12 following a 24 hour exposure ranged from 0.66% (Formulation 6, 0.258 µg/cm²) to 4.4% (Formulation 7, 2.08 µg/cm²) of the applied dose. The concentration of Compound 12 (633.5 g/mole) in the dermis is thereby calculated as 6.7µM (Formulation 6) or greater (i.e., Formulation 7 provides a concentration in the dermis of 54.1 µM) for Formulations 1 to 9 in the dermis. These concentrations are well above the in-vitro EC50 concentration for half maximal effect in inhibiting release of inflammatory cytokines by Compound 12, as shown in Example 3. These results are therefore predictive for the ability of a variety of formulations, which incorporate 1% W/W Compound 12, to provide therapeutically effective levels of in-vivo inhibition of cytokine release.

**Table 10. Cumulative Receptor Phase and Tissue Levels of Compound 12 After 24 Hours of Topical Exposure.**

| Formulation # | | Receptor Phase Content at 24 hours | | Epidermis | | Dermis | | |
|---|---|---|---|---|---|---|---|---|
| | | µg/cm² | % Dose Applied | µg/cm² | % Dose Applied | µg/cm² | µg/ml | % Dose Applied |
| 1 | Mean | <Limit of Quantitation | | 3.93 | 7.48 | 1.14 | 18.8 | 2.15 |
| | SD¹ | | | 2.92 | 5.50 | 0.91 | 14.9 | 1.73 |
| | %CV² | | | 74 | 74 | 80 | 80 | 80 |
| 2 | Mean | <Limit of Quantitation | | 6.03 | 11.9 | 0.750 | 12.3 | 1.49 |
| | SD | | | 2.56 | 5.1 | 0.304 | 5.0 | 0.63 |
| | %CV | | | 43 | 42 | 40 | 40 | 42 |
| 3 | Mean | <Limit of Quantitation | | 6.03 | 12.1 | 1.40 | 23.0 | 2.74 |
| | SD | | | 2.97 | 6.4 | 0.27 | 4.4 | 0.47 |
| | %CV | | | 49 | 53 | 19 | 19 | 17 |
| 4 | Mean | <Limit of Quantitation | | 7.92 | 17.0 | 0.975 | 16.0 | 2.10 |
| | SD | | | 3.41 | 7.2 | 0.350 | 5.8 | 0.75 |
| | %CV | | | 43 | 42 | 36 | 36 | 36 |
| 5 | Mean | <Limit of Quantitation | | 5.71 | 14.6 | 0.670 | 11.0 | 1.71 |
| | SD | | | 1.73 | 4.2 | 0.351 | 5.8 | 0.87 |
| | %CV | | | 30 | 29 | 52 | 52 | 51 |
| 6 | Mean | <Limit of Quantitation | | 6.47 | 16.8 | 0.258 | 4.25 | 0.657 |
| | SD | | | 1.07 | 2.7 | 0.158 | 2.6 | 0.394 |
| | %CV | | | 17 | 16 | 61 | 61 | 60 |
| 7 | Mean | <Limit of Quantitation | | 7.22 | 15.0 | 2.08 | 34.3 | 4.35 |
| | SD | | | 2.15 | 4.5 | 0.84 | 13.7 | 1.83 |
| | %CV | | | 30 | 30 | 40 | 40 | 42 |
| 8 | Mean | <Limit of Quantitation | | 8.58 | 18.0 | 1.48 | 24.3 | 3.09 |
| | SD | | | 3.53 | 7.7 | 0.99 | 16.2 | 2.07 |
| | %CV | | | 41 | 43 | 67 | 67 | 67 |
| 9 | Mean | 0.660 | 1.43 | 5.78 | 13.2 | 1.19 | 19.6 | 2.63 |
| | SD | 0.253 | 0.49 | 3.18 | 8.3 | 0.49 | 8.1 | 1.15 |
| | %CV | 38 | 34 | 55 | 63 | 41 | 41 | 44 |

| | | | | | | | | |
|---|---|---|---|---|---|---|---|---|
| 1. Standard Deviation. 2. Percent Coefficient of Variation. | | | | | | | | |

### Example 6: Pharmacological Activity of Compound 12 for Treatment of Keratoconjunctivitis Sicca (KCS) (Reference Example)

Dogs were enrolled in this study if the following criteria were met: more than one year of age, a Schimer tear test (STT) of less than 10 mm wetting per minute, bilateral involvement, and at least one of the following clinical signs: blepharospasm, conjunctivial hyperemia, exposure keratopathy (irregular surface), corneal pigmentation, corneal neovascularization or ropey mucopurulent discharge, no congenital KCS, no traumatic KCS, toxic KCS, and no facial nerve paralysis. If dogs had been treated with topical CsA or tacrolimus in the previous six months, they were not enrolled.

The dogs were administered one 35µl drop of Compound 12,1% solution (Formulation 15, 0.35mg /eye), in each affected eye three times daily, with approximately 4 hours (± 1 hour) between the daily doses for 12 weeks. CsA will be administered for a further four weeks by administering commercially available 0.2% ointment three times a day, after the Compound 12 is discontinued at twelve weeks.

Animals were subjected to an ocular examination once during the initial visit and during five visits over sixteen weeks of the study (Weeks, 2, 4, 8, 12 and 16). The last OE was approximately four weeks after the last dose of Compound 12 and after one month of CsA treatment. The adnexa and anterior portions of both eyes were examined using an indirect opthalmoscope. The eyes were dilated with a mydriatic when applicable, to allow evaluation of the lens and fundus, including the retina. An evaluation using a modified McDonald-Shaddock scoring system was performed in conjunction with the slitlamp ocular examinations at each interval.

Tears were measured using STT strips during the initial visit and each of the five follow-up visits on Weeks 2, 4, 8, 12 and 16. One strip of STT paper was used for each eye for each interval. At each collection interval, the STT paper was folded and placed in the inferior cul de sac for sixty seconds. The length, in mm, of wetting below the notch of the paper was recorded.

Fluorescein and rose bengal staining was performed at the each of the initial and follow up examinations. Intraocular pressure measurements (IOPs) were performed using a Tono-Pet Vet® in conjunction with each of the OEs. Digital ocular images were taken before and after staining (with fluorescein and rose bengal) during each of the OEs.

Conjunctival biopsies were taken at the initial (pretreatment) visit and the Week 12 visit. The second biopsy was taken more lateral (approx. 1 mm) to the initial biopsy. Following appropriate preparation a small conjunctival biopsy was taken from the ventral fornix of each eye.

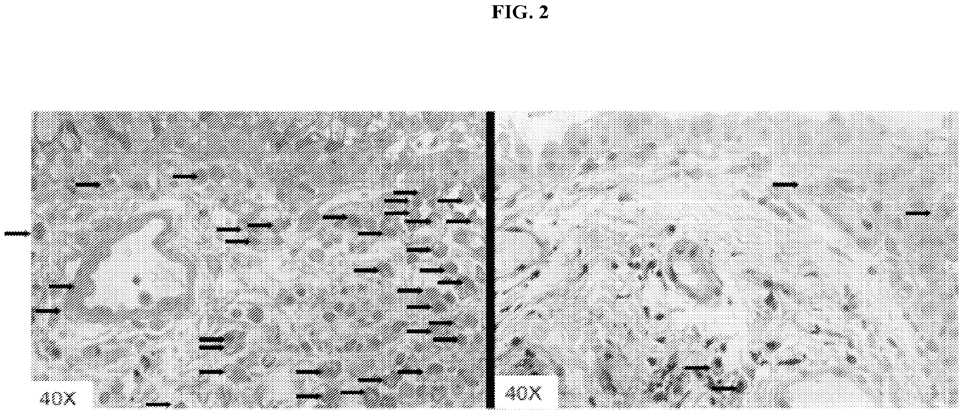

Seven dogs completed the study; for two dogs, only one eye was studied. The results are shown in Tables 11 and 12. Overall, a 3.3 mm average improvement in OD (right eye) STT and 4.5 mm in OS (left eye) STT was observed during the treatment period with Compound 12. Results for all 12 eyes show an average of 4 mm improvement. A Maximum-Minimum analysis was performed using the maximal change in STT values for each eye in each dog over weeks 1-12, as shown in Table 13. This calculation yields a total maximal change in STT for total of eyes of 72 mm, which upon division by 12 (number of KCS eyes in the analysis), yields a 6.0 mm average improvement. Other clinical signs improved in some dogs, such as a decrease in mucopurulent discharge or conjunctival erythema. Histopathological evaluation of biopsies taken before and after Compound 12 revealed an attenuation of lymphocyte accumulation. **Figure 2** illustrates this phenomenon in samples taken from dog #1. No significant additional benefit was seen from four subsequent weeks of CsA administration.

**Table 11. Schirmer Tear Test Results (OD).**

| Dog ID | Week 1 | Week 2 | Week 4 | Week 8 | Week 12 | Week 16 |
|---|---|---|---|---|---|---|
| 1 | 15 | 18 | 12 | 16 | 13 | 12 |
| 2 | 0 | 2 | 0 | 8 | 8 | 8 |
| 3 | 6 | 11 | 5 | 7 | 7 | 8 |
| 4 | 5 | 11 | 10 | 7 | 13 | 8 |
| 5 | 8 | 11 | 10 | 11 | 9 | 22** |
| 6 | 8 | 10 | 15 | 17 | 16 | 18 |
| 7 | 6 | 2 | 2 | 1 | 0 | 12 |
| Mean * | 5.5 | 7.8 | 7.0 | 8.5 | 8.8 | 11.7 |

| | | | | | | |
|---|---|---|---|---|---|---|
| *Dog #1 not included in mean or Maximum- Minimum analysis for OD as there is no KCS in that eye for that animal. ** Data for Dog #5 is anomalous for this day, and is not included in the mean or Maximum- Minimum analysis. | | | | | | |

**Table 12. Schirmer Tear Test Results (OS).**

| Dog ID | Week 1 | Week 2 | Week 4 | Week 8 | Week 12 | Week 16 |
|---|---|---|---|---|---|---|
| 1 | 0 | 0 | 0 | 0 | 3 | 3 |
| 2 | 0 | 0 | 0 | 2 | 7 | 5 |
| 3 | 9 | 14 | 7 | 17 | 15 | 16 |
| 4 | 0 | 3 | 5 | 6 | 4 | 7 |
| 5 | 7 | 8 | 14 | 8 | 8 | 19 |
| 6 | 9 | 4 | 14 | 8 | 8 | 17 |
| 7 | 18 | NA | NA | 19 | 18 | 18 |
| Mean * | 4.2 | 4.8 | 6.7 | 6.5 | 8.7 | 11.0 |

| | | | | | | |
|---|---|---|---|---|---|---|
| *Dog #7 not included in mean or Maximum- Minimum analysis for OS as there is no KCS in that eye for that animal. | | | | | | |

**Table 13. Maximum- Minimum Analysis for Weeks 1-12 of Compound 12 Administration.**

| | | |
|---|---|---|
| OD | OS | Total OD plus Total OS: 72 |
| NA | 3 | |
| 8 | 7 | |
| 5 | 10 | Grand Total/Number of Eligible Eyes: 6.0 mm Average Improvement |
| 8 | 6 | |
| 3 | 7 | |
| 8 | 11 | |
| -4 | NA | |
| Total= 28 | Total = 44 | |

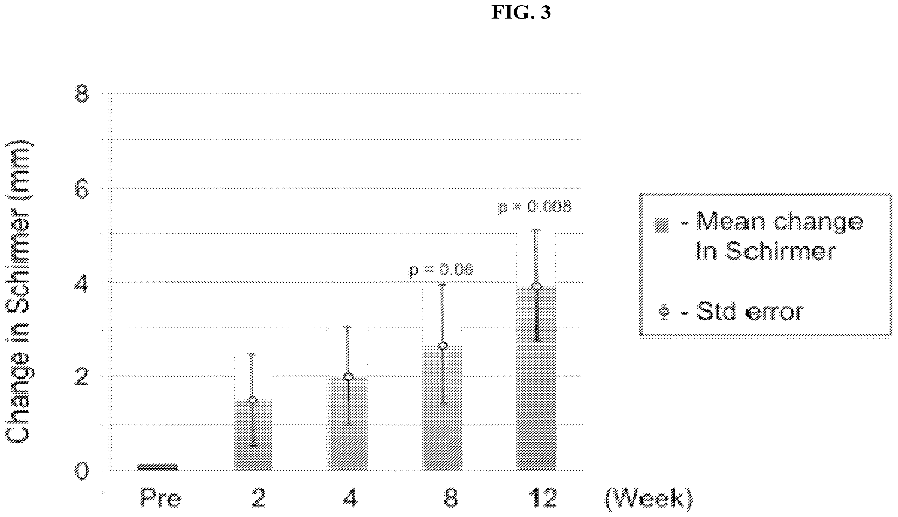

**Figure 3** illustrates the mean change in Schirmer test score at weeks 2, 4, 8, and 12. Significant improvement in Schirmer test scores over pretreatment was observed in week 12.

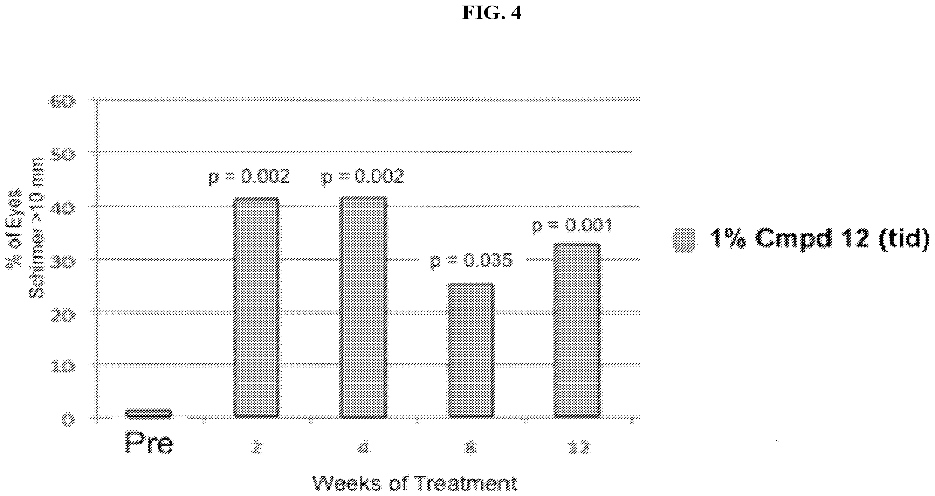

**Figure 4** illustrates the percentage of eyes with a Schirmer test score of greater than 10 mm at 2, 4, 8, and 12-weeks with 1% Compound 12 (TID). Compound 12 canine KCS study results exceeded human CsA data. The basis of restasis approval was an improvement of Schirmer test score to greater than 10 m. Restasis treatment resulted in 15% of eyes with Schirmer test score greater than 10 mm.

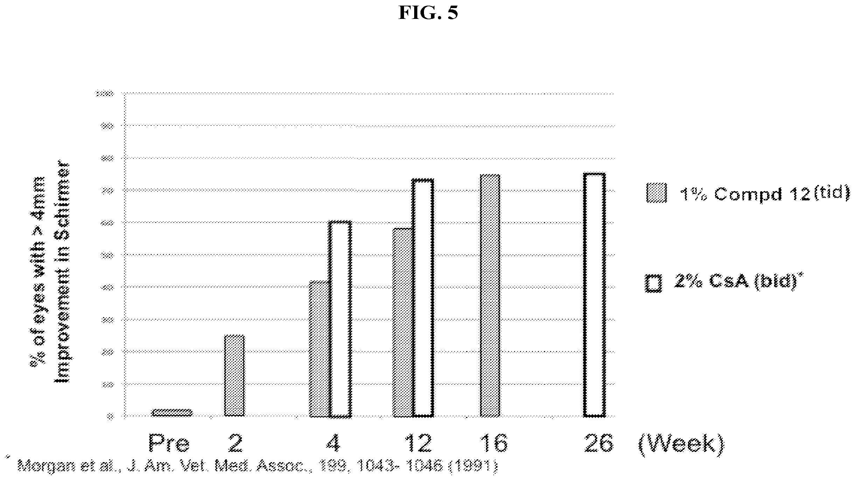

**Figure 5** illustrates the percentage of eyes with a greater than 4 mm improvement in Schirmer test score at 2, 4, 12, 16, and 26 weeks for subjects treated with 1% Compound 12 (tid) or 2% CsA (bid) (using historic CsA data; Morgan et al., J. Am. Vet. Med. Assoc., 199, 1043-1046 (1991)). Compound 12 timecourse was simlar to historic CsA data.

In summary, the Canine KCS study demonstrated that administering Compound 12 resulted in rapid improvement in Schirmer test score in 2-8 weeks, improvement in histology, and rapid anti-inflammatory effect.

### Example 7: T-Cell Proliferation Assay:

This assay is an in vitro model of lymphocyte proliferation resulting from activation, induced by engagement of the T-cell receptor and LFA-1, upon interaction with antigen presenting cells (Springer, Nature 346: 425 (1990)).

Microtiter plates (Nunc 96 well ELISA certified) are pre-coated overnight at 4°C. with 50 µl of 2 µg/ml of goat anti-human Fc(Caltag H10700) and 50 µl of 0.07 µg/ml monoclonal antibody to CD3 (Immunotech 0178) in sterile PBS. The next day coat solutions are aspirated. Plates are then washed twice with PBS and 100 µl of 17 ng/ml 5d-ICAM-1-IgG is added for 4 hours at 37°C. Plates are washed twice with PBS prior to addition of CD4+ T cells. Lymphocytes from peripheral blood are separated from heparinized whole blood drawn from healthy donors. An alternative method is to obtain whole blood from healthy donors through leukophoresis. Blood is diluted 1:1 with saline, layered and centrifuged at 2500Xg for 30 minutes on LSM (6.2 g Ficoll and 9.4 g sodium diztrizoate per 100 ml) (Organon Technica, N.J.). Monocytes are depleted using a myeloid cell depletion reagent method (Myeloclear, Cedarlane Labs, Hornby, Ontario, Canada). PBLs are resuspended in 90% heat-inactivated Fetal Bovine serum and 10% DMSO, aliquoted, and stored in liquid nitrogen. After thawing, cells are resuspended in RPMI 1640 medium (Gibco, Grand Island, N.Y.) supplemented with 10% heat-inactivated Fetal Bovine serum (Intergen, Purchase, N.Y.), 1 mM sodium pyruvate, 3 mM L-glutamine, 1 mM nonessential amino acids, 500 µg/ml penicillin, 50 µg/ml streptomycin, 50 µg/ml gentamycin (Gibco).

Purification of CD4+ T cells are obtained by negative selection method (Human CD4 Cell Recovery Column Kit # CL110-5 Accurate). 100,000 purified CD4+ T cells (90% purity) per microtiter plate well are cultured for 72 hours at 37°C. in 5% CO₂ in 100 ml of culture medium (RPMI 1640 (Gibco) supplemented with 10% heat inactivated FBS (Intergen), 0.1 mM non-essential amino acids, 1 nM Sodium Pyruvate, 100 units/ml Penicillin, 100 µg/ml Streptomycin, 50 µg/ml Gentamicin, 10 mM Hepes and 2 mM Glutamine). Inhibitors are added to the plate at the initiation of culture. Proliferative responses in these cultures are measured by addition of 1 µCi/well titrated thymidine during the last 6 hours before harvesting of cells. Incorporation of radioactive label is measured by liquid scintillation counting (Packard 96 well harvester and counter). Results are expressed in counts per minute (cpm).

### Example 8: In vitro Mixed Lymphocyte Culture Model

The mixed lymphocyte culture model, which is an in vitro model of transplantation (A. J. Cunningham, "Understanding Immunology, Transplantation Immunology" pages 157-159 (1978) examines the effects of various LFA-1 antagonists in both the proliferative and effector arms of the human mixed lymphocyte response.

Isolation of Cells: Mononuclear cells from peripheral blood (PBMC) are separated from heparanized whole blood drawn from healthy donors. Blood is diluted 1:1 with saline, layered, and centrifuged at 2500Xg for 30 minutes on LSM (6.2 g Ficoll and 9.4 g sodium diztrizoate per 100 ml) (Organon Technica, N.J.). An alternative method is to obtain whole blood from healthy donors through leukophoresis. PBMCs are separated as above, resuspended in 90% heat inactivated Fetal Bovine serum and 10% DMSO, aliquoted and stored in liquid nitrogen. After thawing, cells are resuspended in RPMI 1640 medium (Gibco, Grand Island, N.Y.) supplemented with 10% heat-inactivated Fetal Bovine serum (Intergen, Purchase, N.Y.), 1 mM sodium pyruvate, 3 mM L-glutamine, 1 mM nonessential amino acids, 500 µg/ml penicillin, 50 µg/ml streptomycin, 50 µg/ml gentamycin (Gibco).

Mixed Lymphocyte Response (MLR): One way human mixed lymphocyte cultures are established are in 96-well flat-bottomed microtiter plates. 1.5x10⁵ responder PBMCs are co-cultured with an equal number of allogeneic irradiated (3000 rads for 3 minutes, 52 seconds stimulator PBMSc in 200 µl of complete medium. LFA-1 antagonists are added at the initiation of cultures. Cultures are incubated at 37°C in 5% CO₂ for 6 days, then pulsed with 1 µCi/well of 3H-thymidine (6.7 Ci/mmol, NEN, Boston, Mass.) for 6 hours. Cultures are harvested on a Packard cell harvester (Packard, Canberra, Canada). [³H] TdR incorporation is measured by liquid scintillation counting. Results are expressed as counts per minute (cpm).

### Example 9: T-cell adhesion assay using Jurkat cells

The purpose of this study was to evaluate the anti-adhesive properties of Compound 12 on the attachment of Jurkat cells to ICAM-1 following *in vitro* exposure.

Stock solutions of Compound 12 and positive control were prepared in DMSO/water (1:1) and diluted into assay media and subsequent dilutions were prepared by addition of assay media to achieve the desired concentration. A reported LFA-1 antagonist was used as the positive control.

Jurkat cells were labeled with an 8 µM solution of BCECF-AM (2',7'-bis-(2-carboxyethyl)-5-(and-6)-carboxyfluorescein) in growth media at room temperature for 15 minutes. Labeled cells were incubated in 70 µL of assay media in each well of a 96 well plate at 500,000 cells per well with 70 µL of Compound 12 or positive control in assay media at 37°C for 30 minutes. A 100 µL aliquot of this fluorescently labeled Jurkat cell suspension was allowed to settle in the presence of Compound 12 or the positive control in wells of a 96 well plate coated with recombinant human ICAM-1 expressed as an Fc chimera at 37°C for 1 hour. Non-adherent cells were removed by washing and centrifugation at 100g for 1 minute. Adherent cells were determined as adherent fluorescent units on a fluorescent plate reader. The test article, Compound 12, demonstrated inhibition of Jurkat cell attachment with increasing dose. The dose response curve and IC₅₀ of Compound 12 in this assay was comparable to that of the known direct competitive LFA-1 antagonist. This demonstrates Compound 12 is an antagonist of LFA-1/ICAM-1 binding.

### Example 10: Murine Pseudomonis Corneal Keratitis (Reference Example)

The cornea is normally clear and leukocyte free. Bacterial infection induces complement mediated leukocyte recruitment and inflammation into the cornea. A murine model of neutrophil keratitis has been developed which inserts a defined number of tobramycin killed *Pseudomonas* into a surgical cut in the cornea. Neutrophil influx and corneal haze are scored at 24 hours. The system provides a pharmacodynamic model of neutrophil adhesion in vasculature and migration into tissue. The system has been described in Sun Y. and Pearlman E. (2009) Invest Ophthalmol Vis Sci. 50:1247-54.

### Example 11: Preclinical and Clinical Safety and Tolerability: pk and systemic and local distribution results (Reference Example)

### A. EFFECTS IN HUMANS

### 1. Phase 1 Clinical Trial Compound 12

A Phase 1 single center randomized, prospective, double masked, placebo controlled study of escalating doses of topical Compound 12 Ophthalmic Solution was conducted in 4 cohorts (0.1%, 0.3%, 1% and 5% Compound 12 dose strengths) in 28 healthy adults (7 subjects per cohort: 5 received Compound 12 Ophthalmic Solution and 2 received placebo solution). The objectives of the trial were to measure safety and tolerability, and pharmacokinetics in tear and plasma. The dosing schedule (OU; Oculus Uterque (each eye or both eyes)) was divided into 3 periods, each separated by a 72-hour wash out interval: once/day x 1 day (drug one eye; placebo fellow eye), twice/day x 10 days, and thrice/day x 10 days, 14-day observation. Slit lamp examination of the eye, BCVA (Best Corrected Visual Acuity), STTs (Schirmer Tear Test), TBUT (Tear Break-Up Time), IOP (Intraocular pressure) were assessed at screening and the beginning and end of each period. For each cohort, masked safety data was reviewed by a Safety Committee prior to allowing dose escalation of the next cohort. A total of 2856 doses (102 drops/subject) were administered over 1148 total subject study days (41 study days/subject) in 56 eyes. All subjects in all cohorts completed the study, and no study drug doses were missed.

No deaths, discontinuations, serious or severe ocular or non-ocular AEs (adverse effects) considered related to Compound 12 Ophthalmic Solution administration occurred at any dose strength or in any dose regimen.

Blood pressure, heart rate, respiratory rate, temperature, body weight, and EKG results were within normal ranges throughout the trial.

All hematologic results and all but one serum chemistry result were within normal ranges with no observable study drug-related trends measured across study duration, dose-strength, or schedule. Total lymphocyte count, CD3, CD4, and CD8 cell counts were within normal ranges with no evidence of lymphocyte or neutrophil suppression. Urinalysis results were unremarkable throughout the trial.

Serum chemistry results were within normal range with no observable study drug-related trends measured across study duration, dose-strength, or schedule.

No serious or severe ocular or non-ocular AEs occurred during the study; there were 38 ocular (N=11 subjects) and 21 non ocular (N=11 subjects) AEs, respectively. There were no trends in the frequency of ocular AEs when analyzed by dose group or by study period. No significant safety trends were noted on BCVA, slit-lamp biomicroscopy, STT, TBUT, or IOP assessments, nor was there evidence of ocular infection, or localized immunosuppression. There was no evidence of localized ocular irritation or infection.

There were no trends in the frequency of non-ocular AEs when analyzed by dose group or by study period. No significant safety trends were noted on vital signs, EKG, laboratory studies (chemistry, liver functions, blood panels); there was no evidence of CD3, CD4, or CD8 T-cell suppression, bone marrow suppression, or clinical evidence of increased infections.

### 2. Pharmacokinetics in tear and plasma

Plasma and tear samples were obtained at baseline and during scheduled intervals in each dosing period to characterize the pharmacokinetics (PK) of Compound 12 Ophthalmic Solution following ocular administration.

### a. Plasma PK Analysis

Samples for plasma Compound 12 analysis were obtained pre-dose, at 5 and 30 minutes post-dose, and at 1, 4, 8, 24 hours post-dose on Days 1, 5, 14, 18 and 27. Samples were also obtained at 48 hours post dose on Days 1, 14 and 27 and a single blood sample was collected at the follow-up visit at the end of the study. Plasma Compound 12 concentrations were determined using a validated LC/MS/MS (liquid chromatography tandem mass spectrometry) method with a LLOQ (Lower Limit of quantitation) of 0.500 ng/mL.

### b. Plasma PK Results

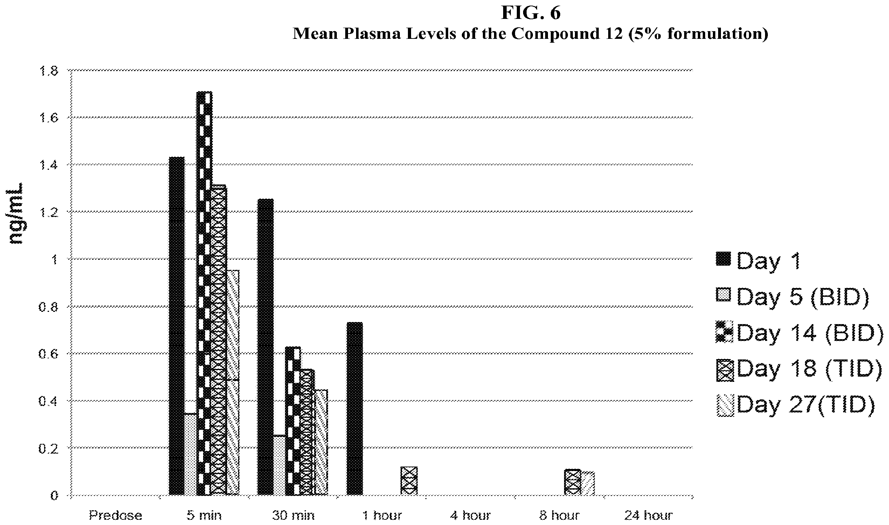

Compound 12 plasma concentrations were BLOQ (below assay lower limit of quantitation) (<0.500 ng/mL) at all timepoints following single- and multiple-doses of 0.1% and 0.3% Compound 12 dose strengths and in 3 of 5 subjects that received the 1% Compound 12 dose strength. Measureable levels of Compound 12 were seen in the plasma of one subject dosed with 1% Compound 12 at the earliest timepoint (5 minutes post-dose) on Days 14 and 27 but were BLOQ for subsequent timepoints. Measurable levels were observed more frequently following administration of the 5% dose strength throughout the trial, although levels were quite low (< 3 ng/mL) and generally were not detectable after the first hour following administration (Figure 6). LFA-1 levels in in vitro cell assays (cell attachment and SEB IL-2 release) where IC50 values of 2 nM have been observed are approximately 0.1 nM. LFA-1 levels in blood are approximately 10 nM. The IC50 for Compound 12 inhibition of SEB stimulated IL-2 release in whole human blood is 69 nM. Compound 12 levels greater than LFA-1 levels are needed to inhibit leukocyte function. Therefore, no significant inhibition of systemic leukocytes is expected from Compound 12 ophthalmic drops.

Plasma Compound 12 half-life or exposure parameters could not be accurately assessed following administration of the Compound 12 Ophthalmic Solution at any dose strength in any study period because the plasma Compound 12 concentrations were not detectable or rapidly declined BLOQ within 1 to 4 hours of dosing.

### c. Tear PK Analysis

Tear samples of Compound 12 were collected in both eyes pre-dose, at 30 minutes post-dose and at 1, 4, 8, and 24 hours post-dose on Days 1, 5, 14, 18, and 27 of the Phase 1 study using paper Schirmer tear strips. A 48-hour post-dose sample was obtained following Day 1, 14, and 27. Tear Compound 12 concentrations were determined using a validated LC/MS/MS method with a LLOQ of 0.500 ng/mL.

### d. Tear PK Results

Dose related increases in tear AUC (area under the concentration-time curve) and Cₘₐₓ (maximum observed plasma concentration) values were seen on dosing day 1 and were generally maintained at the timepoints evaluated throughout the trial. BID (two times daily) and TID (three times daily) dosing produced higher Cₘₐₓ and AUC values relative to a single dose, but there were no significant differences in exposure between BID and TID dose schedules. There was clear evidence of Compound 12 exposure in the anticipated therapeutic dose range and no obvious evidence of accumulation with multiple ocular dose administration.

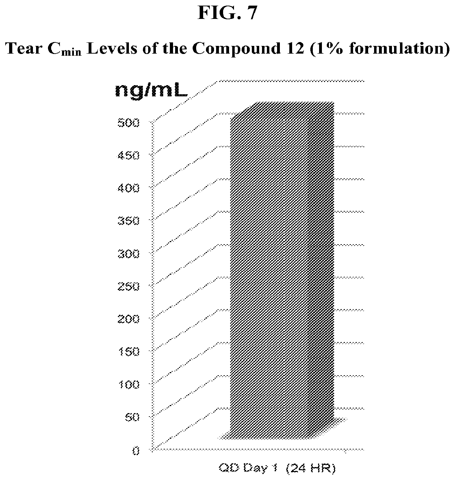

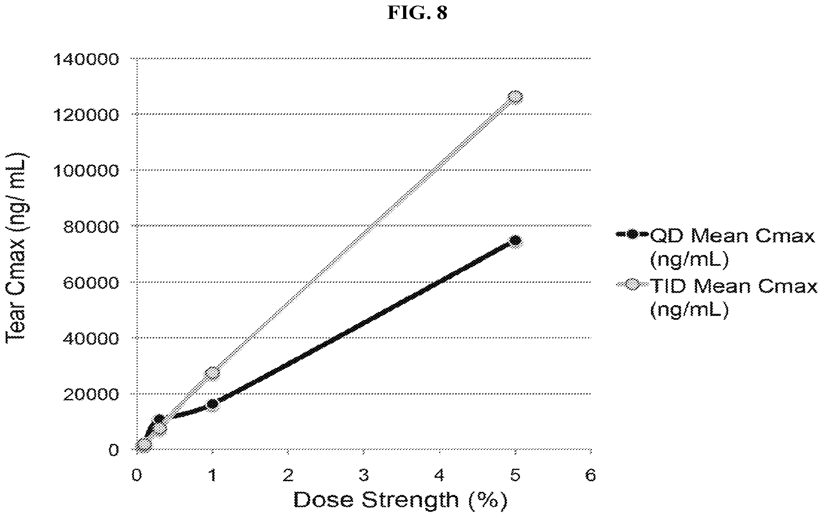

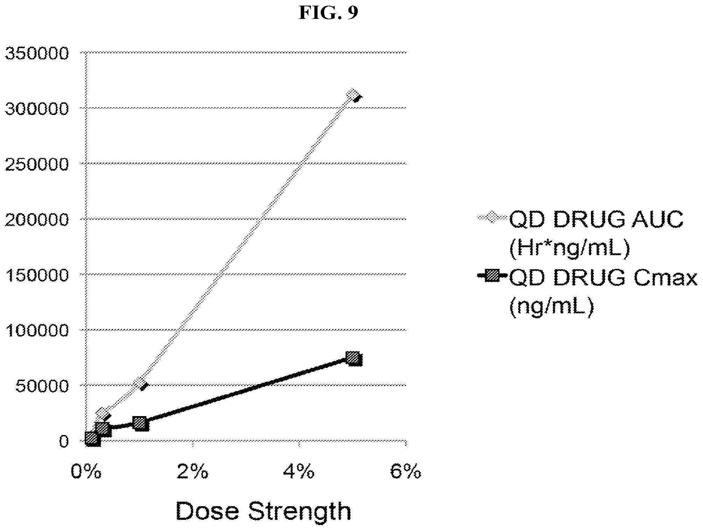

**Figure 7** illustrates 1% Compound 12 tear Cₘᵢₙ levels. **Figure 8** illustrates that dose was proportional to the₌Compound 12 Cₘₐₓ tear levels. **Figure 9** illustrates that dose was proportional to Compound 12 QD AUC and Cₘₐₓ in tears.

Overall, Compound 12 Ophthalmic Solution administered by topical ocular instillation to healthy adult subjects at dose strengths up to 5% TID appears safe and well-tolerated and appropriate for further investigation in subjects with ocular inflammation secondary to allergic conjunctivitis or dry eye.

### B. NONCLINICAL STUDIES Compound 12 IND-Enabling Nonclinical Program (Safety Pharmacology and Toxicology Studies)

### 1. Preclinical Toxicology Formulation

**Table 14.**

| |
|---|
| Phosphate buffered saline |
| pH 7 |
| 290 mOsM/l |
| Compound 12 sodium salt |
| 4 dose levels (0.1% to 3%) |
| EDTA |
| Parabens preservative |
| 0.02% methyl parabens |
| 0.04% propyl parabens |
| Multidose dropper bottle |

### 2. Safety Pharmacology

An *in vitro* study to evaluate the effects of Compound 12 on hERG channel current (a surrogate for I_{Kr}, the rapidly activating, delayed rectifier cardiac potassium current) was conducted in stably transfected kidney HEK293 cells. Single doses of Compound 12 were 20 µM, 100 µM, 200 µM, and 600 µM. Compound 12 effects on the current were weak (IC₅₀ of 478 µM) indicating minimal risk of I_{Kr} channel inhibition given the low systemic exposure observed following topical ocular administration.

The cardiovascular effects of Compound 12 in conscious telemetry-instrumented dogs (beagles) when administered via IV bolus injection were assessed. No effects on electrocardiography or hemodynamic parameters were observed.

The effects of Compound 12 on the CNS when administered as a single dose via bolus IV injection were assessed in rats. Transient miosis was observed in animals given 10.0 mg/kg from 1 minute to 6 hours postdose in 2/6 animals at each time point. No effect on any other parameters was observed.

Respiratory function (tidal volume, respiration rate, and minute volume) in rats following a single IV bolus dose of Compound 12 using head-out plethysomograph chambers was assessed. No adverse changes in respiratory function or adverse effects were observed at any dose.

**3. Genotoxicity studies:** Compound 12 displayed no effect in *in vitro* Ames chromosomal aberration assays or an *in vivo* rat micronucleus study.

### a. In Vitro Ames Bacterial Reverse Mutation Assay

In an Ames assay, Compound 12 did not cause an increase in the mean number of revertants per plate with any of the tester strains either in the presence or absence of microsomal (S9) enzymes. Therefore, Compound 12 was judged to be not mutagenic.

### b. In Vitro Chromosomal Abberation Assay in CHO cells

The ability of Compound 12 to induce chromosomal aberrations was assessed in cultured Chinese hamster ovary (CHO) cells with and without an exogenous metabolic activation following 20 hours of co-incubation. Compound 12 is considered negative for inducing structural chromosomal aberrations in CHO cells with and without metabolic activation, except at a single toxic dose without metabolic activation (3-hour treatment; 3500 µg/mL). The biological relevance of this response is equivocal due to cytotoxicity.

### c. In Vivo Mouse Bone Marrow Micronucleus Assay

The ability of repeated IV administrations of Compound 12 to induce in vivo clastogenic activity and/or disruption of the mitotic apparatus, by detecting micronuclei in polychromatic erythrocytes (PCE), was assessed in CD-1® (ICR) BR mice by evaluating their bone marrow. Based on the results of this study, Compound 12 is considered negative in the mouse bone marrow micronucleus assay.

**4. Acute Toxicity Studies:** For single dose IV in rats, the no observable adverse effect level (NOAEL) was 10 mg/kg IV. For escalating single dose IV and 7-day repeated dose with TK (toxicokinetics) in dogs, the NOAEL was 10 mg/kg IV. For single dose ocular tolerance in rabbits, the NOAEL was 3.5 mg/eye/3x per day (10%).

**5. Repeated Dose Toxicity Studies:** In a 4-week IV toxicity study in dogs with 2-week recovery, the NOAEL was 10 mg/kg. In a 13-week IV toxicity study in rats with 4-week recovery, the NOAEL was 30 mg/kg. In a 13-week ocular toxicity study in rabbits with a 4-week recovery, the NOAEL was 1.05 mg/eye/3x per day (3%). In a 13-week ocular toxicity study in dogs with a 4-week recovery, the NOAEL was 1.05 mg/eye/3x per day (3%).

### 6. ADME Studies

The absorption, distribution, metabolism and excretion (ADME) of Compound 12 was characterized in studies conducted in rats, rabbits and dogs utilizing two routes of administration; intravenous and topical ocular administration, the clinical route of administration. An in vitro hepatocyte study was also performed.

Compound 12 levels were assessed in plasma, tear and vitreous humor samples by tandem mass spectrometry. Some in vivo studies used [¹⁴C]-Compound 12 to determine PK and the extent of absorption, distribution, and excretion of [¹⁴C]-Compound 12-derived radioactivity. Additionally, the metabolic profile and identification of metabolites of [¹⁴C]-Compound 12 were determined in plasma, urine and feces.

Single dose ocular and IV dose administration ADME studies were conducted in pigmented (Long-Evans strain) and albino (Sprague Dawley strain) rats using [¹⁴C]-Compound 12. Quantitative whole body radiography assessments were performed.

Male and female rats received a single dose of 1 mg/eye or 10 mg/kg IV [¹⁴C]-Compound 12. The main route of excretion following either ocular or IV administration was the feces, accounting for approximately 60% (ocular administration) and 95% (IV administration) of the administered radioactivity over 0 to 168 hours postdose. Urinary excretion accounted for up to 2% of the administered radioactivity. The highest tissue levels of [¹⁴C]-Compound 12 were measured in the tissues of the gastrointestinal tract with either ocular or IV dosing. With ocular administration, [¹⁴C]-Compound 12 was also measured in ocular tissues and those of excretion, indicating that the administered dose passed from the eye through the nasal turbinates, into the esophagus and was ultimately excreted through the gastrointestinal tract. These data indicate that ocular, nasal, or oral administration of Compound 12 will result in ultimate excretion through the gastrointestinal tract. A signficant proportion of drug dose administered as ocular drops, distributed locally to the periocular region, and more interestingly via nasal turbinates into the gastrointestinal tract. Drug is seen to accumulate first in the epithelium of the GI tract and pass into the liver via the portal vein, where it is eliminated from the liver and re-delivered back to the lower GI tract. Little or no drug is observed in systemic distribution. Therefore, for administration of the compound of Formula I via either aerosol or drops to the nose, or via oral administration may provide similar specific direct localized delivery to the epithelium of the upper GI and localized delivery to the lower GI via clearance through the liver. In both cases, little or no systemic delivery of drug may be delivered.

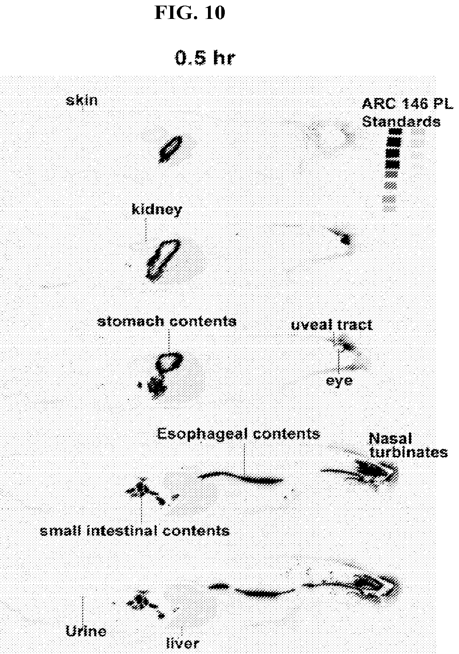

Following a topical ocular dose of [¹⁴C]-Compound 12 to male Sprague Dawley (albino) rats, the distribution of radioactivity into tissues was limited at the first time point (0.5 hour postdose) and was generally associated with the gastrointestinal tract, the tissues associated with metabolism, and the eye. **Figure 10** illustrates a whole body autoradiograph for a male Sprague Dawley Animal 0.5 hour after a single topical ocular administration of [¹⁴C]-Compound 12 (1 mg/eye). The highest concentrations of radioactivity were determined at this time point in esophageal contents, nasal turbinates, and small intestinal contents, with concentrations of 399000, 352000, and 349000 ng equivalents [¹⁴C]-Compound 12/g, respectively. However, it should be noted that the measurements in these tissues were above the upper limit of quantification and therefore should be interpreted with some caution. High levels of radioactivity were also determined in the esophagus and stomach contents. Radioactivity was detected in the eye at this time point, with a concentration of 18100 ng equivalents [¹⁴C]-Compound 12/g. Low levels of radioactivity were also associated with the liver (272 ng equivalents [¹⁴C]-Compound 12/g), kidney (151 ng equivalents [¹⁴C]-Compound 12/g) and uveal tract (9330 ng equivalents [¹⁴C]-Compound 12/g).

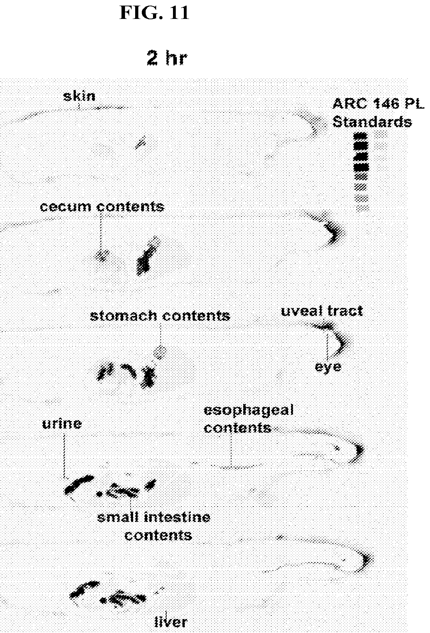

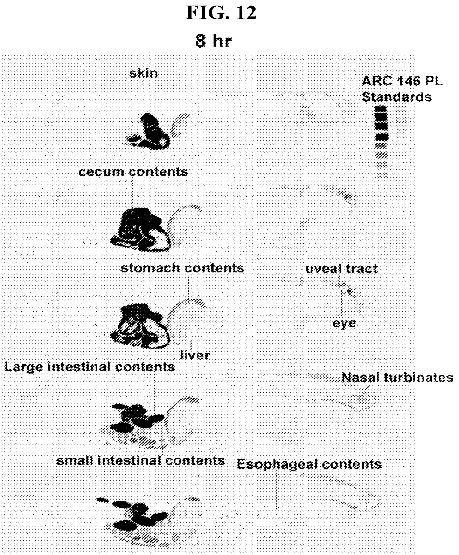

Concentrations of radioactivity in the eye and eye lens had declined considerably by 2 hours postdose; with the level in the eye lens BLQ. Radioactivity concentrations had also declined in the esophagus and esophageal contents by approximately 50- and 100-fold at 2 hours postdose. **Figure 11** illustrates a whole-body autoradiograph for a male Sprague Dawley Animal 2 hours after a single topical ocular administration of [¹⁴C]-Compound 12 (1 mg/eye). At 8 hours postdose level of radioactivity had fallen in all tissues; however, high concentrations were associated with the large intestinal contents (133000 ng equivalents [¹⁴C]-Compound 12/g) and cecum contents (57600 ng equivalents [¹⁴C]-Compound 12/g), indicating the passage of radioactivity through the gastrointestinal tract. **Figure 12** illustrates a whole-body autoradiograph for a male Sprague Dawley Animal 8 hours after a single topical ocular administration of [¹⁴C]-Compound 12 (1 mg/eye).

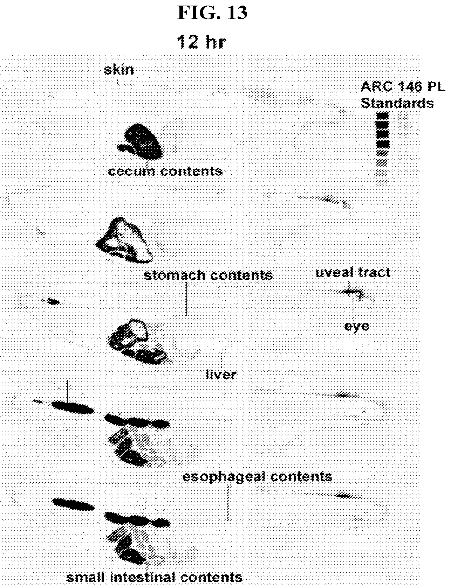

By 12 hours postdose radioactivity concentrations had decreased further, the maximal concentrations being associated with the cecum and large intestinal contents. The concentration determined in the uveal tract increased at this time point to 610 ng equivalents [¹⁴C]-Compound 12/g. **Figure 13** illustrates a whole-body autoradiograph for a male Sprague Dawley Animal 12 hours after a single topical ocular administration of [¹⁴C]-Compound 12 (1 mg/eye).

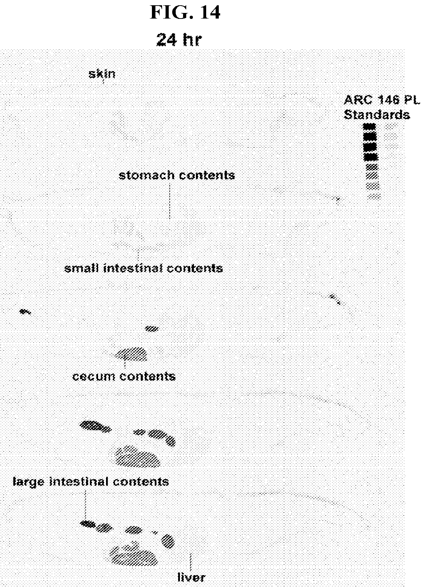

Radioactivity concentrations at 24 hours postdose were maximal in the cecum contents (5870 ng equivalents [¹⁴C]-Compound 12/g) and the large intestinal contents (18000 ng equivalents [¹⁴C]-Compound 12/g); low levels were also present in the small intestinal and stomach contents. **Figure 14** illustrates a whole-body autoradiograph for a male Sprague Dawley Animal 24 hours after a single topical ocular administration of [¹⁴C]-Compound 12 (1 mg/eye). For all other tissues, with the exception of the non-pigmented skin and the liver radioactivity was not detectable.

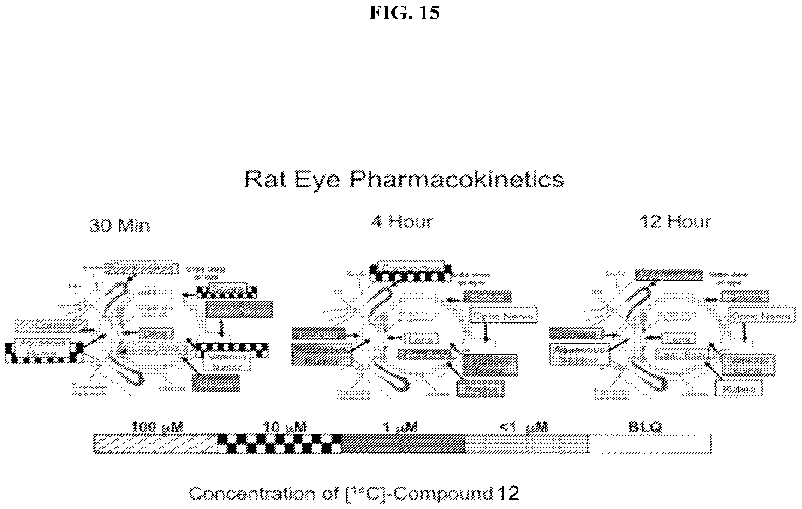

Low levels of [¹⁴C]-Compound 12 were measured in the vitreous humor at all timepoints following ocular dosing and up to 2 hrs following an IV dose (see schematic in **Figure 15** and **Table 15** for ocular dosing in rats).

**Table 15 Compound 12 Concentration, ng Equivalents [¹⁴C]-Compound 12/g tissue.**

| Physical region | 0.5 hour after administration | 4.0 hours after administration |
|---|---|---|
| Aqueous humor | 1770 | 116 |
| Conjunctiva (bulbar) | 31500 | 4480 |
| Conjunctiva (palpebral) | 26300 | 21830 |
| Cornea | 17150 | 1346 |
| Iris-ciliary body | 17550 | 500 |
| Lens | 38.8 | 9.69 |
| Optic Nerve | 796 | 0 |
| Retina and Choroid (with RPE) | 510 | 46.7 |
| Sclera | 2750 | 387 |
| Vitreous Humor | 1330 | 183 |

Tissue distribution of [¹⁴C]-Compound 12 in pigmented and albino rats was comparable and indicated that Compound 12 did not preferentially bind to melanin. There were no obvious differences seen in results from male and female rats. Furthermore, no preferential distribution of [¹⁴C]-Compound 12-derived radioactivity was seen in red blood cells and no metabolites were isolated from samples of pooled plasma, urine and fecal homogenates collected up to 168 hrs following either a topical ocular or IV dose administration of [¹⁴C]-Compound 12.

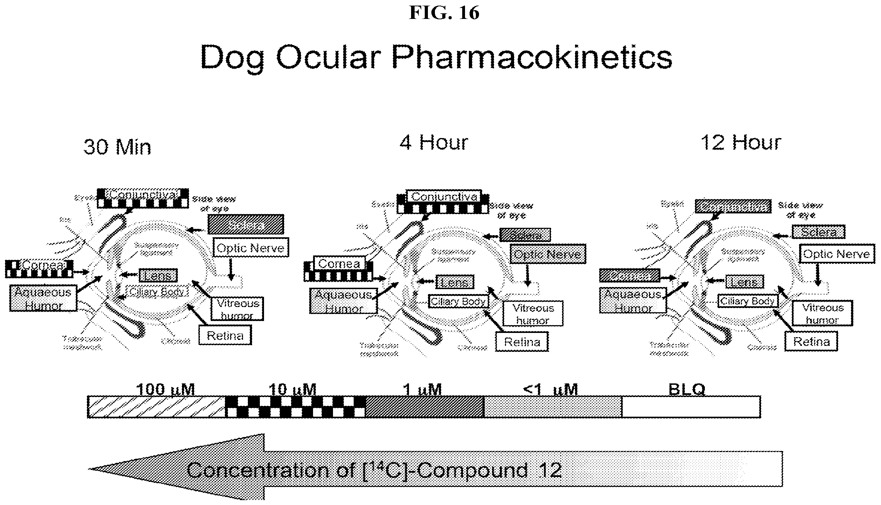

Similar single dose studies using [¹⁴C]-Compound 12 utilizing the same routes of administration were conducted in male and female dogs (3 mg/eye or 3 mg/dog) and showed comparable patterns of excretion, distribution and metabolism as rats. Following an ocular dose, the highest average [¹⁴C]-Compound 12 levels were detected in anterior ocular tissues (see **Figure 16**). Lower levels were detected in posterior ocular tissues, indicating that absorption into the eye had occurred. The metabolic profile in pooled plasma, urine and fecal homogenate samples was comparable to that seen in rats, with no metabolites detected up to 168 hrs post-dose. No differences in results from male and female dogs were observed.

Compound 12 levels in conjunctiva/cornea are greater than 1 micromolar/100 nanomolar for 16 hrs (dog/rat).

### a. Compound 12 Pharmacokinetics after Single and Repeated IV Administration

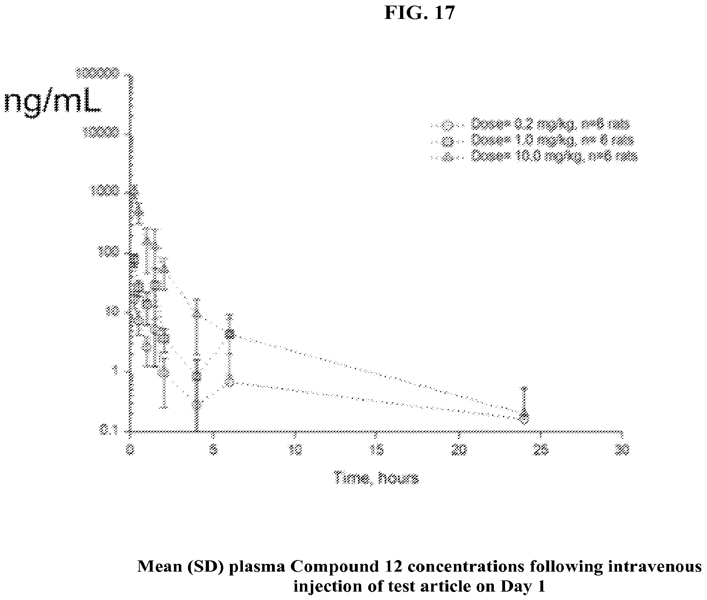

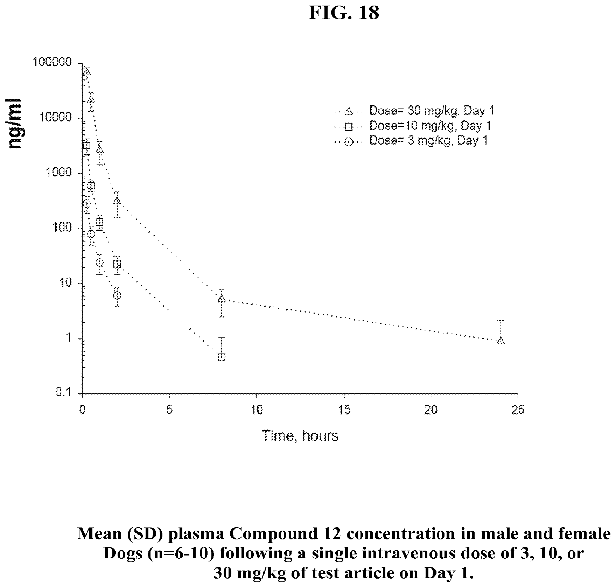

Plasma Compound 12 concentrations over time following a single IV doses in rats and dogs are shown in **Figures 17** and **18**, respectively. Plasma concentrations of Compound 12 declined in an expected, exponential manner following a single IV bolus dose in both species.

The plasma PK parameters determined using standard noncompartmental methods after a single IV administration of Compound 12 to rats at doses ranging from 0.2 to 30.0 mg/kg or to dogs after single doses up to 30 mg/kg and 7 daily doses of 3 or 10 mg/kg are shown in **Table 16** (rats). PK results from both species show very high clearance of Compound 12 (liver blood flow is ∼ 3.3 L/hr/kg and 1.9L/hr/kg in rats and dogs, respectively; (Davies, 1993, Pharm Res). Rat PK data indicated a high distribution volume, and moderate half-life following a single IV dose while low distribution volume and a shorter half-life drug was seen following IV administration to dogs. There was no obvious accumulation of Compound 12 in plasma after daily administration of Compound 12 for 7 days as plasma Compound 12 Cₘₐₓ and AUC₀₋ₙ values measured on Study Day 1 approximated those obtained on Study Day 7.

**Table 16 Summary of Plasma PK Parameters Rats Following a Single IV Bolus Dose of Compound 12³**

| | **CL** | **Vss** | **T_{1/2}** | **MRT** | **Cₘₐₓ** | **AUC₀₋ₙ** |
|---|---|---|---|---|---|---|
| **Dose** | **L/hr/kg** | **L/kg** | **hr** | **hr** | **ng/mL¹** | **hr x ng/mL²** |
| 10.0 mg/kg | 10.4 | 9.56 | 3.76 | 0.920 | 1056 | 728 |
| 30.0 mg/kg⁴ | - | - | - | - | 5117.3 | 2345.5 |

| | | | | | | |
|---|---|---|---|---|---|---|
| ¹ Maximum observed plasma Compound 12 concentration estimated from the mean concentration versus time profile. ² Plasma Compound 12 AUC₀₋ₙ during the dose interval estimated from the mean concentration versus time profile. ³ Estimated from mean plasma Compound 12 concentration versus time profiles. ⁴ From rat safety pharmacology study | | | | | | |

In longer term repeated-dose studies, dogs and rats received daily IV bolus doses of 3, 10 or 30 mg/kg/day for 4 and 13 weeks, respectively. As was seen in the 7-day dog study, plasma Compound 12 concentrations declined in an expected, exponential manner and there was no evidence of Compound 12 accumulation in the plasma. The plasma clearance, distribution volume, and half-life of Compound 12 in dogs were dose-dependent over the dose range of
3 mg/kg to 30 mg/kg. In rats, the plasma Compound 12 exposure data suggested nonlinear disposition of Compound 12 following daily IV doses ranging from 10 to 30 mg/kg and unexpected accumulation at Week 13 (Table 17).

**Table 17: Plasma Compound 12 Exposure Parameters in Rats Following Daily IV Bolus Doses for 13 Weeks³**

| | **Dose= 3 mg/kg** | | **Dose= 10 mg/kg** | | **Dose= 30 mg/kg** | |
|---|---|---|---|---|---|---|
| | Cₘₐₓ ng/mL¹ | AUC₀₋ₙ hr x **ng/mL²** | Cₘₐₓ ng/mL¹ | AUC₀₋ₙ hr x **ng/mL²** | Cₘₐₓ ng/mL¹ | AUC₀₋ₙ hr x **ng/mL²** |
| Day 1 | 305.2 | 148.3 | 1045.3 | 535.6 | 5117.3 | 2345.5 |
| Week 13 | 377.5 | 241.4 | 1691.5 | 907.1 | 16932.8 | 7471.5 |

| | | | | | | |
|---|---|---|---|---|---|---|
| ¹ Maximum observed plasma Compound 12 concentration during the dose interval. ² Plasma Compound 12 AUC₀₋ₙ during the dose interval. ³ Estimated from mean plasma Compound 12 concentration versus time profile, n= 6 rats (3 males and 3 females) per timepoint. | | | | | | |

### b. Compound 12 Pharmacokinetics after Single and Repeated Ocular Administration

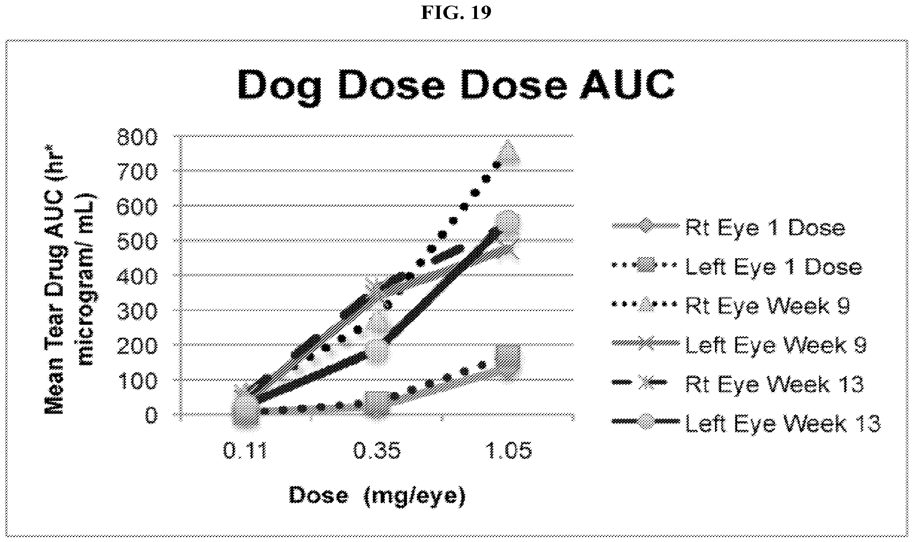

After a single topical ocular instillation of a 0.1, 1.0 or 3.0% dose strength of Compound 12 Ophthalmic Solution (0.105, 0.35 and 1.05 mg/eye, respectively), mean tear Compound 12 concentrations rose in a dose-related manner achieving maximal values within 30 minutes of administration and returning to baseline by 4 hours. The tear Cₘₐₓ and AUC₀₋ₙ of Compound 12 generally increased with increasing dose. Figure 19 illustrates that the dose of Compound 12 is proportional to PK in tears (AUC) for dogs. For example, mean tear Cₘₐₓ values were 34,014 ng/mL, 21460 ng/mL and 313,906 ng/mL in the right eyes of rabbits dosed with 0.105, 0.35 and 1.05 mg/eye, respectively. Mean tear AUCs were 18864 hr x ng/mL, 18931 hr x ng/mL and 182978 hr x ng/mL in the right eyes from the same dose groups, respectively.

Plasma Compound 12 concentrations rose after topical ocular instillation as the drug moved from the ocular application site into the plasma circulation. Dose-related amounts of Compound 12 were detected in the plasma of dogs and rabbits 30 minutes following topical ocular administration. Plasma Compound 12 concentrations rapidly declined from maximum values measured at about 0.25 hrs post-dose to baseline levels by about 4 hours, probably owing to the high Compound 12 plasma clearance as seen in the IV administration studies. Plasma Cₘₐₓ (mean ± SD) values were 11.7 ± 8.80 ng/mL, 13.1 ± 2.12 ng/mL, and 38.9 ± 19.7 ng/mL and AUC₀₋ₙ (mean ± SD) values were 5.19 ± 5.39 hr x ng/mL, 7.35 ± 1.52 hr x ng/mL, and 22.9 ± 10.1 hr x ng/mL in the 0.105, 0.35, and 1.05 mg/eye/dose groups, respectively.

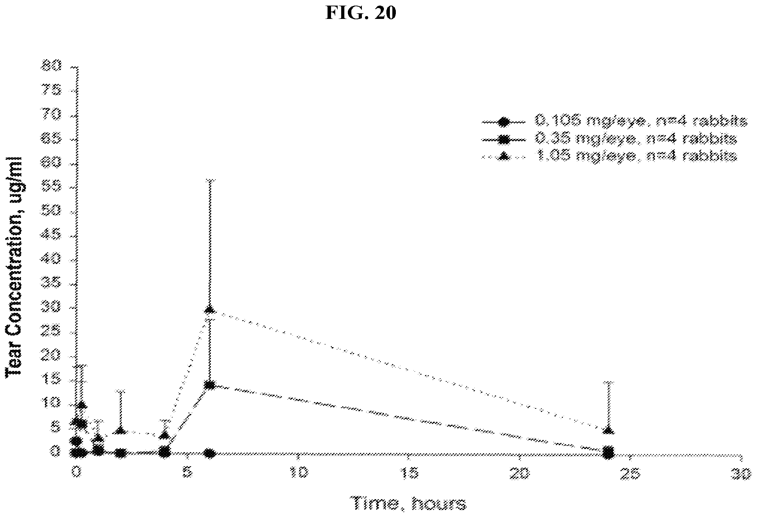

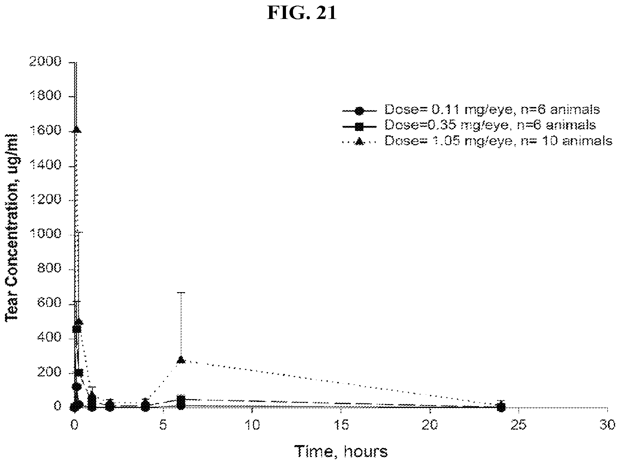

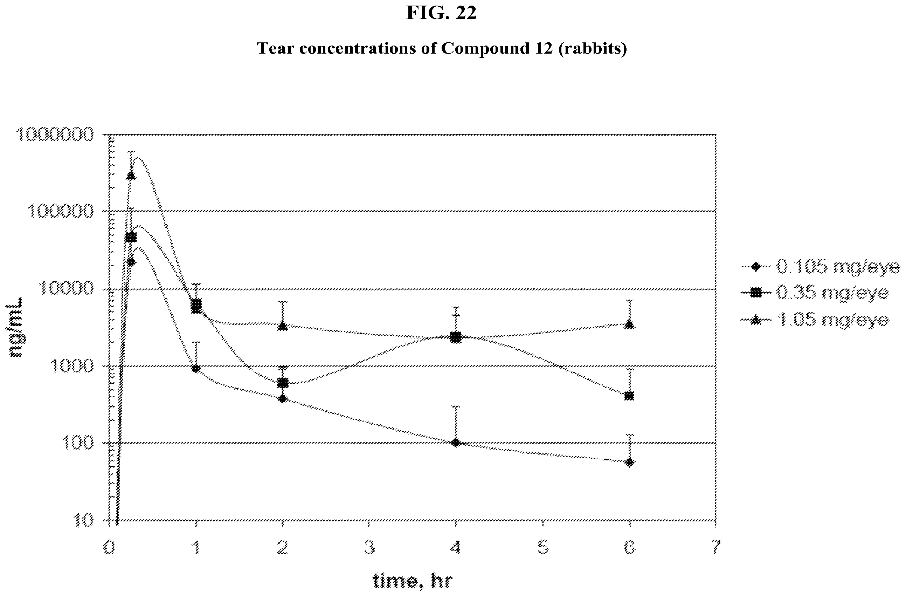

In repeated dose studies conducted in rabbits and dogs, Compound 12 Ophthalmic Solution was administered TID by bilateral ocular instillation at the same doses as for single dose studies for 13 weeks. A pilot study in dogs administered 3.5 mg/eye (10% dose strength) for 3 days. The Cₘₐₓ and AUC₀₋ₙ of Compound 12 in tear samples increased expectedly with increasing dose in rabbits and dogs. The Cₘₐₓ and AUC₀₋ₙ data indicate that Compound 12 accumulated in dog tears by Week 9 during TID instillation, but thereafter continued accumulation was not noted. A similar pattern was observed in the rabbit study. Representative tear concentration over time profiles measured after 13 weeks of TID ocular dosing in rabbits and dogs are shown in **Figures 20** and **21****,** respectively (left eye, TID, ∼4 hours apart). TK (toxicokinetics) analyses indicate adequate ocular Compound 12 exposure with tear levels above 1 µM (600 ng/mL) throughout the day. **Figure 22** illustrates mean Compound 12 tear concentrations in right and left eyes of rabbits following topical instillation of a single dose.

Compound 12 was not detected in the vitreous fluid in both 13-week rabbit and dog studies in samples obtained at sacrifice (terminal and recovery phase sacrifices). Variable levels of Compound 12 were seen in the vitreous fluid of dogs dosed TID for three days with 3.5 mg/eye (10%) and ranged from BLOQ to 18 ng/mL.

Nonclinical studies showed that about 6.9 to 32% of the Compound 12 ocular dose was absorbed from the ocular topical instillation site into the systemic circulation but this systemic availability estimate has been based on limited available data which includes an ocular dose that is 1/100^{th} the intravenous dose. Low systemic plasma exposure to the drug was observed in animals after ocular instillation. Importantly, the Compound 12 plasma clearance is high in these species indicating that the absorbed Compound 12 is efficiently removed from the systemic circulation, thereby assisting to minimize systemic exposure.

The PK profiles from all nonclinical species support a clinical dose topical ocular instillation regimen of up to three times per day for at least 13 weeks.

### c. Pilot Ocular Tolerance of Topically Administered Compound 12 in Dogs-PK

A pilot ocular tolerance of topically administered Compound 12 in dogs-PK was performed. Animals were dosed with 35 µL of Compound 12 TID (0, 4, 8 hrs). 1% solution was administered on days 1-14; 3% solution was administered on Days 17-21, and 10% solution was administered on Days 24-27. Compound 12 trough levels in tear/periocular tissue are greater than 1000 times the IC₅₀ for T-cell attachment/IL-2 release. Compound 12 is safe and well tolerated at up to 10% strength at 3 doses/day. Dose dependent increases in Compound 12 concentration were detected in tear (30 min - 16 hours) and plasma (30 min) following ocular instillation. Vitreous concentrations of Compound 12 were greater than 1000 fold lower.

### C. DERMAL

### 1. Compound 12 Preclinical Dermal Studies

Compound 12 displays 2% (w/w) solubility in water/glycol/transcutol solution and 10% (w/w) solubility in ethanol/glycol/transcutol solution. Solubility studies suggest an emulsion formulation. Prototypes have been developed and tested on microtomed human skin from elective surgery at 1% (w/w). The forms include gels, ointment, or lotion. Stability and compatibility has been demonstrated in all formulations. Skin transport studies performed with LC/MS/MS analysis indicate high Compound 12 levels in epidermis and dermis and low levels in the receiver. There can be greater than 10 micromolar Compound 12 in dermis, with 2-4% dose penetration, as determined using [¹⁴C]-Compound 12. Pilot rat and mini-pig studies demonstrate low systemic exposure which indicates drug penetration into vascularized levels of skin (i.e. dermis).

### 2. Nonclinical dermal program

### Dermal Sensitization Study in Guinea-Pigs: Buehler test

A Buehler test using healthy, young adult (4 to 6 weeks), randomly bred albino guinea pigs (strain Crl:(Ha)BR) is used to determine the potential of Compound 12 to induce hypersensitivity. The diet consists of certified guinea pig diet (#5026, PMI Nutrition International LLC) ad libitum. Water is administered ad libitum. Room temperature is 18 to 26°C, relative humidity is 30 to 70%, and a 12-hour light/12-hour dark cycle is used. Animals are acclimated for at least 5 days.

**Experimental design:** 34 acclimated animals are placed in an irritation screening group of 4 guinea pigs, a test group of 10 guinea pigs (Group 1), a naive control group of 5 guinea pigs (Group 2), 10 positive control guinea pigs (Group 3), and 5 positive naive control guinea pigs (Group 4).

**Irritation screen:** Hair from the back of 4 animals is removed by clipping and four application sites per animal are selected. Each site is treated with 0.4 mL of 0.1%, 1%, or 10% w/v Compound 12 and 0.4-g dose of Compound 12. Appropriate concentrations of Compound 12 are selected for induction exposure (highest to cause mild-to-moderate skin irritation) and challenge exposure (highest non-irritant dose).

**Definitive phase:** Prior to the test, hair is removed using electric clippers from animals in Group 1. Occlusive patch systems (Hill Top Chamber®, 25-mm diameter) are saturated with 0.4 mL solution of vehicle with a concentration of compound of Formula I as determined in the irritation screen. The occlusive patches are applied to the flanks of Group 1 guinea pigs for 6 hours. Restraints are used to maintain even pressure over the patches. The procedure is repeated on days 6-8 and 13-15 after the initial exposure. The positive control material, HCA (alpha-hexylcinnamaldehyde), 2.5% w/v in ethanol, is applied in a similar manner to the Group 3 guinea pigs. The naive control animals (Groups 2 and 4) are not treated during the induction phase.

Two weeks after the last induction patch, animals are challenged with patches saturated with a nonirritating concentration of Compound 12 applied to the dorsal anterior right quandrant, and along the dorsal anterior left quadrant with a challenge application of water. Group 2 animals (naive control) are shaved with electric clippers and treated on the dorsal anterior right quadrant with Compound 12 and along the dorsal anterior left quandrant with vehicle. HCA is administered at 5.0% and 7.0% w/v in acetone on two respective challenge sites along the right side of each animal in Group 3 in the same manner as the induction phase (0.4 mL dose volume). Group 4 animals are treated with two challenge applications of the postive control material in the same manner as Group 3.

After 6 hr, the patches are removed and the area depilated (by applying Nair®). Test sites are evaluated visually 24 and 48 hr after patch removal. Animals developing erythematous responses are considered sensitized (if irritant control animals do not respond). The number of positive reactions and the average intensity of the responses are calculated. Reactions to the challenge doses determine the sensitization. Grades of 1 or greater in the test animals to a respective material indicates evidence of sensitization, provided that grades of less than one are seen in the naive control animals to this same material. If grades of one or greater are noted in the naive control animals, then the reactions of test aniamls exceeding the most severe naive control reactions are considered sensitization reactions.

### 3. Compound 12 Pilot Rat Dermal Study

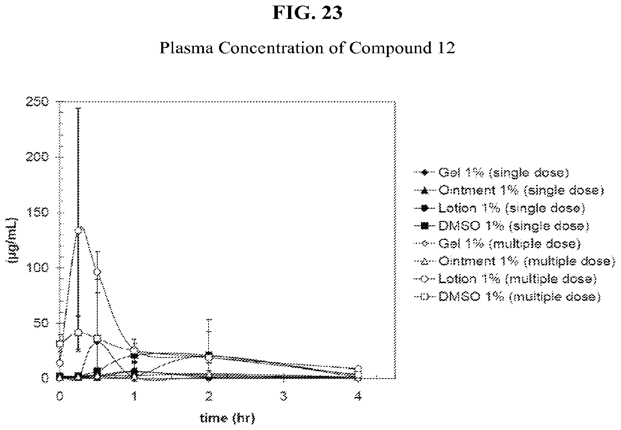

The safety and tolerability of prototypical dermal formulations (1% lotion, ointment, and gel) were assessed on rats given TID for seven consecutive days-approximately 6 cm² with 10 mg/cm². 1% DMSO was given as a high bioavailability control. **Figure 23** illustrates that Compound 12 is detectable in serum.

### 4. Compound 12 Pilot Mini-Pig Dermal Study

The tolerability and systemic exposure of various formulations of Compound 12 (DMSO, gel, ointment, lotion at 1%) was assessed by giving these formulations to mini-pigs as multiple dermal does TID for 7 days, approximately 50 cm² with 10 mg/cm². One pig/dose formulation was used. In-life PK analysis was completed. No toxicity was reported with any formulation. Plasma PK revealed low levels of Compound 12 in all groups but below the LLOQ of 0.5 ng/ml.

The rat and mini-pig pilot studies indicate that PK were comparable with gel and ointment and Compound 12 is safe for evaluation in humans as a gel or ointment formulation.

Prototypical 1% topical derm formulations have been developed (lotion, gel, and ointment). There is good delivery of Compound 12 to epidermis and dermis in human skin Franz cell. Pilot toxicology studies of lotion, gel, and ointment reveal the PK demonstrates good bioavailability.

### Example 12: Phase 2 Trial Allergic conjunctivitis (Reference Example)

Subjects with positive history of ocular allergies and a positive skin test reaction to cat hair, cat dander, dog dander, grasses, ragweed, trees, dust mites, and/or cockroaches within the past 24 months (as demonstrated by positive skin tests) will be challenged with allergen administered to the conjunctiva to induce ocular itching and conjunctival redness. Subjects will be treated with both preserved and unpreserved formulations of Compound 12 ophthalmic drops. Unpreserved drug will be supplied as a sterile unit dose in a single use blow-fill-seal container containing Compound 12 formulated in PBS. Preserved drug will be supplied as a sterile multi-use container containing Compound 12 formulated in PBS containing preservative. Each group of test subjects will be treated QD, BID or TID with different dose strengths of Compound 12 or placebo in preserved or unpreserved formulations. Drug will be self administered by each subject as a single drop to each eye once, twice or three times a day as directed. Administered dose strengths will include placebo (PBS vehicle) 0.1%, 0.3%, 1% and 5% solutions of Compound 12.

At enrollment, subjects will be evaluated for sensitivity to allergen using a conjunctival provocation test (also referred to as a "conjunctival allergen challenge test"). Patients responding with itchiness and redness of at least 2.0 [0-4 point scale with 0.5 point increments] will be supplied with drug and required to record the administration of each drug dose in patient diaries. Patient response to allergen (itchiness and redness) will be assessed in follow-up visits with subsequent challenges 6,7 days and/or 13,14 days after their enrollment. Challenges in these visits will occur at variable times (approximately 15 minutes, 8 hours, or 24 hours) after their last Compound 12 dose. Conversely, patients will be challenged with allergen and then treated with Compound 12 at variable times (5 minutes, 10 minutes, 20 minutes, 40 minutes or 1 hour) after the challenge. Patient exams will include assessments of safety, visual acuity, slit-lamp exam, dilated fundoscopy. A mean difference of at least 1.0 point [0-4 point scale with 0.5 point increments] in ocular itching and hyperemia comparing Compound 12 and vehicle is considered clinically meaningful when evaluated in the first 10 minutes following allergen challenge.

Objective measures of efficacy (physician reported) will include: 1) conjunctival hyperemia, 2) episcleral hyperemia, 3) ciliary hyperemia, and 4) chemosis.

Subjective measures of efficacy (patient reported) will include: 1) ocular itching, 2) blepharitis, 3) rhinorrhea, 4) nasal congestion, and 5) nasal pruritis.

For seasonal allergies, subjects will be treated daily at QD, BID or TID doses for up to 8 consecutive weeks during peak allergy season for common grass and tree pollens (also referred to as "environmental studies"). Similar measures of objective and subjective efficacy measures will be evaluated.

For either environmental or conjunctival provocation studies, a safety trial of at least 6 months will be conducted in normal adult and pediatric patients.

Results of this trial will support regulatory claims to the treatment or prevention of signs and symptoms from allergic conjunctivitis (both seasonal and perennial); steroid sparing treatment of allergic conjunctivitis - no steroid safety events (glaucoma, cataracts); Compound 12 can be used in conjunction with mast cell stabilizers and antihistamines to enhance or prolong efficacy; treatment of both ocular and nasal signs and symptoms of allergy.

### Example 13: Phase 2 Trial for Dry Eye (Reference Example)

Subjects with moderate to severe dry eye will be treated for 12 weeks (efficacy trials) and up to 1 year (safety trials) with both preserved and unpreserved formulations of Compound 12 ophthalmic drops. Unpreserved drug will be supplied as a sterile unit dose in a single use blow-fill-seal container containing Compound 12 formulated in PBS. Preserved drug will be supplied as a sterile multi-use container containing Compound 12 formulated in PBS containing preservative. Each group of test subjects will be treated QD or BID with different dose strengths of Compound 12 or placebo in preserved or unpreserved formulations. Drug will be self administered by each subject as a single drop to each eye once or twice a day. Administered dose strengths will include placebo (PBS vehicle) 0.1%, 0.3%, 1% and 5% solutions of Compound 12.

At enrollment, subjects will be evaluated for signs and symptoms of Dry Eye. Patients will be supplied with drug and required to record the administration of each drug dose in patient diaries. Patient signs and symptoms of Dry Eye will be assessed in follow-up visits at the end of week 2, week 4, week 6, week 8 and/or week 12. Patient exams will include assessments of safety, visual acuity, slit-lamp exam, dilated fundoscopy. Endpoints will be measured at the clinic in normal office conditions (referred to as "environmental" conditions) and measured during and/or immediately following prolonged exposure to a controlled environment (i.e., controlled humidity, temperature, air-flow, and visual tasking; also referred to as a "controlled ambient environment").

Objective clinical measures of efficacy will include: 1) corneal staining with fluorescein, 2) conjunctival staining with lissamine green, 3) tear film break up time with fluorescein, 4) Schirmer tear tests with and without anesthesia, 5) conjunctival impression cytology (ICAM-1), 6) tear osmolarity, 7) blink rate, 8) ocular hyperemia, 9) Cochet Bonnet corneal sensitivity, 10) tear fluorophotometry, and 11) ocular protection index.

Subjective clinical measures of efficacy will include: 1) Ocular Surface Disease Index, 2) Patient global self-assessment (self-scored ocular discomfort) 3) Visual analog scale, and 4) drop comfort (tolerability assessment).

Results of this trial will support regulatory claims to the treatment or prevention of signs and symptoms from keratoconjunctivitis sicca (dry eye) with or without concomitant use of lubricating eye drops.

### Example 14: Diabetic Retinopathy (DR) and Diabetic Macular Edema (DME) (Reference Example)

DR and DME are leukocyte mediated diseases. Adhesion of leukocyte to capillary epithelial cells seems critical in ischemia reperfusion mechanism.

### Human study

Subjects with type I or type II diabetes will be treated with Compound 12 for up to 3 years with both preserved and unpreserved formulations of Compound 12 ophthalmic drops. Unpreserved drug will be supplied as a sterile unit dose in a single use blow-fill-seal container containing Compound 12 formulated in PBS. Preserved drug will be supplied as a sterile multi-use container containing Compound 12 formulated in PBS containing preservative. Each group of test subjects will be treated QD, BID or TID with different dose strengths of Compound 12 ophthalmic drops or placebo in preserved or unpreserved formulations. Drug will be self administered by each subject as a single drop to each eye once, twice or three times a day. Administered dose strengths will include placebo (PBS vehicle) 0.1%, 0.3%, 1% and 5% solutions of Compound 12. To enhance patient compliance, Compound 12 can be administered as a slow release formulation which delivers drug to the retina over the course of the study.

At enrollment, patients must have a diagnosis of type I or type II diabetes and non-proliferative diabetic retinopathy. Patients may also have concomitant diabetic macular edema. Patients will be supplied with drug and required to record the administration of each drug dose in patient diaries. Patients will be assessed every 2 months for the duration of the study. Each patient exam will include assessments of safety, visual acuity, slit-lamp exam, dilated fundoscopy.

Objective measures of efficacy will include: 1) Best corrected visual acuity using Early Treatment of Diabetic Retinopathy (ETDRS) method at 4 meters, 2) Reduction in retinal thickness measured by optical coherence tomography (OCT), and 3) Progression of diabetic retinopathy.

Subjective clinical measures of efficacy will include: 1) improvement NEI-VFQ 25 and other validated patient-reported outcome instruments.

Results of this trial will support regulatory claims to the prevention of the progression of diabetic retinopathy at 4, 8 weeks, 1, 2, and 3 years; maintenance or improvement in visual acuity; prevention, treatment, and/or reduction in macular edema; can be used in combination with focal and grid laser, intravitreal steroids, photodynamic therapy, and/or anti-VEGF therapies.

**Rat STZ model of Diabetic Macular Edema (DME) Pilot study** Anti-ICAM antibodies have shown efficacy in a rat STZ model of DME. Compound 12 radiolabel distribution studies in rat demonstrate delivery to retina. STZ (strptozocin) is used to generate an animal model for Type 1 diabetes. A definitive STZ rat study with Compound 12 will include 5 groups with 18 animals. Group no. 1 is normal SD rats that will receive no treatment. Group no. 2 is STZ rats that receive vehicle drops BID/2 months. Group no. 3 is STZ rats that receive 1% Compound 12 drops BID/2 months. Group no. 4 is STZ rats that will receive 5% Compound 12 drops BID/2 months. Group no. 5 is STZ rats that will receive celecoxib positive control. Endpoints for the study will include: retinal FITC-dextran leakage, vitreous-plasma protein ratio, myeloperoxidase assay, and retinal leukostasis.

Leukostasis is studied as described in U.S. Patent Application No. 20080019977 using Acridine Orange Leukocyte Fluorography (AOLF) and Fluorescein Angiography. Leukocyte dynamics in the retina are studied with AOLF (Miyamoto, K., et al., Invest. Opthalmol. Vis. Sci., 39:2190-2194 (1998); Nishiwaki, H., et al., Invest. Opthalmol. Vis. Sci., 37:1341-1347 (1996); Miyamoto, K., et al., Invest. Opthalmol. Vis. Sci., 37:2708-2715 (1996)). Intravenous injection of acridine orange causes leukocytes and endothelial cells to fluoresce through the non-covalent binding of the molecule to double stranded nucleic acid. When a scanning laser opthalmoscope is utilized, retinal leukocytes within blood vessels can be visualized in vivo. Twenty minutes after acridine orange injection, static leukocytes in the capillary bed can be observed. Immediately after observing and recording the static leukocytes, fluorescein angiography is performed to study the relationship between static leukocytes and retinal vasculature.

Twenty-four hours before AOLF and fluorescein angiography is performed, all rats had a heparin-lock catheter surgically implanted in the right jugular vein for the administration of acridine orange or sodium fluorescein dye. The catheter is subcutaneously externalized to the back of the neck. The rats are anesthetized for this procedure with xylazine hydrochloride (4 mg/kg) and ketamine hydrochloride (25 mg/kg). Immediately before AOLF, each rat is again anesthetized, and the pupil of the left eye is dilated with 1% tropicamide to observe leukocyte dynamics. A focused image of the peripapillary fundus of the left eye is obtained with a scanning laser opthalmoscope (SLO). Acridine orange is dissolved in sterile saline (1.0 mg/ml) and 3 mg/kg is injected through the jugular vein catheter at a rate of 1 ml/min. The fundus is observed with the SLO using the argon blue laser as the illumination source and the standard fluorescein angiography filter in the 40° field setting for 1 minute. Twenty minutes later, the fundus is again observed to evaluate leukostasis in the retina. Immediately after evaluating retinal leukostasis, 20 µl of 1% sodium fluorescein dye is injected into the jugular vein catheter. The images are recorded on a videotape at the rate of 30 frames/sec. The video recordings are analyzed on a computer equipped with a video digitizer that digitizes the video image in real time (30 frames/sec) to 640 x 480 pixels with an intensity resolution of 256 steps. For evaluating retinal leukostasis, an observation area around the optic disc measuring ten disc diameters in diameter is determined by drawing a polygon surrounded by the adjacent major retinal vessels. The area is measured in pixels and the density of trapped leukocytes is calculated by dividing the number of trapped leukocytes, which are recognized as fluorescent dots, by the area of the observation region. The leukocyte densities are calculated generally in eight peripapillary observation areas and an average density is obtained by averaging the eight density values.

Compound 12 is expected to reduce leukostasis and blood-retinal barrier leakage in STZ treated rats.

### Example 15: Age Related Macular Degeneration (AMD) (Reference Example)

Subjects with wet or dry AMD will be treated with Compound 12 for up to 3 years with both preserved and unpreserved formulations of Compound 12 ophthalmic drops. Unpreserved drug will be supplied as a sterile unit dose in a single use blow-fill-seal container containing Compound 12 formulated in PBS. Preserved drug will be supplied as a sterile multi-use container containing Compound 12 formulated in PBS containing preservative. Each group of test subjects will be treated QD, BID or TID with different dose strengths of Compound 12 ophthalmic drops or placebo in preserved or unpreserved formulations. Drug will be self administered by each subject as a single drop to each eye once, twice, or three times a day. Administered dose strengths will include placebo (PBS vehicle) 0.1%, 0.3%, 1% and 5% solutions of Compound 12. To enhance patient compliance, Compound 12 can be administered as a slow release formulation which delivers drug to the retina over the course of the study.

At enrollment, patients must have a diagnosis of wet or dry AMD. Patients may also have concomitant diabetic macular edema. Patients will be supplied with drug and required to record the administration of each drug dose in patient diaries. Patients will be assessed every 2 months for the duration of the study. Each patient exam will include assessments of safety, visual acuity, slit-lamp exam, dilated fundoscopy.

Objective measures will include: best corrected visual acuity; prevention of progression of geographic atrophy; and prevention of conversion to neovascular (wet AMD).

Results of this trial will support regulatory claims to the prevention of geographic atrophy related to dry AMD; can be used in conjunction with genetic biomarker or other type of diagnostic study that predicts subjects at high risk; and can be used in conjunction with anti-oxidant and/or anti-neovascular or anti-VEGF agents.

### Example 16: Phase 2 Atopic dermatitis

Subjects with atopic dermatitis will be treated with Compound 12 for up to 12 months. Drug will be supplied as a suitable dermatologic formulation for local application (cream, lotion, gel or ointment) containing Compound 12. Each group of test subjects will be treated QD, BID or TID with different dose strengths of Compound 12 ophthalmic drops or placebo in formulation. Drug will be self administered by each subject by gentle rubbing onto the effected area. Administered dose strengths will include placebo (vehicle) 0.1%, 0.3%, 1%, and 2% preparations of Compound 12. To enhance effect, treated areas may covered with an occlusive dressing. To improve patient compliance, drug may be administered as a slow release drug-impregated patch.

At enrollment, patients must have a diagnosis of atopic dermatitis. Patients will be supplied with drug and required to record the administration of each drug dose in patient diaries. Patients will be assessed every 2 weeks for the duration of the study. Each patient exam will include assessments of safety and tolerability. Measures of efficacy will include a physician's global assessment, a reduction in affected body surface area or a reduction in pruritis score.

Results of this trial will support regulatory claims to treatment of atopic dermatitis.

### Example 17: Crohn's Disease, Ulcerative Colitis or IBD (Reference Example)

Subjects with Crohn's disease, ulcerative colitis or IBD will be treated with Compound 12 for up to 12 months. Drug will be supplied as a formulation suitable for oral administration (solution, pill, or capsule) containing Compound 12. A typical oral solution dosage form would include Compound 12 dissolved in PBS adjusted to pH 7. Each group of test subjects will be treated QD, BID or TID with different dose strengths of Compound 12 or placebo in formulation. Drug will be self administered by each subject by mouth. Administered dose strengths will include placebo (vehicle) 1 mg per dose, 5 mg per dose, 10 mg per dose and up to 100 mg per dose of Compound 12 in formulation.

At enrollment, patients must have a diagnosis of Crohn's disease, ulcerative colitis or IBD. Patients will be supplied with drug and required to record the administration of each drug dose in patient diaries. Treatment with Compound 12 can be used in conjunction with current anti-inflammatories (eg, salicylates) and immunosuppressants (methotrexates, steroids, antibodies).

Patients will be assessed every 2 weeks for the duration of the study. Each patient exam will include assessments of safety and tolerability. Measures of efficacy will include the Crohn's Disease Activity Index (CDAI); disease activity index or similar scale for ulcerative colitis.

Results of this trial will support regulatory claims to the treatment and maintenance of remission of Crohn's disease, ulcerative colitis and/or IBD.

## Claims

1. A pharmaceutical formulation comprising an LFA-1 antagonist or a pharmaceutically acceptable salt or ester thereof, and an excipient formulated for topical administration to skin, wherein the LFA-1 antagonist has a systemic clearance rate greater than about 2 mL/min/kg when administered to a subject, and wherein the formulation is:
(a) a gel comprising about 1% W/V of a LFA-1 antagonist; up to about 15% WN Dimethyl Isosorbide; up to about 25% W/V Transcutol; up to about 1% WN Hydroxyethyl Cellulose; up to about 12%WN Hexylene glycol, up to about 0.15% W/V Methylparaben; up to about 0.05% W/V Propylparaben; and water; or
(b) a ointment comprising about 1% W/V of a LFA-1 antagonist, up to about 10% WN Dimethyl Isosorbide; up to about 0.02% W/V Butylated Hydroxytoluene; up to about 2% W/V Span 80; up to about 10% W/V White Wax; and White Petrolatum; or
(c) a water based lotion comprising about 1% W/V of a LFA-1 antagonist, up to about 15% W/V Dimethyl Isosorbide; up to about 25% W/V Transcutol; up to about 12% W/V Hexylene glycol; up to about 5% W/V Propylene Glycol; and pH 6.0 25% Trolamine, wherein the lotion is buffered to a pH of about 4.0 to about 7.5; or
(d) an aqueous solution buffered to a pH of about 6.0 to about 8.0 with Sodium Phosphate, Monobasic, comprising about 1% W/V of a LFA-1 antagonist, up to about 0.1% W/V EDTA, and, optionally, up to about 0.4% w/w Methylparaben and up to about 0.02% w/w Propylparaben;
and wherein the LFA-1 antagonist comprises a compound of one of the following formulae and/or a pharmaceutically acceptable salt or ester thereof:

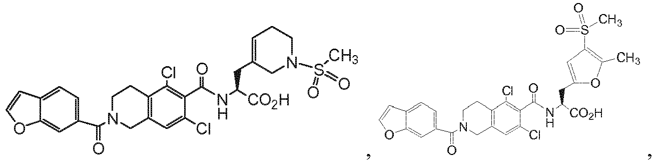

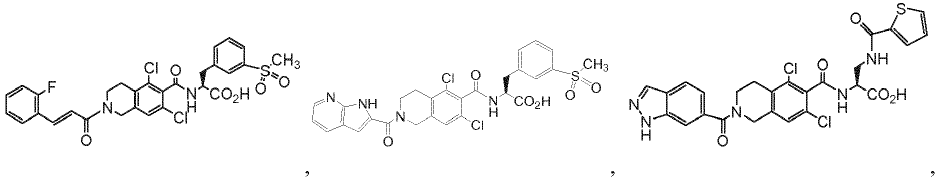

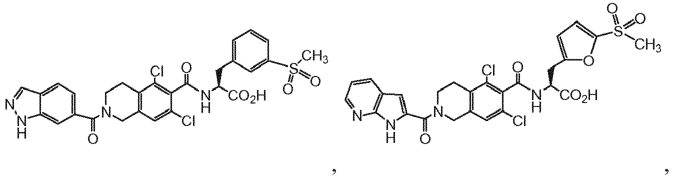

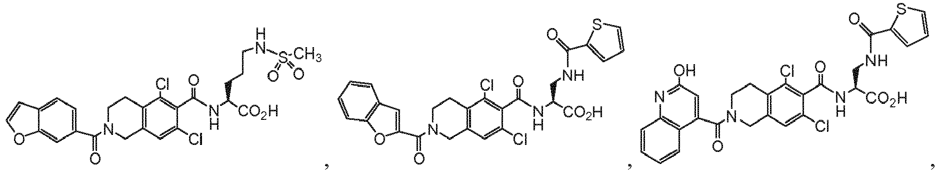

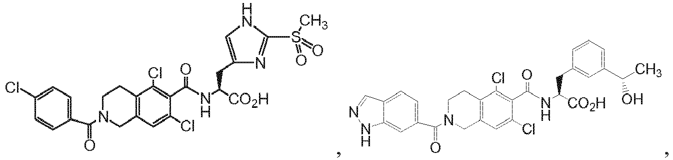

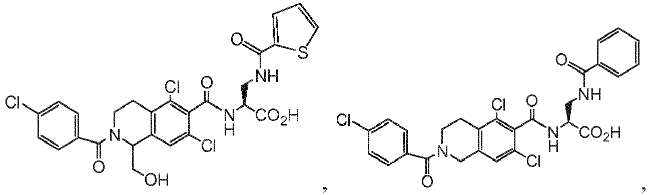

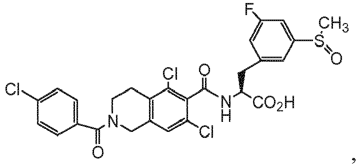

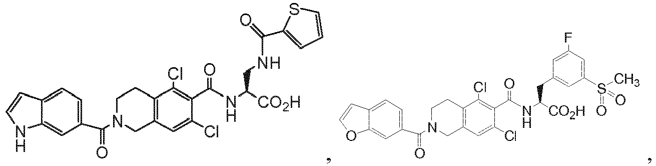

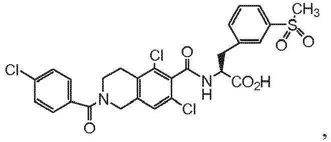

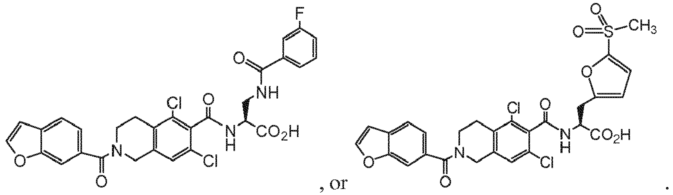

2. The formulation of claim 1 wherein the LFA-1 antagonist is a sodium, potassium, lithium, magnesium, zinc, or calcium salt.

3. The formulation of claim 1, wherein the excipient is water, buffered aqueous solution, surfactant, volatile liquid, starch, polyol, granulating agent, microcrystalline cellulose, diluent, lubricant, acid, base, salt, emulsion, oil, wetting agent, chelating agent, antioxidant, sterile solution, complexing agent or disintegrating agent.

4. The formulation of claim 3, wherein the surfactant is oleic acid, cetylpyridinium chloride, soya lecithin, polyoxyethylene sorbitan monolaurate, polyoxyethylene sorbitan monostearate, polyoxyethylene sorbitan monooleate, polyoxyethylene stearyl ether, polyoxyethylene oleyl ether, polyoxyethylene-polyoxypropylene-ethylenediamine block copolymer, polyoxypropylene-polyoxyethylene block copolymer or castor oil ethoxylate.

5. The formulation of claim 1, further comprising a topical penetration enhancer.

6. The formulation of claim 5, wherein the topical penetration enhancer is a sulfoxide, ether, surfactant, alcohol, fatty acid, fatty acid ester, polyol, amide, terpene, alkanone or organic acid.

7. The formulation of claim 1, further comprising at least one additional therapeutic agent.

8. The formulation of claim 7, wherein the additional therapeutic agent is an antioxidant, antiinflammatory agent, antimicrobial agent, antiangiogenic agent, anti-apoptotic agent, vascular endothelial growth factor inhibitor, antiviral agent, calcineurin inhibitor, corticosteroid, or immunomodulator.

9. A formulation as defined in any of claims 1-8 for use in the treatment of an inflammatory or immune related disorder in a subject, wherein the inflammatory or immune related disorder is psoriasis, irritant contact dermatitis, eczematous dermatitis, seborrhoeic dermatitis, cutaneous manifestations of immunologically-mediated disorders, alopecia, alopecia areata, scleredoma, scar formation, atopic dermatitis, hidradentis supporativa, rheumatoid arthritis, psoriatic arthritis, or arthralgia.

10. The formulation for use according to claim 9, wherein the formulation is administered in a dose from about 0.01 to about 5 mg.

## Patentansprüche

1. Pharmazeutische Formulierung, umfassend einen LFA-1-Antagonisten oder ein pharmazeutisch unbedenkliches Salz oder einen pharmazeutisch unbedenklichen Ester davon und einen für die topische Verabreichung auf die Haut formulierten Exzipienten, wobei der LFA-1-Antagonist bei der Verabreichung an ein Subjekt eine systemische Clearance-Rate von mehr als etwa 2 ml/min/kg hat, und wobei es sich bei der Formulierung um:
(a) ein Gel, das etwa 1% (w/v) eines LFA-1-Antagonisten, bis zu etwa 15% (w/v) Dimethylisosorbid, bis zu etwa 25% (w/v) Transcutol, bis zu etwa 1% (w/v) Hydroxyethylcellulose, bis zu etwa 12% (w/v) Hexylenglykol, bis zu etwa 0,15% (w/v) Methylparaben, bis zu etwa 0,05% (w/v) Propylparaben und Wasser umfasst, oder
(b) eine Salbe, die etwa 1% (w/v) eines LFA-1-Antagonisten, bis zu etwa 10% (w/v) Dimethylisosorbid, bis zu etwa 0,02% (w/v) Butylhydroxytoluol, bis zu etwa 2% (w/v) Span 80, bis zu etwa 10% (w/v) Chinawachs und Vaseline umfasst, oder
(c) eine Lotion auf Wasserbasis, die etwa 1% (w/v) eines LFA-1-Antagonisten, bis zu etwa 15% (w/v) Dimethylisosorbid, bis zu etwa 25% (w/v) Transcutol, bis zu etwa 12% (w/v) Hexylenglykol, bis zu etwa 5% (w/v) Propylenglykol und pH 6,0 25% Trolamin umfasst, wobei die Lotion auf einen pH-Wert von etwa 4,0 bis etwa 7,5 gepuffert ist, oder
(d) eine mit Natriumdihydrogenphosphat auf einen pH-Wert von etwa 6,0 bis etwa 8,0 gepufferte wässrige Lösung, die etwa 1% (w/v) eines LFA-1-Antagonisten, bis zu etwa 0,1% (w/v) EDTA und gegebenenfalls bis zu etwa 0,4% (w/w) Methylparaben und bis zu etwa 0,02% (w/w) Propylparaben umfasst,
handelt und wobei der LFA-1-Antagonist eine Verbindung einer der folgenden Formeln und/oder ein pharmazeutisch unbedenkliches Salz oder einen pharmazeutisch unbedenklichen Ester davon umfasst:

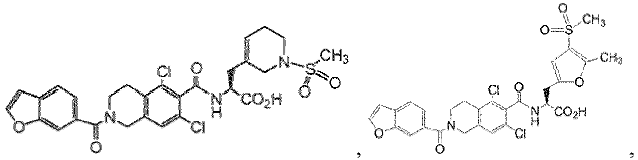

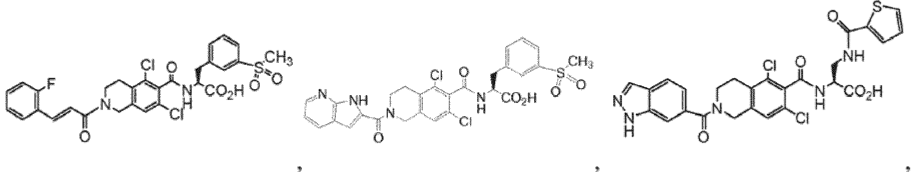

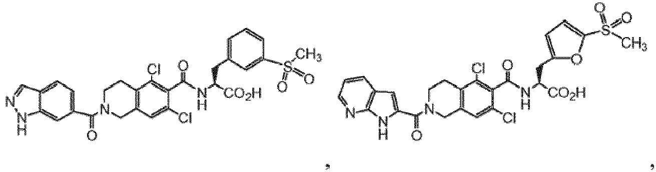

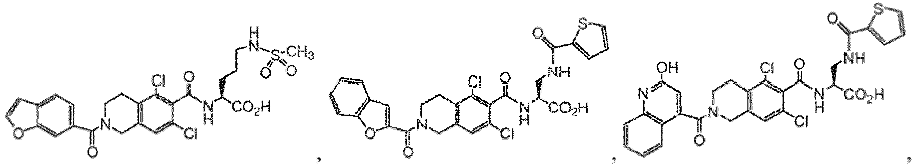

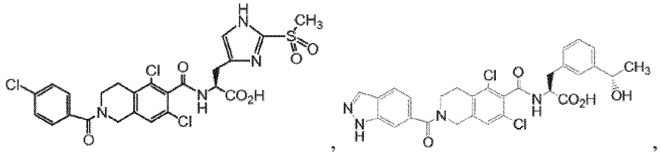

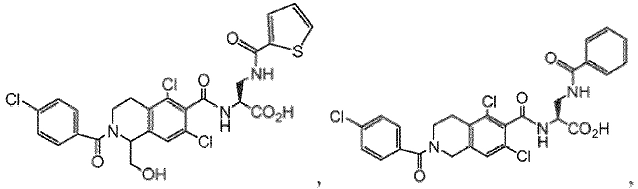

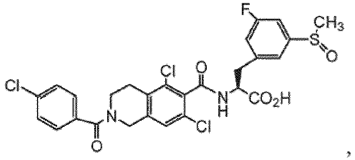

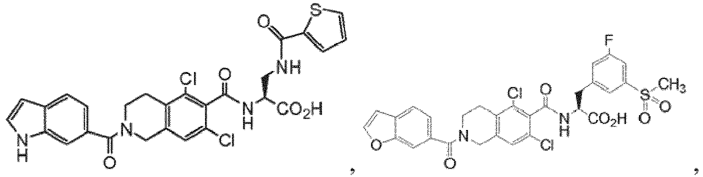

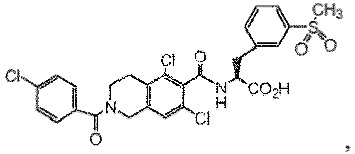

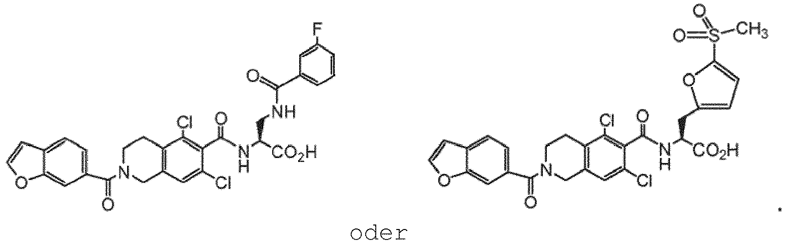

2. Formulierung nach Anspruch 1, wobei es sich bei dem LFA-1-Antagonisten um ein Natrium-, Kalium-, Lithium-, Magnesium-, Zink- oder Calziumsalz handelt.

3. Formulierung nach Anspruch 1, wobei es sich bei dem Exzipienten um Wasser, eine gepufferte wässrige Lösung, Tensid, eine flüchtige Flüssigkeit, Stärke, Polyol, ein Granuliermittel, mikrokristalline Cellulose, ein Verdünnungsmittel, ein Schmiermittel, eine Säure, eine Base, ein Salz, eine Emulsion, ein Öl, ein Netzmittel, einen Chelator, ein Antioxidationsmittel, eine sterile Lösung, ein Komplexiermittel oder ein Sprengmittel handelt.

4. Formulierung nach Anspruch 3, wobei es sich bei dem Tensid um Ölsäure, Cetylpyridiniumchlorid, Sojalecithin, Polyoxyethylensorbitanmonolaurat, Polyoxyethylensorbitanmonostearat, Polyoxyethylensorbitanmonooleat, Polyoxyethylenstearylether, Polyoxyethylenoleylether, Polyoxyethylen-Polyoxypropylen-Ethylendiamin-Blockcopolymer, Polyoxypropylen-Polyoxyethylen-Blockcopolymer oder Rizinusölethoxylat handelt.

5. Formulierung nach Anspruch 1, welche weiterhin einen topischen Penetrationsverstärker umfasst.

6. Formulierung nach Anspruch 5, wobei es sich bei dem topischen Penetrationsverstärker um ein Sulfoxid, einen Ether, ein Tensid, einen Alkohol, eine Fettsäure, einen Fettsäureester, ein Polyol, ein Amid, ein Terpen, ein Alkanon oder eine organische Säure handelt.

7. Formulierung nach Anspruch 1, welche weiterhin mindestens ein zusätzliches Therapeutikum umfasst.

8. Formulierung nach Anspruch 7, wobei es sich bei dem zusätzlichen Therapeutikum um ein Antioxidationsmittel, ein entzündungshemmendes Mittel, ein antimikrobielles Mittel, ein antiangiogenes Mittel, ein antiapoptotisches Mittel, einen Inhibitor des Vascular Endothelial Growth Factor, ein antivirales Mittel, einen Calcineurin-Inhibitor, ein Corticosteroid oder einen Immunmodulator handelt.

9. Formulierung, definiert wie in einem der Ansprüche 1-8, zur Verwendung bei der Behandlung einer entzündlichen oder mit dem Immunsystem in Zusammenhang stehenden Erkrankung in einem Subjekt, wobei es sich bei der entzündlichen bzw. mit dem Immunsystem in Zusammenhang stehenden Erkrankung um Psoriasis, reizende Kontaktdermatitis, ekzematöse Dermatitis, seborrhoische Dermatitis, kutane Manifestationen von durch das Immunsystem vermittelten Erkrankungen, Alopezie, Alopecia areata, Skleroderm, Narbenbildung, atopische Dermatitis, Hidradentis supporativa, rheumatoide Arthritis, Psoriasisarthritis oder Arthralgie handelt.

10. Formulierung zur Verwendung nach Anspruch 9, wobei die Formulierung in einer Dosis von etwa 0,01 bis etwa 5 mg verabreicht wird.

## Revendications

1. Formulation pharmaceutique comprenant un antagoniste du LFA-1 ou un sel ou ester pharmaceutiquement acceptable de celui-ci et un excipient formulée pour l'administration topique à la peau, l'antagoniste du LFA-1 ayant une vitesse de clairance systémique supérieure à environ 2 ml/min/kg lorsqu'il est administré à un sujet et la formulation étant :
(a) un gel comprenant environ 1 % p/v d'un antagoniste du LFA-1 ; jusqu'à environ 15 % p/v de diméthylisosorbide ; jusqu'à environ 25 % p/v de Transcutol ; jusqu'à environ 1 % p/v d'hydroxyéthylcellulose ; jusqu'à environ 12 % p/v d'hexylèneglycol, jusqu'à environ 0,15 % p/v de méthylparabène ; jusqu'à environ 0,05 % p/v de propylparabène ; et de l'eau ; ou
(b) un onguent comprenant environ 1 % p/v d'un antagoniste du LFA-1, jusqu'à environ 10 % p/v de diméthylisosorbide ; jusqu'à environ 0,02 % p/v d'hydroxytoluène butylé ; jusqu'à environ 2 % p/v de Span 80 ; jusqu'à environ 10 % p/v de cire blanche ; et de la vaseline ; ou
(c) une lotion à base d'eau comprenant environ 1 % p/v d'un antagoniste du LFA-1, jusqu'à environ 15 % p/v de diméthylisosorbide ; jusqu'à environ 25 % p/v de Transcutol ; jusqu'à environ 12 % p/v d'hexylèneglycol ; jusqu'à environ 5 % p/v de propylèneglycol ; et de la Trolamine à 25 % à pH 6, 0, la lotion étant tamponnée à un pH d'environ 4, 0 à environ 7,5 ; ou
(d) une solution aqueuse tamponnée à un pH d'environ 6,0 à environ 8,0 avec du dihydrogénophosphate de sodium, comprenant environ 1 % p/v d'un antagoniste du LFA-1, jusqu'à environ 0,1 % p/v d'EDTA et, éventuellement, jusqu'à environ 0,4 % p/p de méthylparabène et jusqu'à environ 0,02 % p/p de propylparabène ;
et dans laquelle l'antagoniste du LFA-1 comprend un composé représenté par l'une des formules suivantes et/ou un sel ou ester pharmaceutiquement acceptable de celui-ci :

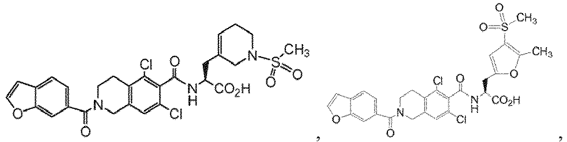

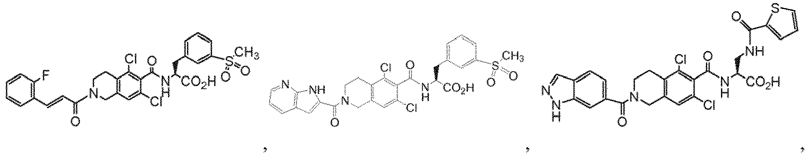

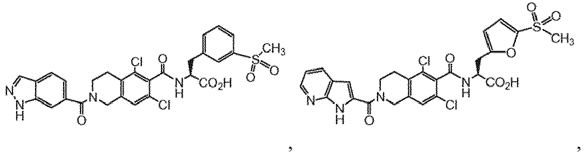

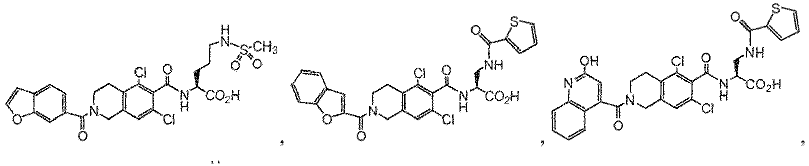

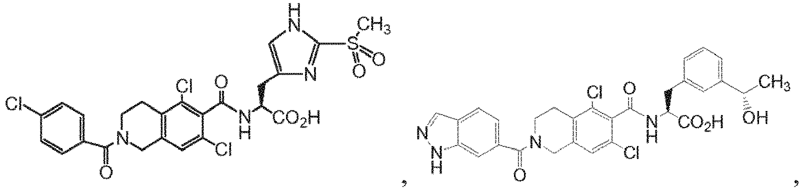

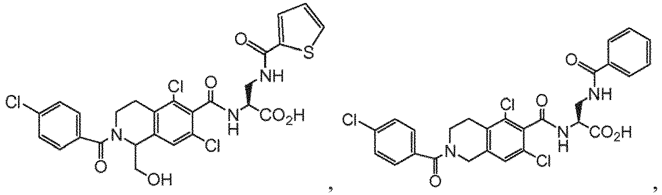

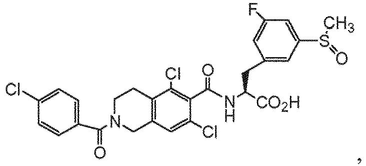

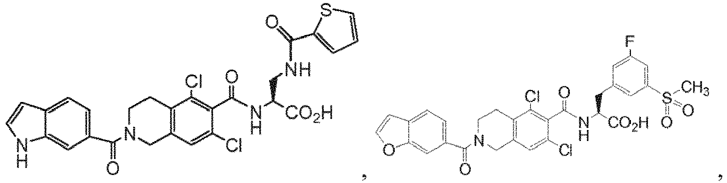

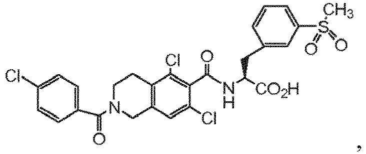

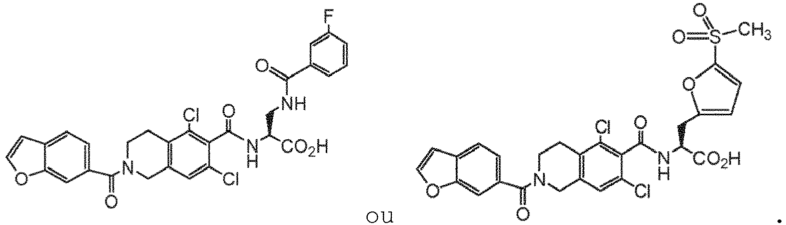

2. Formulation selon la revendication 1 dans laquelle l'antagoniste du LFA-1 est un sel de sodium, de potassium, de lithium, de magnésium, de zinc ou de calcium.

3. Formulation selon la revendication 1, dans laquelle l'excipient est l'eau, une solution aqueuse tamponnée, un tensioactif, un liquide volatil, l'amidon, un polyol, un agent de granulation, la cellulose microcristalline, un diluant, un lubrifiant, un acide, une base, un sel, une émulsion, une huile, un agent mouillant, un agent chélatant, un antioxydant, une solution stérile, un agent complexant ou un agent délitant.

4. Formulation selon la revendication 3, dans laquelle le tensioactif est l'acide oléique, le chlorure de cétylpyridinium, la lécithine de soja, le monolaurate de sorbitane polyoxyéthylénique, le monostéarate de sorbitane polyoxyéthylénique, le monooléate de sorbitane polyoxyéthylénique, l'éther stéarylique de polyoxyéthylène, l'éther oléylique de polyoxyéthylène, un copolymère séquencé de polyoxyéthylène-polyoxypropylène-éthylènediamine, un copolymère séquencé de polyoxypropylène-polyoxyéthylène ou un produit d'éthoxylation d'huile de ricin.

5. Formulation selon la revendication 1, comprenant en outre un agent améliorant la pénétration topique.

6. Formulation selon la revendication 5, dans laquelle l'agent améliorant la pénétration topique est un sulfoxyde, un éther, un tensioactif, un alcool, un acide gras, un ester d'acide gras, un polyol, un amide, un terpène, une alcanone ou un acide organique.

7. Formulation selon la revendication 1, comprenant en outre au moins un agent thérapeutique supplémentaire.

8. Formulation selon la revendication 7, dans laquelle l'agent thérapeutique supplémentaire est un antioxydant, un agent antiinflammatoire, un agent antimicrobien, un agent antiangiogénique, un agent antiapoptotique, un inhibiteur du facteur de croissance endothélial vasculaire, un agent antiviral, un inhibiteur de la calcineurine, un corticostéroïde ou un immunomodulateur.

9. Formulation telle que définie dans l'une quelconque des revendications 1-8 destinée à être utilisée dans le traitement d'un trouble inflammatoire ou lié au système immunitaire chez un sujet, le trouble inflammatoire ou lié au système immunitaire étant le psoriasis, la dermatite de contact irritative, la dermatite eczémateuse, la dermatite séborrhéique, des manifestations cutanées de troubles à médiation immunologique, l'alopécie, l'alopécie en aires, la sclérodermie, la formation d'une cicatrice, la dermatite atopique, l'hidrosadénite suppurée, la polyarthrite rhumatoïde, l'arthrite psoriasique ou une arthralgie.

10. Formulation destinée à être utilisée selon la revendication 9, la formulation étant administrée en une dose d'environ 0,01 à environ 5 mg.
